# EUROPEAN PATENT APPLICATION

(11) **EP 4 524 250 A1**
(43) Date of publication of application: **19.03.2025**
(21) Application number: 23803035.7
(22) Date of filing: 12.05.2023
(51) Int. Cl.: C12N 15/50, A61K 39/215, A61K 9/127, A61K 47/24, A61P 31/14

(54) **NUCLEIC ACID CONSTRUCT COMPRISING UTR AND USE THEREOF**

(30) Priority: 13.05.2022 CN 202210522056
(71) Applicant: Shanghai Regenelead Therapies Co., Ltd., Shanghai 201206 (CN)
(72) Inventor: JIANG, Jun, Shanghai 201206 (CN); YANG, Linfeng, Shanghai 201206 (CN); CHEN, Qinjun, Shanghai 201206 (CN); SONG, Xiaoyu, Shanghai 201206 (CN); NING, Wei, Shanghai 201206 (CN); LIAO, Cheng, Shanghai 201206 (CN)
(74) Representative: Potter Clarkson
(86) International application number: PCT/CN2023/093851
(87) International publication number: WO 2023/217267

(57) **Abstract**

The present invention relates to a nucleic acid construct comprising a UTR and use thereof. Specifically, the UTR can significantly improve the expression efficiency of a target gene in the nucleic acid construct. The nucleic acid construct may comprise a nucleic acid sequence expressing, for example, a human hepatocyte growth factor (hHGF), and can be used in a gene therapy for serious lower limb ischemia, diabetic foot, and other diseases.

## Description

The present disclosure claims priority to the following patent application: Chinese Patent Application No. CN202210522056.8, filed on May 13, 2022 and entitled "NUCLEIC ACID CONSTRUCT COMPRISING UTR AND USE THEREOF", which is incorporated herein by reference in its entirety.

### TECHNICAL FIELD

The present disclosure belongs to the field of nucleic acids and relates to a nucleic acid construct comprising UTRs and use thereof for preventing or treating a disease. The nucleic acid construct can comprise a nucleic acid sequence that expresses human hepatocyte growth factor (hHGF) and is used as a gene therapy drug for diseases such as critical limb ischemia and diabetic foot ulcer.

### BACKGROUND

Gene therapy and genetic vaccination can offer highly specific and personalized treatment and prevention plans for a variety of diseases, including genetic diseases, autoimmune diseases, cancer or tumor-related diseases, and inflammatory diseases.

Both DNA and RNA can be used in gene therapy or genetic vaccination. DNA is stable and easy to manipulate but involves a risk of mutational events (e.g., loss of function of a damaged gene) and the like due to the insertion of a DNA fragment into the genome of the patient. RNA can avoid unwanted genomic integration but is prone to degradation due to ubiquitous RNases. Therefore, it is necessary to improve RNA stability, allowing its encoded protein product to accumulate *in vivo* so as to realize disease treatment or prevention, and maintaining the structural and functional integrity of RNA during storage and administration. Naturally occurring eukaryotic mRNA molecules have been found to contain stabilizing elements, such as untranslated regions (UTRs) at the 5' and 3' ends, and other structural features such as the 5' cap structure and the 3' polyadenylic acid tail. The 5'UTR and 3'UTR are premature mRNA elements, and during mRNA processing, structural features unique to mature mRNA (such as the 5' cap and the 3' polyadenylic acid tail) are added to the transcribed (premature) mRNA. Regarding the correlation between UTRs and mRNA stability, studies have shown that the 3'UTR of α-globin mRNA is an important factor in α-globin mRNA stability (Nancy D Rodgers et al, RNA. 2002 Dec;8(12):1526-37; Z Wang et al, Mol Cell Biol. 1999 Jul;19(7):4552-60).

Peripheral artery disease (PAD) refers to a non-coronary arterial system syndrome caused by a range of structural and functional abnormalities in the arteries supplying the limbs, internal organs, and brain, is characterized by narrowing, occlusion, and tumor-like pathological changes in the non-coronary blood circulation, and affects the aorta and branch arteries. In PAD, ischemic diseases of the lower limbs are most clinically common. The main causes include atherosclerosis obliterans (ASO), diabetic artery obliterans (DAO), and thrombosis angiitis obliterans (TAO). Critical limb ischemia (CLI) is an advanced stage of ASO, and diabetic foot ulcer (DFU) is a type of DAO. Both are peripheral vasculopathic diseases characterized by pain, ulcers, and necrosis in the lower limbs due to narrowing or blockage of the lower limb blood vessels and insufficient distal blood perfusion. Currently, revascularization through operation or intracavity intervention is an effective treatment for CLI and DFU. However, more than 40% of patients are not eligible for revascularization due to age, comorbidities, etc., leaving conservative treatment with drugs as the only option. Drug therapy can only slow disease progression and cannot cure it.

Hepatocyte growth factor (HGF) is a multifunctional mesenchyme-derived growth factor. When binding to the cell membrane surface receptor c-met, it triggers phosphorylation of intracellular tyrosine residues, recruits adapter proteins, enhances kinase activity, and thereby activates downstream signaling pathways. HGF is an important regulatory factor in processes such as embryonic development, tissue and organ regeneration, wound healing, and angiogenesis. It can promote the proliferation and migration of endothelial cells and smooth muscle cells, promote microvascular network reconstruction in ischemic areas, and inhibit apoptosis. Research has shown that intramuscularly injecting naked plasmids to deliver hHGF-cDNA can significantly promote blood perfusion in ischemic lower limbs of rats and domestic rabbits (Y Taniyama et al. Therapeutic angiogenesis induced by human hepatocyte growth factor gene in rat and rabbit hindlimb ischemia models: preclinical study for treatment of peripheral arterial disease. Gene Ther. 2001 Feb;8(3):181-9). Clinical data show that injecting naked plasmids into calf muscles can promote wound healing in patients (S Cui et al. Clinical Safety and Preliminary Efficacy of Plasmid pUDK-HGF Expressing Human Hepatocyte Growth Factor (HGF) in Patients with Critical Limb Ischemia. Eur J Vasc Endovasc Surg. 2015 Oct;50(4):494-501). However, using naked plasmids to delivery drugs has drawbacks, such as low *in vivo* transfection efficiency, heavy administration dosage burden, high risk of DNA integration, and high treatment costs. Moreover, clinical data have not shown improvements in toe-brachial index, transcutaneous partial pressure of oxygen, or amputation rates. Therefore, there is still much room for improvement in the drugs.

The present disclosure provides an mRNA with a 5'UTR and a 3'UTR of new structures that reduce early degradation of the mRNA or stabilize degradation of the mRNA but do not compromise more or enhance protein translation efficiency. The mRNA is more stable and can be applied to gene therapy and genetic vaccination. Additionally, the present disclosure provides an mRNA capable of expressing human hepatocyte growth factor (hHGF) and a lipid nanoparticle (LNP) delivery system thereof, which enable efficient and rapid *in vivo* conversion of exogenous hHGF protein, have the advantages of involving no risk of integration and being easy to scale up industrially, are a more ideal treatment regimen than naked plasmids, and can be used as a gene therapy drug for a variety of diseases such as CLI and DFU.

### SUMMARY

The present disclosure provides a nucleic acid construct comprising at least one nucleic acid element capable of regulating the expression of a target gene, the nucleic acid element being a UTR. Additionally, the nucleic acid construct may comprise one or more target genes, the target genes being, for example, HGF.

### Nucleic Acid Construct

The present disclosure provides a nucleic acid construct comprising:
(a) an open reading frame (ORF), and
(b) an untranslated region element (UTR).

In some embodiments, the nucleic acid construct is a DNA molecule; in some embodiments, the nucleic acid construct is an RNA molecule (e.g., mRNA).

In some embodiments, the ORF is a polynucleotide sequence encoding a target gene.

In some embodiments, the target gene is heterologous. In some other embodiments, the target gene is endogenous. In some embodiments, one or more (e.g., 2, 3, or 4) said target genes are present.

In some embodiments, the UTR is derived from a UTR of the gene ACTG1, ATP6V0B, ATP6V0E1, CFL1, COX4I1, CTSB, FAM166A, NDUFB9, CHCHD10, SLC38A2, NDUFA11, NDUFV3, PRDX5, GUK1, IAH1, ABHD16A, SLC25A39, ATPIF1, ANAPC11, CCDC12, MRPL14, or APOA1BP. In some embodiments, the gene described above is a human gene.

In some embodiments, the UTR is a 3' untranslated region element (3'UTR) or a 5' untranslated region element (5'UTR).

In some embodiments, the 3'UTR and 5'UTR are of the same origin or different origins, e.g., derived from the same gene or different genes. For example, the 3'UTR is derived from a 3'UTR of the gene ACTG1, and the 5'UTR is derived from a 5'UTR of the gene ACTG1. As another example, the 3'UTR is derived from a 3'UTR of the gene CTSB, and the 5'UTR is derived from a 5'UTR of the gene CHCHD10. In some embodiments, the 5'UTR and 3'UTR are derived from the same species or different species.

In some embodiments, the 5'UTR is located upstream of the ORF. In some embodiments, the 5'UTR in the nucleic acid construct is located at the 5' end of the ORF. In some embodiments, the 3'UTR is located downstream of the ORF. In some embodiments, the 3'UTR in the nucleic acid construct is located at the 3' end of the ORF.

In some embodiments, the aforementioned nucleic acid construct comprises:
(a) an open reading frame (ORF);
(b-1) a 3'UTR, the 3'UTR being derived from a 3'UTR of the gene ACTG1, ATP6V0B, ATP6V0E1, CFL1, COX4I1, CTSB, FAM166A, or NDUFB9; and
(b-2) a 5'UTR, the 5'UTR being derived from a 5'UTR of the gene ACTG1, ATP6V0B, ATP6V0E1, CFL1, COX4I1, CTSB, FAM166A, NDUFB9, CHCHD10, SLC38A2, NDUFA11, NDUFV3, PRDX5, GUK1, IAH1, ABHD16A, SLC25A39, ATPIF1, ANAPC11, CCDC12, MRPL14, or APOA1BP.

In some embodiments, the aforementioned nucleic acid construct comprises:
(a) an open reading frame (ORF);
(b-1) a 3'UTR, the 3'UTR being derived from a 3'UTR of the gene CTSB, FAM166A, or NDUFB9; and
(b-2) a 5'UTR, the 5'UTR being derived from a 5'UTR of the gene ACTG1, CHCHD10, or NDUFA11.

In some embodiments, in the aforementioned nucleic acid construct (e.g., DNA or RNA molecule), the 3'UTR is derived from a 3'UTR of the gene ACTG1, comprising the sequence set forth in SEQ ID NO: 1 or SEQ ID NO: 58 or a sequence having identity to any one of SEQ ID NO: 1 and SEQ ID NO: 58;
the 3'UTR is derived from a 3'UTR of the gene ATP6V0B, comprising the sequence set forth in SEQ ID NO: 2 or 3 or SEQ ID NO: 59 or 60 or a sequence having identity to any one of SEQ ID NO: 2 or 3 and SEQ ID NO: 59 or 60;
the 3'UTR is derived from a 3'UTR of the gene ATP6V0E1, comprising the sequence set forth in SEQ ID NO: 4 or 5 or SEQ ID NO: 61 or 62 or a sequence having identity to any one of SEQ ID NO: 4 or 5 and SEQ ID NO: 61 or 62;
the 3'UTR is derived from a 3'UTR of the gene CFL1, comprising the sequence set forth in SEQ ID NO: 6, 7, or 8 or SEQ ID NO: 63, 64, or 65 or a sequence having identity to any one of SEQ ID NO: 6, 7, or 8 and SEQ ID NO: 63, 64, or 65;
the 3'UTR is derived from a 3'UTR of the gene COX4I1, comprising the sequence set forth in SEQ ID NO: 9, 10, or 11 or SEQ ID NO: 66, 67, or 68 or a sequence having identity to any one of SEQ ID NO: 9, 10, or 11 and SEQ ID NO: 66, 67, or 68;
the 3'UTR is derived from a 3'UTR of the gene CTSB, comprising the sequence set forth in SEQ ID NO: 12 or SEQ ID NO: 69 or a sequence having identity to any one of SEQ ID NO: 12 and SEQ ID NO: 69;
the 3'UTR is derived from a 3'UTR of the gene FAM166A, comprising the sequence set forth in SEQ ID NO: 13 or SEQ ID NO: 70 or a sequence having identity to any one of SEQ ID NO: 13 and SEQ ID NO: 70; or
the 3'UTR is derived from a 3'UTR of the gene NDUFB9, comprising the sequence set forth in SEQ ID NO: 14 or SEQ ID NO: 71 or a sequence having identity to any one of SEQ ID NO: 14 and SEQ ID NO: 71.

In some embodiments, in the aforementioned nucleic acid construct (e.g., DNA or RNA molecule), the 5'UTR is derived from a 5'UTR of the gene ACTG1, comprising the sequence set forth in SEQ ID NO: 15 or SEQ ID NO: 72 or a sequence having identity to any one of SEQ ID NO: 15 and SEQ ID NO: 72;
the 5'UTR is derived from a 5'UTR of the gene ATP6V0B, comprising the sequence set forth in SEQ ID NO: 16 or 17 or SEQ ID NO: 73 or 74 or a sequence having identity to any one of SEQ ID NO: 16 or 17 and SEQ ID NO: 73 or 74;
the 5'UTR is derived from a 5'UTR of the gene ATP6V0E1, comprising the sequence set forth in SEQ ID NO: 18 or 19 or SEQ ID NO: 75 or 76 or a sequence having identity to any one of SEQ ID NO: 18 or 19 and SEQ ID NO: 75 or 76;
the 5'UTR is derived from a 5'UTR of the gene CFL1, comprising the sequence set forth in SEQ ID NO: 20, 21, or 22 or SEQ ID NO: 77, 78, or 79 or a sequence having identity to any one of SEQ ID NO: 20, 21, or 22 and SEQ ID NO: 77, 78, or 79;
the 5'UTR is derived from a 5'UTR of the gene COX4I1, comprising the sequence set forth in SEQ ID NO: 23, 24, or 25 or SEQ ID NO: 80, 81, or 82 or a sequence having identity to any one of SEQ ID NO: 23, 24, or 25 and SEQ ID NO: 80, 81, or 82;
the 5'UTR is derived from a 5'UTR of the gene CTSB, comprising the sequence set forth in SEQ ID NO: 26 or SEQ ID NO: 83 or a sequence having identity to any one of SEQ ID NO: 26 and SEQ ID NO: 83;
the 5'UTR is derived from a 5'UTR of the gene FAM166A, comprising the sequence set forth in SEQ ID NO: 27 or SEQ ID NO: 84 or a sequence having identity to any one of SEQ ID NO: 27 and SEQ ID NO: 84;
the 5'UTR is derived from a 5'UTR of the gene NDUFB9, comprising the sequence set forth in SEQ ID NO: 28 or SEQ ID NO: 85 or a sequence having identity to any one of SEQ ID NO: 28 and SEQ ID NO: 85;
the 5'UTR is derived from a 5'UTR of the gene CHCHD10, comprising the sequence set forth in SEQ ID NO: 29 or 30 or SEQ ID NO: 86 or 87 or a sequence having identity to any one of SEQ ID NO: 29 or 30 and SEQ ID NO: 86 or 87;
the 5'UTR is derived from a 5'UTR of the gene SLC38A2, comprising the sequence set forth in SEQ ID NO: 31 or SEQ ID NO: 88 or a sequence having identity to any one of SEQ ID NO: 31 and SEQ ID NO: 88;
the 5'UTR is derived from a 5'UTR of the gene NDUFA11, comprising the sequence set forth in SEQ ID NO: 32 or SEQ ID NO: 89 or a sequence having identity to any one of SEQ ID NO: 32 and SEQ ID NO: 89;
the 5'UTR is derived from a 5'UTR of the gene NDUFV3, comprising the sequence set forth in SEQ ID NO: 33 or SEQ ID NO: 90 or a sequence having identity to any one of SEQ ID NO: 33 and SEQ ID NO: 90;
the 5'UTR is derived from a 5'UTR of the gene PRDX5, comprising the sequence set forth in SEQ ID NO: 34 or SEQ ID NO: 91;
the 5'UTR is derived from a 5'UTR of the gene GUK1, comprising the sequence set forth in SEQ ID NO: 35, 36, or 37 or SEQ ID NO: 92, 93, or 94 or a sequence having identity to any one of SEQ ID NO: 35, 36, or 37 and SEQ ID NO: 92, 93, or 94;
the 5'UTR is derived from a 5'UTR of the gene IAH1, comprising the sequence set forth in SEQ ID NO: 38 or SEQ ID NO: 95 or a sequence having identity to any one of SEQ ID NO: 38 and SEQ ID NO: 95;
the 5'UTR is derived from a 5'UTR of the gene ABHD16A, comprising the sequence set forth in SEQ ID NO: 39 or SEQ ID NO: 96 or a sequence having identity to any one of SEQ ID NO: 39 and SEQ ID NO: 96;
the 5'UTR is derived from a 5'UTR of the gene SLC25A39, comprising the sequence set forth in SEQ ID NO: 40 or SEQ ID NO: 97 or a sequence having identity to any one of SEQ ID NO: 40 and SEQ ID NO: 97;
the 5'UTR is derived from a 5'UTR of the gene ATPIF1, comprising the sequence set forth in SEQ ID NO: 41 or SEQ ID NO: 98 or a sequence having identity to any one of SEQ ID NO: 41 and SEQ ID NO: 98;
the 5'UTR is derived from a 5'UTR of the gene ANAPC11, comprising the sequence set forth in SEQ ID NO: 42 or 43 or SEQ ID NO: 99 or 100 or a sequence having identity to any one of SEQ ID NO: 42 or 43 and SEQ ID NO: 99 or 100;
the 5'UTR is derived from a 5'UTR of the gene CCDC12, comprising the sequence set forth in SEQ ID NO: 44 or SEQ ID NO: 101 or a sequence having identity to any one of SEQ ID NO: 44 and SEQ ID NO: 101;
the 5'UTR is derived from a 5'UTR of the gene MRPL14, comprising the sequence set forth in SEQ ID NO: 45 or SEQ ID NO: 102 or a sequence having identity to any one of SEQ ID NO: 45 and SEQ ID NO: 102; or
the 5'UTR is derived from a 5'UTR of the gene APOA1BP, comprising the sequence set forth in SEQ ID NO: 46 or 47 or SEQ ID NO: 103 or 104 or a sequence having identity to any one of SEQ ID NO: 46 or 47 and SEQ ID NO: 103 or 104.

In some embodiments, provided is a nucleic acid construct (e.g., DNA or RNA molecule) comprising:
(a) an open reading frame (ORF),
(b-1) a 3'UTR, and
(b-2) a 5'UTR,
wherein the 3'UTR and 5'UTR are selected from the group consisting of any one of the following combinations:
   1) the 3'UTR comprises the sequence set forth in SEQ ID NO: 1 or SEQ ID NO: 58 or a sequence having identity to any one of SEQ ID NO: 1 and SEQ ID NO: 58, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   2) the 3'UTR comprises the sequence set forth in SEQ ID NO: 2 or SEQ ID NO: 59 or a sequence having identity to any one of SEQ ID NO: 2 and SEQ ID NO: 59, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   3) the 3'UTR comprises the sequence set forth in SEQ ID NO: 3 or SEQ ID NO: 60 or a sequence having identity to any one of SEQ ID NO: 3 and SEQ ID NO: 60, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   4) the 3'UTR comprises the sequence set forth in SEQ ID NO: 4 or SEQ ID NO: 61 or a sequence having identity to any one of SEQ ID NO: 4 and SEQ ID NO: 61, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   5) the 3'UTR comprises the sequence set forth in SEQ ID NO: 5 or SEQ ID NO: 62 or a sequence having identity to any one of SEQ ID NO: 5 and SEQ ID NO: 62, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   6) the 3'UTR comprises the sequence set forth in SEQ ID NO: 6 or SEQ ID NO: 63 or a sequence having identity to any one of SEQ ID NO: 6 and SEQ ID NO: 63, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   7) the 3'UTR comprises the sequence set forth in SEQ ID NO: 7 or SEQ ID NO: 64 or a sequence having identity to any one of SEQ ID NO: 7 and SEQ ID NO: 64, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   8) the 3'UTR comprises the sequence set forth in SEQ ID NO: 8 or SEQ ID NO: 65 or a sequence having identity to any one of SEQ ID NO: 8 and SEQ ID NO: 65, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   9) the 3'UTR comprises the sequence set forth in SEQ ID NO: 9 or SEQ ID NO: 66 or a sequence having identity to any one of SEQ ID NO: 9 and SEQ ID NO: 66, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   10) the 3'UTR comprises the sequence set forth in SEQ ID NO: 10 or SEQ ID NO: 67 or a sequence having identity to any one of SEQ ID NO: 10 and SEQ ID NO: 67, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   11) the 3'UTR comprises the sequence set forth in SEQ ID NO: 11 or SEQ ID NO: 68 or a sequence having identity to any one of SEQ ID NO: 11 and SEQ ID NO: 68, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   12) the 3'UTR comprises the sequence set forth in SEQ ID NO: 12 or SEQ ID NO: 69 or a sequence having identity to any one of SEQ ID NO: 12 and SEQ ID NO: 69, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   13) the 3'UTR comprises the sequence set forth in SEQ ID NO: 13 or SEQ ID NO: 70 or a sequence having identity to any one of SEQ ID NO: 13 and SEQ ID NO: 70, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   14) the 3'UTR comprises the sequence set forth in SEQ ID NO: 14 or SEQ ID NO: 71 or a sequence having identity to any one of SEQ ID NO: 14 and SEQ ID NO: 71, and the 5'UTR comprises the sequence set forth in any one of SEQ ID NOs: 15-47 or any one of SEQ ID NOs: 72-104 or a sequence having identity to any one of SEQ ID NOs: 15-47 and SEQ ID NOs: 72-104;
   15) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 15 or SEQ ID NO: 72 or a sequence having identity to any one of SEQ ID NO: 15 and SEQ ID NO: 72;
   16) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 16 or SEQ ID NO: 73 or a sequence having identity to any one of SEQ ID NO: 16 and SEQ ID NO: 73;
   17) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 17 or SEQ ID NO: 74 or a sequence having identity to any one of SEQ ID NO: 17 and SEQ ID NO: 74;
   18) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 18 or SEQ ID NO: 75 or a sequence having identity to any one of SEQ ID NO: 18 and SEQ ID NO: 75;
   19) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 19 or SEQ ID NO: 76 or a sequence having identity to any one of SEQ ID NO: 19 and SEQ ID NO: 76;
   20) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 20 or SEQ ID NO: 77 or a sequence having identity to any one of SEQ ID NO: 20 and SEQ ID NO: 77;
   21) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 21 or SEQ ID NO: 78 or a sequence having identity to any one of SEQ ID NO: 21 and SEQ ID NO: 78;
   22) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 22 or SEQ ID NO: 79 or a sequence having identity to any one of SEQ ID NO: 22 and SEQ ID NO: 79;
   23) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 23 or SEQ ID NO: 80 or a sequence having identity to any one of SEQ ID NO: 23 and SEQ ID NO: 80;
   24) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 24 or SEQ ID NO: 81 or a sequence having identity to any one of SEQ ID NO: 24 and SEQ ID NO: 81;
   25) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 25 or SEQ ID NO: 82 or a sequence having identity to any one of SEQ ID NO: 25 and SEQ ID NO: 82;
   26) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 26 or SEQ ID NO: 83 or a sequence having identity to any one of SEQ ID NO: 26 and SEQ ID NO: 83;
   27) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 27 or SEQ ID NO: 84 or a sequence having identity to any one of SEQ ID NO: 27 and SEQ ID NO: 84;
   28) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 28 or SEQ ID NO: 85 or a sequence having identity to any one of SEQ ID NO: 28 and SEQ ID NO: 85;
   29) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 29 or SEQ ID NO: 86 or a sequence having identity to any one of SEQ ID NO: 29 and SEQ ID NO: 86;
   30) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 30 or SEQ ID NO: 87 or a sequence having identity to any one of SEQ ID NO: 30 and SEQ ID NO: 87;
   31) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 31 or SEQ ID NO: 88 or a sequence having identity to any one of SEQ ID NO: 31 and SEQ ID NO: 88;
   32) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 32 or SEQ ID NO: 89 or a sequence having identity to any one of SEQ ID NO: 32 and SEQ ID NO: 89;
   33) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 33 or SEQ ID NO: 90 or a sequence having identity to any one of SEQ ID NO: 33 and SEQ ID NO: 90;
   34) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 34 or SEQ ID NO: 91 or a sequence having identity to any one of SEQ ID NO: 34 and SEQ ID NO: 91;
   35) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 35 or SEQ ID NO: 92 or a sequence having identity to any one of SEQ ID NO: 35 and SEQ ID NO: 92;
   36) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 36 or SEQ ID NO: 93 or a sequence having identity to any one of SEQ ID NO: 36 and SEQ ID NO: 93;
   37) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 37 or SEQ ID NO: 94 or a sequence having identity to any one of SEQ ID NO: 37 and SEQ ID NO: 94;
   38) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 38 or SEQ ID NO: 95 or a sequence having identity to any one of SEQ ID NO: 38 and SEQ ID NO: 95;
   39) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 39 or SEQ ID NO: 96 or a sequence having identity to any one of SEQ ID NO: 39 and SEQ ID NO: 96;
   40) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 40 or SEQ ID NO: 97 or a sequence having identity to any one of SEQ ID NO: 40 and SEQ ID NO: 97;
   41) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 41 or SEQ ID NO: 98 or a sequence having identity to any one of SEQ ID NO: 41 and SEQ ID NO: 98;
   42) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 42 or SEQ ID NO: 99 or a sequence having identity to any one of SEQ ID NO: 42 and SEQ ID NO: 99;
   43) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 43 or SEQ ID NO: 100 or a sequence having identity to any one of SEQ ID NO: 43 and SEQ ID NO: 100;
   44) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 44 or SEQ ID NO: 101 or a sequence having identity to any one of SEQ ID NO: 44 and SEQ ID NO: 101;
   45) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 45 or SEQ ID NO: 102 or a sequence having identity to any one of SEQ ID NO: 45 and SEQ ID NO: 102;
   46) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 46 or SEQ ID NO: 103 or a sequence having identity to any one of SEQ ID NO: 46 and SEQ ID NO: 103; or
   47) the 3'UTR comprises the sequence set forth in any one of SEQ ID NOs: 1-14 or any one of SEQ ID NOs: 58-71 or a sequence having identity to any one of SEQ ID NOs: 1-14 and SEQ ID NOs: 58-71, and the 5'UTR comprises the sequence set forth in SEQ ID NO: 47 or SEQ ID NO: 104 or a sequence having identity to any one of SEQ ID NO: 47 and SEQ ID NO: 104.

In some embodiments, provided is a nucleic acid construct (e.g., DNA or RNA molecule) comprising:
(a) an open reading frame (ORF),
(b-1) a 3'UTR, and
(b-2) a 5'UTR,
the 3'UTR is derived from a 3'UTR of the gene CTSB, FAM166A, or NDUFB9, and the 5'UTR is derived from a 5'UTR of the gene ACTG1, CHCHD10, or NDUFA11;
for example, the 3'UTR comprises any one of SEQ ID NOs: 12, 13, and 14 or any one of SEQ ID NOs: 69, 70, and 71 or a sequence having identity to any one of SEQ ID NOs: 12, 13, and 14 and SEQ ID NOs: 69, 70, and 71, and the 5'UTR comprises any one of SEQ ID NOs: 15, 29, 30, and 32 or any one of SEQ ID NOs: 72, 86, 87, and 89 or a sequence having identity to any one of SEQ ID NOs: 15, 29, 30, and 32 and SEQ ID NOs: 72, 86, 87, and 89;
as another example, the 3'UTR comprises the sequence set forth in SEQ ID NO: 12 or SEQ ID NO: 69 or a sequence having identity thereto any one of SEQ ID NO: 12 and SEQ ID NO: 69, and the 5'UTR comprises the sequence set forth in SEQ ID NO: 15 or SEQ ID NO: 72 or a sequence having identity to any one of SEQ ID NO: 15 and SEQ ID NO: 72;
the 3'UTR comprises the sequence set forth in SEQ ID NO: 13 or SEQ ID NO: 70 or a sequence having identity thereto any one of SEQ ID NO: 13 and SEQ ID NO: 70, and the 5'UTR comprises the sequence set forth in SEQ ID NO: 15 or SEQ ID NO: 72 or a sequence having identity to any one of SEQ ID NO: 15 and SEQ ID NO: 72;
the 3'UTR comprises the sequence set forth in SEQ ID NO: 14 or SEQ ID NO: 71 or a sequence having identity thereto any one of SEQ ID NO: 14 and SEQ ID NO: 71, and the 5'UTR comprises the sequence set forth in SEQ ID NO: 15 or SEQ ID NO: 72 or a sequence having identity to any one of SEQ ID NO: 15 and SEQ ID NO: 72;
the 3'UTR comprises the sequence set forth in SEQ ID NO: 12 or SEQ ID NO: 69 or a sequence having identity thereto any one of SEQ ID NO: 12 and SEQ ID NO: 69, and the 5'UTR comprises the sequence set forth in SEQ ID NO: 29 or SEQ ID NO: 86 or a sequence having identity to any one of SEQ ID NO: 29 and SEQ ID NO: 86;
the 3'UTR comprises the sequence set forth in SEQ ID NO: 13 or SEQ ID NO: 70 or a sequence having identity thereto any one of SEQ ID NO: 13 and SEQ ID NO: 70, and the 5'UTR comprises the sequence set forth in SEQ ID NO: 29 or SEQ ID NO: 86 or a sequence having identity to any one of SEQ ID NO: 29 and SEQ ID NO: 86;
the 3'UTR comprises the sequence set forth in SEQ ID NO: 14 or SEQ ID NO: 71 or a sequence having identity thereto any one of SEQ ID NO: 14 and SEQ ID NO: 71, and the 5'UTR comprises the sequence set forth in SEQ ID NO: 29 or SEQ ID NO: 86 or a sequence having identity to any one of SEQ ID NO: 29 and SEQ ID NO: 86;
the 3'UTR comprises the sequence set forth in SEQ ID NO: 12 or SEQ ID NO: 69 or a sequence having identity thereto any one of SEQ ID NO: 12 and SEQ ID NO: 69, and the 5'UTR comprises the sequence set forth in SEQ ID NO: 30 or SEQ ID NO: 87 or a sequence having identity to any one of SEQ ID NO: 30 and SEQ ID NO: 87;
the 3'UTR comprises the sequence set forth in SEQ ID NO: 13 or SEQ ID NO: 70 or a sequence having identity thereto any one of SEQ ID NO: 13 and SEQ ID NO: 70, and the 5'UTR comprises the sequence set forth in SEQ ID NO: 30 or SEQ ID NO: 87 or a sequence having identity to any one of SEQ ID NO: 30 and SEQ ID NO: 87;
the 3'UTR comprises the sequence set forth in SEQ ID NO: 14 or SEQ ID NO: 71 or a sequence having identity thereto any one of SEQ ID NO: 14 and SEQ ID NO: 71, and the 5'UTR comprises the sequence set forth in SEQ ID NO: 30 or SEQ ID NO: 87 or a sequence having identity to any one of SEQ ID NO: 30 and SEQ ID NO: 87;
the 3'UTR comprises the sequence set forth in SEQ ID NO: 12 or SEQ ID NO: 69 or a sequence having identity thereto any one of SEQ ID NO: 12 and SEQ ID NO: 69, and the 5'UTR comprises the sequence set forth in SEQ ID NO: 32 or SEQ ID NO: 89 or a sequence having identity to any one of SEQ ID NO: 32 and SEQ ID NO: 89;
the 3'UTR comprises the sequence set forth in SEQ ID NO: 13 or SEQ ID NO: 70 or a sequence having identity thereto any one of SEQ ID NO: 13 and SEQ ID NO: 70, and the 5'UTR comprises the sequence set forth in SEQ ID NO: 32 or SEQ ID NO: 89 or a sequence having identity to any one of SEQ ID NO: 32 and SEQ ID NO: 89; or
the 3'UTR comprises the sequence set forth in SEQ ID NO: 14 or SEQ ID NO: 71 or a sequence having identity thereto any one of SEQ ID NO: 14 and SEQ ID NO: 71, and the 5'UTR comprises the sequence set forth in SEQ ID NO: 32 or SEQ ID NO: 89 or a sequence having identity to any one of SEQ ID NO: 32 and SEQ ID NO: 89.

In the above embodiments, "having identity" encompasses having at least 80%, 85%, 90%, 95%, 96%, 97%, 98%, 99%, and 100% identity, and ranges between any two of the aforementioned numerical values, including integers and decimals, for example, "having at least 90% identity" or "having at least 95% identity".

In some embodiments, the 3'UTR and/or 5'UTR is a variant of the 3'UTR and/or 5'UTR described above, wherein the variant is, for example, a truncation or a nucleotide mutant, and the variant still maintains activity or functions similar to those of the aforementioned 3'UTR and/or 5'UTR of the present disclosure, e.g., still maintains a function of regulating expression of a protein from an encoding target gene of the ORF. In some embodiments, the aforementioned nucleic acid construct provided by the present disclosure also comprises:
(c) a polyadenylic acid (poly-A) tail.

In some specific embodiments, the poly-A tail in the nucleic acid construct is located downstream of the 3'UTR. In some specific embodiments, the poly-A tail in the nucleic acid construct is located at the 3' end of the 3'UTR. In some specific embodiments, the poly-A tail is at the 3' end of the nucleic acid construct. In some specific embodiments, the poly-A tail is at least about 50, 100, 150, 200, 300, 400, or 500 nucleotides in length. In some specific embodiments, the poly-A tail is selected from the group consisting of A120, A30L70, HGH polyA, SV40 polyA, BGH polyA, rbGlob polyA, and SV40late polyA. For example, the A30L70 is the sequence set forth in SEQ ID NO: 52.

In some embodiments, in the aforementioned nucleic acid construct provided by the present disclosure, the expressed target gene (i.e., open reading frame ORF) is hepatocyte growth factor (HGF), an antibody, or an antigen-binding fragment thereof, for example, an antibody that binds to a tumor antigen or an antigen-binding fragment thereof, or an antibody that binds to a viral antigen or an antigen-binding fragment thereof.

In some specific embodiments, the polypeptide or protein encoded by the ORF is a fluorescent protein or a luciferase, e.g., the sequence set forth in SEQ ID NO: 126.

In some specific embodiments, the HGF is human hepatocyte growth factor (hHGF).

In some specific embodiments, a sequence encoding hHGF comprises any one selected from the group consisting of the following 1)-3):
1) a nucleic acid sequence encoding the amino acid sequence set forth in SEQ ID NO: 109 or a codon-optimized nucleic acid sequence;
2) DNA sequences set forth in SEQ ID NOs: 110-113 or sequences having at least 80%, 85%, 90%, 95%, 96%, 97%, 98%, 99%, or 100% identity thereto; and
3) an RNA sequence set forth in any one of SEQ ID NOs: 128-131 or an RNA sequence having at least 80%, 85%, 90%, 95%, 96%, 97%, 98%, 99%, or 100% identity thereto.

In some specific embodiments, the nucleic acid construct expressing HGF as a target gene comprises the sequence set forth in any one of SEQ ID NOs: 115, 116, and 127 or a sequence having at least 80%, 85%, 90%, 95%, 96%, 97%, 98%, 99%, or 100% identity thereto.

In some specific embodiments, the antibody or the antigen-binding fragment thereof is an anti-PD-1 antibody or an antigen-binding fragment thereof.

In some specific embodiments, a sequence encoding the anti-PD-1 antibody or the antigen-binding fragment thereof comprises any one selected from the group consisting of the following 1)-4):
1) a nucleic acid sequence encoding a heavy chain amino acid sequence set forth in SEQ ID NO: 117 or a codon-optimized nucleic acid sequence, and a nucleic acid sequence encoding a light chain amino acid sequence set forth in SEQ ID NO: 118 or a codon-optimized nucleic acid sequence;
2) a nucleic acid sequence encoding a HCDR1, a HCDR2, and a HCDR3 in the heavy chain amino acid sequence set forth in SEQ ID NO: 117 or a codon-optimized nucleic acid sequence, and a nucleic acid sequence encoding a LCDR1, a LCDR2, and a LCDR3 in the light chain amino acid sequence set forth in SEQ ID NO: 118 or a codon-optimized nucleic acid sequence, the CDRs being defined according to the Kabat, IMGT, Chothia, AbM, or Contact numbering scheme, e.g., the Kabat numbering scheme;
3) a DNA sequence set forth in SEQ ID NO: 119 or a DNA sequence having at least 80%, 85%, 90%, 95%, 96%, 97%, 98%, 99%, or 100% identity thereto, and a DNA sequence set forth in SEQ ID NO: 120 or a DNA sequence having at least 80%, 85%, 90%, 95%, 96%, 97%, 98%, 99%, or 100% identity thereto; and
4) a DNA sequence set forth in SEQ ID NO: 121 or a DNA sequence having at least 80%, 85%, 90%, 95%, 96%, 97%, 98%, 99%, or 100% identity thereto, and a DNA sequence set forth in SEQ ID NO: 122 or a DNA sequence having at least 80%, 85%, 90%, 95%, 96%, 97%, 98%, 99%, or 100% identity thereto.

In some specific embodiments, the nucleic acid construct expressing the anti-PD-1 antibody or the antigen-binding fragment thereof comprises the sequences set forth in SEQ ID NOs: 124 and 125, or sequences having at least 80%, 85%, 90%, 95%, 96%, 97%, 98%, 99%, or 100% identity to SEQ ID NOs: 124 and 125.

In some embodiments, the aforementioned nucleic acid construct provided by the present disclosure is a DNA or RNA, e.g., an mRNA.

In some embodiments, in the 5'-to-3' direction, the aforementioned nucleic acid construct (DNA or RNA) provided by the present disclosure comprises any one of the following 1)-6):
1) a 5'UTR and an ORF;
2) an ORF and a 3'UTR;
3) a 5'UTR, an ORF, and a 3'UTR;
4) a 5'UTR, an ORF, a 3'UTR, and a poly-A tail;
5) a 5'UTR, an ORF, and a poly-A tail; and
6) an ORF, a 3'UTR, and a poly-A tail;
the 5'UTR and 3'UTR may be derived from the same gene or different genes.

In some embodiments, in the 5'-to-3' direction, the aforementioned nucleic acid construct (DNA) provided by the present disclosure comprises any one of the following 1)-4):
1) a 5'UTR and an ORF;
2) an ORF and a 3'UTR;
3) a 5'UTR, an ORF, and a 3'UTR; and
4) a 5'UTR, an ORF, a 3'UTR, and a poly-A tail;
the 5'UTR and 3'UTR may be derived from the same gene or different genes.

In some specific embodiments, the 5'UTR in 1), 3), and 4) comprises or is the nucleotide sequence set forth in any one of SEQ ID NOs: 15-47. In some specific embodiments, the ORF in 1)-4) comprises or is the nucleotide sequence set forth in SEQ ID NO: 110 or a codon-optimized nucleotide sequence thereof (e.g., SEQ ID NOs: 111-113). In some specific embodiments, the ORF in 1)-4) comprises or is the nucleotide sequences set forth in SEQ ID NOs: 119 and 120. In some specific embodiments, the 3'UTR in 2)-4) comprises or is the nucleotide sequence set forth in any one of SEQ ID NOs: 1-14.

In some embodiments, the aforementioned nucleic acid construct (RNA or mRNA) provided by the present disclosure also comprises:
(d) a 5' cap structure (5'Cap).

In some specific embodiments, the 5' cap structure in the RNA molecule is located upstream of the 5'UTR. In some embodiments, the 5' cap structure in the RNA molecule is located at the 5' end of the 5'UTR. In some embodiments, the 5' cap structure is a cap structure known to those skilled in the art, such as Cap0 (methylation of the first nucleobase, e.g., m⁷GpppN), Cap1 (additional methylation of the ribose of a nucleotide adjacent to m⁷GpppN, e.g., m⁷G(5')ppp(5')(2'OMeA)pG), Cap2 (additional methylation of the ribose of the 3rd nucleotide downstream of m⁷GpppN), Cap3 (additional methylation of the ribose of the 3rd nucleotide downstream of m⁷GpppN), Cap4 (additional methylation of the ribose of the 4th nucleotide downstream of m⁷GpppN), ARCA (anti-reverse cap analog), modified ARCA (e.g., phosphorothioate-modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In some specific embodiments, the 5' cap structure (e.g., Cap0 or Cap1) is formed using chemical RNA synthesis or RNA *in vitro* transcription (co-transcriptional capping).

In some specific embodiments, the 5'-cap structure (e.g., Cap0 or Cap1) is formed via enzymatic capping using a capping enzyme (e.g., vaccinia virus capping enzyme and/or cap-dependent 2'-O methyltransferase). In some embodiments, the 5' cap structure (Cap0 or Cap1) is added using an immobilized capping enzyme. The capping method and means in WO2016/193226 are incorporated herein by reference in their entirety.

In some specific embodiments, the 5'Cap is selected from the group consisting of ARCA, 3'-O-Me-m⁷G(5')ppp(5')G, m⁷G(5')ppp(5')(2'OMeA)pU, m⁷Gppp(A2'O-MOE)pG, m⁷G(5')ppp(5')(2'OMeA)pG, m⁷G(5')ppp(5')(2'OMeG)pG, m⁷(3'OMeG)(5')ppp(5')(2'OMeG)pG, and m⁷(3'OMeG)(5')ppp(5')(2'OMeA)pG. In some specific embodiments, the 5'Cap is 3'-O-Me-m⁷G(5')ppp(5')G or m⁷G(5')ppp(5')(2'OMeA)pG.

In some embodiments, in the 5'-to-3' direction, the aforementioned nucleic acid construct (RNA or mRNA) provided by the present disclosure comprises any one of the following 1)-5):
1) a 5'UTR and an ORF;
2) an ORF and a 3'UTR;
3) a 5'UTR, an ORF, and a 3'UTR;
4) a 5'UTR, an ORF, a 3'UTR, and a poly-A tail; and
5) a 5'Cap, a 5'UTR, an ORF, a 3'UTR, and a poly-A tail;
the 5'UTR and 3'UTR may be derived from the same gene or different genes.

In some specific embodiments, the 5'UTR in 1), 3), 4), and 5) comprises or is the nucleotide sequence set forth in any one of SEQ ID NOs: 72-104. In some specific embodiments, the ORF in 1)-5) comprises or is the nucleotide sequence set forth in any one of SEQ ID NOs: 128-131. In some specific embodiments, the 3'UTR in 2)-5) comprises or is the nucleotide sequence set forth in any one of SEQ ID NOs: 58-71. In some specific embodiments, structures include, but are not limited to, Cap0, Cap1 (e.g., m⁷G(5')ppp(5')(2'OMeA)pG), Cap2, Cap3, Cap4, and ARCA.

In some embodiments, the present disclosure provides a nucleic acid construct (DNA) comprising, in the 5'-to-3' direction, a 5'UTR, an ORF, and a 3'UTR in sequence; optionally, the nucleic acid construct may further comprise a poly-A tail in the 3' direction. In some embodiments, the 5'UTR comprises or is the nucleotide sequence set forth in any one of SEQ ID NOs: 15-47, the ORF comprises or is the nucleotide sequence set forth in SEQ ID NO: 110 or a codon-optimized nucleotide sequence thereof (e.g., SEQ ID NOs: 111-113), and the 3'UTR comprises or is the nucleotide sequence set forth in any one of SEQ ID NOs: 1-14. In some embodiments, the 5'UTR comprises or is the nucleotide sequence set forth in any one of SEQ ID NOs: 15-47, the ORF comprises or is the nucleotide sequences set forth in SEQ ID NOs: 119 and 120, and the 3'UTR comprises or is the nucleotide sequence set forth in any one of SEQ ID NOs: 1-14.

In some embodiments, the present disclosure provides a nucleic acid construct (RNA or mRNA) comprising, in the 5'-to-3' direction, a 5'UTR, an ORF, and a 3'UTR in sequence; optionally, the nucleic acid construct may further comprise a poly-A tail in the 3' direction. In some embodiments, the 5'UTR comprises or is the nucleotide sequence set forth in any one of SEQ ID NOs: 72-104, the ORF comprises or is the nucleotide sequence set forth in any one of SEQ ID NOs: 128-131, the 3'UTR comprises or is the nucleotide sequence set forth in any one of SEQ ID NOs: 58-71, and the poly-A tail comprises or is 120 contiguous adenylic acids or the nucleotide sequence set forth in SEQ ID NO: 52. In some specific embodiments, the nucleic acid construct comprises the nucleotide sequence set forth in SEQ ID NO: 115, 116, or 127.

In some embodiments, the 5'UTR comprises or is the nucleotide sequence set forth in any one of SEQ ID NOs: 72-104, the ORF comprises or is the nucleotide sequence set forth in any one of SEQ ID NOs: 121 and 122, the 3'UTR comprises or is the nucleotide sequence set forth in any one of SEQ ID NOs: 58-71, and the poly-A tail comprises or is 120 contiguous adenylic acids or the nucleotide sequence set forth in SEQ ID NO: 52. In some specific embodiments, the nucleic acid construct comprises the nucleotide sequence set forth in SEQ ID NO: 124 or 125.

In some embodiments, provided is any one of the aforementioned nucleic acid constructs (DNA or RNA molecules), wherein the UTR is used to increase an expression level of a protein expressed by the ORF. Illustratively, in some specific embodiments, the 5'UTR set forth in any one of the sequences SEQ ID NOs: 15-47 and 72-104 in the present disclosure has an increased expression level of a target protein expressed by the ORF as compared to the 5'UTR set forth in SEQ ID NO: 48 or 50. In some specific embodiments, the 3'UTR set forth in any one of the sequences SEQ ID NOs: 1-14 and 58-71 in the present disclosure has an increased expression level of a target protein expressed by the ORF as compared to the 5'UTR set forth in SEQ ID NO: 49 or 51. In some specific embodiments, a combination of the 5'UTR set forth in any one of the sequences SEQ ID NOs: 15-47 and 72-104 and the 3'UTR set forth in any one of the sequences SEQ ID NOs: 1-14 and 58-71 in the present disclosure has an increased expression level of a target protein expressed by the ORF as compared to a combination of the 5'UTR and 3'UTR set forth in SEQ ID NOs: 48 and 49. In some specific embodiments, a combination of the 5'UTR set forth in any one of the sequences SEQ ID NOs: 15-47 and 72-104 and the 3'UTR set forth in any one of the sequences SEQ ID NOs: 1-14 and 58-71 in the present disclosure has an increased expression level of a target protein expressed by the ORF as compared to a combination of the 5'UTR and 3'UTR set forth in SEQ ID NOs: 50 and 51.

In some embodiments, an expression level of a target protein expressed by the nucleic acid construct of the present disclosure is about 1 to about 20 times, e.g., about 1 time, about 1.1 times, about 1.2 times, about 1.3 times, about 1.4 times, about 1.5 times, about 1.6 times, about 1.7 times, about 1.8 times, about 1.9 times, about 2 times, about 2.1 times, about 2.3 times, about 2.5 times, about 2.8 times, about 3 times, about 3.2 times, about 3.4 times, about 3.8 times, about 4 times, about 4.5 times, about 5 times, about 5.2 times, about 5.5 times, about 5.8 times, about 6 times, about 7 times, about 8 times, about 10 times, about 12 times, or about 15 times, that by a BioN vector. In some specific embodiments, the nucleic acid construct of the present disclosure is an mRNA molecule expressing a target protein, and an expression level of the target protein expressed by the mRNA molecule is about 1 to about 20 times that by the BioN vector. In some specific embodiments, the BioN vector comprises a 5'UTR set forth in SEQ ID NO: 50 or 107 and/or a 3'UTR set forth in SEQ ID NO: 51 or 108.

In some embodiments, an expression level of a target protein expressed by the nucleic acid construct of the present disclosure is about 1 to about 20 times, e.g., about 1 time, about 1.1 times, about 1.2 times, about 1.3 times, about 1.4 times, about 1.5 times, about 1.6 times, about 1.7 times, about 1.8 times, about 1.9 times, about 2 times, about 2.1 times, about 2.3 times, about 2.5 times, about 2.8 times, about 3 times, about 3.2 times, about 3.4 times, about 3.8 times, about 4 times, about 4.5 times, about 5 times, about 5.2 times, about 5.5 times, about 5.8 times, about 6 times, about 7 times, about 8 times, about 10 times, about 12 times, or about 15 times, that by a Mod vector. In some specific embodiments, the nucleic acid construct of the present disclosure is an mRNA molecule expressing a target protein, and an expression level of the target protein expressed by the mRNA molecule is about 1 to about 20 times that by the BioN vector. In some specific embodiments, the Mod vector comprises a 5'UTR set forth in SEQ ID NO: 48 or 105 and/or a 3'UTR set forth in SEQ ID NO: 49 or 106.

In some embodiments, the nucleic acid construct of the present disclosure is delivered into a subject and expresses a target protein (e.g., hHGF protein) after about 0.5-1.5 h. In some embodiments, the nucleic acid construct of the present disclosure is delivered into a subject and achieves an expression peak at about 2 h-10 h (e.g., about 2 h, about 3 h, about 4 h, about 5 h, about 6 h, about 7 h, about 8 h, or about 10 h). In some specific embodiments, the nucleic acid construct of the present disclosure is an mRNA molecule expressing a target protein, e.g., the nucleotide construct is an mRNA molecule expressing hHGF protein.

In some embodiments, the nucleic acid construct of the present disclosure is delivered into a subject and has higher pharmacokinetic properties than the Collategene plasmid. In some specific embodiments, the nucleic acid construct is an mRNA molecule, the mRNA molecule having improved pharmacokinetic properties (e.g., Cmax, AUC_{0-inf}, and MRT_{0-inf}) as compared to the Collategene plasmid.

In some embodiments, the nucleic acid construct of the present disclosure (e.g., m-A16-B12 (hHGF) expressing hHGF protein) is capable of improving a blood perfusion ratio in an ischemic lower limb of a subject. Illustratively, injecting 50 ng/animal and 500 ng/animal of m-A16-B12 (hHGF) into mice with ischemic lower limbs can achieve improved blood perfusion ratios, wherein injecting 500 ng/animal of m-A16-B12 (hHGF) into mice with ischemic lower limbs can restore blood perfusion to about 90% or higher.

In some embodiments, the nucleic acid construct of the present disclosure (e.g., m-A16-B12 (hHGF) expressing hHGF protein) is capable of ameliorating ischemic necrosis in a subject with induced ischemia in a lower limb. Illustratively, mice with ischemic lower limbs injected with 50 ng/animal and 500 ng/animal of m-A16-B12 (hHGF) can all maintain good lower limb integrity and do not develop necrosis in the lower limbs.

In some embodiments, the nucleic acid construct of the present disclosure (e.g., m-A16-B12 (hHGF) expressing hHGF protein) can promote angiogenesis in ischemic lower limb muscles of each group. Illustratively, both treatment with 50 ng/animal of m-A16-B12 (hHGF) and treatment with 500 ng/animal of m-A16-B12 (hHGF) can significantly promote angiogenesis in ischemic lower limb muscles of each group, and the number of new blood vessels is statistically significantly different (p < 0.05) from that of the PBS group.

In some embodiments, the nucleic acid construct of the present disclosure (e.g., m-A16-B12 (hHGF) expressing hHGF protein) is capable of improving wound healing in a diabetes subject. Illustratively, in a Db/Db diabetes mouse model injected with 50 ng/animal, 200 ng/animal, and 500 ng/animal of m-A16-B12 (hHGF), the mice's wounds all well healed, wherein at day 14, the 50 ng/animal dose of m-A16-B12 (hHGF) achieved a wound healing rate of 66%, and the 200 ng/animal and 500 ng/animal doses of m-A16-B12 (hHGF) can achieve 100% wound healing.

The present disclosure provides an mRNA with a 5'UTR and a 3'UTR of new structures that reduce early degradation of the mRNA or stabilize degradation but do not compromise more or enhance protein translation efficiency. The mRNA is more stable and can be applied to gene therapy and genetic vaccination. Additionally, the present disclosure provides an mRNA capable of expressing human hepatocyte growth factor (hHGF) and a lipid nanoparticle (LNP) delivery system thereof, which enable efficient and rapid *in vivo* conversion of exogenous hHGF protein, have the advantages of involving no risk of integration and being easy to scale up industrially, are a more ideal treatment regimen than naked plasmids, and can be used as a gene therapy drug for a variety of diseases such as CLI and DFU.

### Polynucleotide

The present disclosure also provides an isolated polynucleotide comprising (a) an open reading frame (ORF). Illustratively, the open reading frame (ORF) encodes hepatocyte growth factor (HGF), e.g., human hepatocyte growth factor (hHGF).

In some embodiments, a sequence encoding the hHGF comprises any one selected from the group consisting of the following 1)-3):
1) a nucleic acid sequence encoding the amino acid sequence set forth in SEQ ID NO: 109 or a codon-optimized sequence thereof;
2) a DNA sequence set forth in any one of SEQ ID NOs: 110-113 or a DNA sequence having at least 80%, 85%, 90%, 95%, 96%, 97%, 98%, 99%, or 100% identity thereto; and
3) an RNA sequence set forth in any one of SEQ ID NOs: 128-131 or an RNA sequence having at least 80%, 85%, 90%, 95%, 96%, 97%, 98%, 99%, or 100% identity thereto.

In some specific embodiments, the 5' end of the polynucleotide may comprise any of the aforementioned 5'UTRs provided by the present disclosure, and/or the 3' end of the polynucleotide may comprise any of the aforementioned 3'UTRs provided by the present disclosure.

In some specific embodiments, the 5' end of the polynucleotide may comprise any of the aforementioned 5'Caps provided by the present disclosure, and/or the 3' end of the polynucleotide may comprise any of the aforementioned poly-A tails provided by the present disclosure.

In some embodiments, the polynucleotide is an RNA, e.g., an mRNA.

### Modification

To further improve the stability in connection with protein expression of the RNA or polynucleotide molecule of the present disclosure, the RNA or polynucleotide may further comprise one or more modifications (including chemical modifications), such as backbone modifications, sugar modifications, base modifications, and/or lipid modifications. In some embodiments, the RNA or polynucleotide is uniformly modified to a particular modification (e.g., complete modification throughout the sequence). For example, the RNA may be uniformly modified with pseudouridines (e.g., N1-methylpseudouridine) such that each U in the sequence is a pseudouridine.

Backbone modifications in connection with the present disclosure refer to chemical modifications of the phosphate of the backbone of the nucleotides that the RNA or polynucleotide of the present disclosure comprises. In some embodiments, the backbone modifications include, but are not limited to, complete substitution of unmodified phosphate moieties in the backbone with modified phosphates; for example, backbone phosphate groups may be modified by substituting one or more oxygen atoms with a different substituent. In some embodiments, the modified phosphates include, but are not limited to, phosphorothioates, phosphoroselenates, boranophosphates, boranophosphates, hydrogen phosphonates, phosphoramidates, alkyl or aryl phosphonates, and phosphotriesters.

Sugar modifications in connection with the present disclosure refer to chemical modifications of the sugar of the nucleotides that the RNA or polynucleotide of the present disclosure comprises. In some embodiments, the sugar modifications include, but are not limited to, modifications or replacements of the 2' hydroxy (OH) of the RNA molecule with many different "oxy" or "deoxy" substituents. In some embodiments, the "oxy" modifications include, but are not limited to, substitution modifications with alkoxy, aryloxy, polyethylene glycol (PEG), and the like. In some embodiments, "deoxy" modifications include, but are not limited to, hydrogen and amino (e.g., NH2, alkylamino, dialkylamino, heterocyclyl, arylamino, diarylamino, heteroarylamino, diheteroarylamino, or amino acid) modifications.

Base modifications in connection with the present disclosure refer to chemical modifications of the base moiety of the nucleotides that the RNA or polynucleotide of the present disclosure comprises. In some embodiments, the base modifications include modifications to adenine, guanine, cytosine, and uracil in nucleotides. For example, the nucleosides and nucleotides described herein may be chemically modified on the surface of the major groove. In some embodiments, chemical modifications to the major groove may include amino, thiol, alkyl, or halogen groups. In some embodiments, the base modifications include, but are not limited to, modifications with pseudouridine, 1-methyl-pseudouridine, 5-azacytidine, 5-methylcytosine-5'-triphosphate, or 2-methoxyadenine. In some embodiments, the base modifications are pseudouridine modifications. For example, the RNA may be uniformly modified with pseudouridines such that each U in the sequence is a pseudouridine.

Lipid modifications in connection with the present disclosure mean that the RNA or polynucleotide of the present disclosure comprises lipid modifications. In some embodiments, the lipid modifications include, but are not limited to, covalent attachment of the RNA or polynucleotide of the present disclosure to at least one adapter, and covalent attachment of the corresponding adapter to at least one lipid. In some embodiments, the lipid modifications include, but are not limited to, covalent attachment of the RNA or polynucleotide of the present disclosure to at least one lipid (no adapter).

### UTRs

UTRs (5'UTR and/or 3'UTR) may be provided as a flanking region to the nucleic acid construct, RNA, or polynucleotide molecule of the present disclosure. UTRs may be homologous or heterologous with coding regions in the nucleic acid construct, RNA, or polynucleotide molecule of the present disclosure. The flanking region may comprise one or more 5'UTRs and/or 3'UTRs, and the UTRs may be identical or different sequences. Any portion of the flanking region may be codon-optimized. Before and/or after codon optimization, any portion of the flanking region may independently comprise one or more different structural or chemical modifications.

To alter one or more properties of the nucleic acid construct, RNA, or polynucleotide of the present disclosure, UTRs heterologous with the ORF of the present disclosure are introduced or engineering-synthesized into the nucleic acid construct, RNA, or polynucleotide of the present disclosure. The recombinant nucleic acid construct, RNA, or polynucleotide is then administered to a cell, tissue, or organism, and the results, such as protein levels, localization, and/or half-life, are measured to assess the beneficial effects of the heterologous UTRs on the RNA or polynucleotide of the present disclosure. In some embodiments, the UTRs include a wild UTR or a variant thereof, the UTR variant comprising the addition or removal of one or more nucleotides, including A, T, C, or G, to or from an end. In some embodiments, the UTR variant also comprises codon optimization or modification performed in any way. In some embodiments, the UTR variant also comprises the derivative sequence according to any embodiment of the present disclosure; for example, on the basis of a natural UTR sequence, some nucleotides are point-mutated, and the post-mutation variant causes the expression level and stability of the target gene to remain unchanged or be improved. The measurement methods for the expression level and stability of the target gene are conventional in the art, such as the measurement methods in Examples 3 and 4 of the present disclosure.

### Vector

The present disclosure also provides a vector comprising the nucleic acid construct, RNA, or polynucleotide according to any one of the foregoing. The nucleic acid construct, RNA, or polynucleotide may be present in the vector and/or may be part of the vector, the vector being, for example, a plasmid, cosmid, YAC, or viral vector. The vector may be an expression vector, i.e., a vector capable of providing for the expression of a polypeptide encoded by the nucleic acid construct, RNA, or polynucleotide. The expression vector generally comprises at least one of the nucleic acids of the present disclosure, which is operatively linked to one or more suitable expression regulatory elements (e.g., promoters and terminators). The selection of the elements and their sequences for expression in a particular host is within the knowledge of those skilled in the art. Regulatory elements and other elements useful or necessary for expression of the encoded polypeptide of the present disclosure include, for example, promoters, terminators, selection labels, leader sequences, and reporter genes. In some embodiments, the vector is a therapeutic vector capable of expressing the target gene of the present disclosure (e.g., HGF), such as a plasmid (e.g., a naked plasmid), an adenovirus vector, an adeno-associated virus vector, and a lentiviral vector.

The nucleic acid construct of the present disclosure may be prepared or obtained by known means (e.g., by automatic DNA synthesis and/or recombinant DNA technology) based on nucleotide sequence information of the present disclosure, and/or may be isolated from a suitable natural source.

In some embodiments, the vector of the present disclosure also comprises a promoter; for example, the promoter is at the 5' end of the 5'UTR of the nucleic acid construct, and for example, the promoter is a T7 promoter, a T7 lac promoter, a Tac promoter, a Lac promoter, or a Trp promoter.

### Host Cell

The present disclosure also provides a host cell comprising the nucleic acid construct, RNA, or polynucleotide according to any one of the foregoing. In some embodiments, the cell is capable of expressing one or more polypeptides encoded by the nucleic acid construct, RNA, or polynucleotide of the present disclosure. In some embodiments, the host cell is a bacterial cell, a fungal cell, or a mammalian cell.

Bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, e.g., cells of the species *Trichoderma, Neurospora,* and *Aspergillus*; or cells of the species *Saccharomyces* (e.g., *Saccharomyces cerevisiae), Schizosaccharomyces* (e.g., *Schizosaccharomyces pombe), Pichia* (e.g., *Pichia pastoris* and *Pichia methanolica),* and *HansenuLa.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

### Production or Preparation Method

The present disclosure provides a method for preparing the nucleic acid construct, RNA, or polynucleotide of the present disclosure, and a method for preparing the encoded polypeptide thereof.

Methods and reagents for preparation and production of the nucleic acid construct, RNA, or polynucleotide and the encoded polypeptide thereof, e.g., specific suitable vectors, transformation or transfection methods, selection labels, methods for inducing protein expression, and culture conditions, are known in the art. Similarly, protein isolation and purification techniques suitable for use in the method for manufacturing the encoded polypeptide of the present disclosure are well known to those skilled in the art.

In some embodiments, the method for preparing the nucleic acid construct or polynucleotide comprises culturing the aforementioned host cell and collecting the produced nucleic acid construct or polynucleotide from the culture. The nucleic acid construct or polynucleotide of the present disclosure and the encoded polypeptide thereof may also be obtained by other production methods known in the art, such as chemical synthesis, including solid-phase, or liquid-phase synthesis.

In some embodiments, a method for preparing an RNA molecule comprises: preparing a nucleic acid construct or a vector, and then using the nucleic acid construct or vector to perform reverse transcription to obtain the RNA molecule. In some specific embodiments, the method also comprises adding a 5'Cap to the 5' end of the RNA molecule.

In some embodiments, the RNA may further comprise one or more modifications (including chemical modifications), such as backbone modifications, sugar modifications, base modifications, and/or lipid modifications. In some embodiments, the RNA or polynucleotide is uniformly modified to a particular modification (e.g., complete modification throughout the sequence). For example, the RNA may be uniformly modified with pseudouridines (e.g., N1-methylpseudouridine) such that each U in the sequence is a pseudouridine (e.g., N1-methylpseudouridine).

### Delivery Vehicle

The present disclosure also provides a delivery vehicle comprising the nucleic acid construct according to any one of the foregoing or any of the aforementioned RNA molecules, polynucleotides, or vectors, wherein the delivery vehicle is a cationic lipid delivery particle. In some embodiments, the particle is a nanoparticle. In some embodiments, the delivery vehicle is a lipid nanoparticle. The nucleic acid construct, RNA molecule, polynucleotide, or vector of the present disclosure may be delivered into cells and/or *in vivo* using any type of lipid nanoparticle in the art; illustratively, lipid nanoparticles include, but are not limited to, lipid particles disclosed in WO2017075531, WO2018081480A1, WO2017049245A2, WO2017099823A1, WO2022245888A1, WO2022150717A1, CN101291653A, CN102119217A, WO2011000107A1, CN107028886A, and US9868692B2, which are incorporated herein by reference in their entirety.

In some embodiments, the delivery vehicle comprises a lipid nanoparticle shown in US9868692B2, for example, an ionizable lipid represented by formula B (also referred to as SM-102). In some embodiments, the delivery vehicle comprises a lipid nanoparticle, a phospholipid, a structural lipid, and/or a PEG lipid. Illustratively, the delivery vehicle comprises lipid components comprising about 50 mol% of the ionizable lipid (e.g., SM-102), about 10 mol% of a phospholipid (e.g., DSPC), about 38.5 mol% of a structural lipid (e.g., cholesterol), and about 1.5 mol% of a PEG lipid (e.g., PEG-DMG).

### Pharmaceutical Composition

The present disclosure also provides a pharmaceutical composition comprising any of the aforementioned nucleic acid constructs, any of the aforementioned RNA molecules, any of the aforementioned polynucleotides, any of the aforementioned vectors, and/or any of the aforementioned delivery vehicles of the present disclosure, and a pharmaceutically acceptable carrier, diluent, or excipient; specifically, the pharmaceutical composition is a solid formulation, an injection, a topical formulation, a spray, a liquid formulation, or a composite formulation.

### Product or Kit

The present disclosure provides a product or kit comprising the nucleic acid construct according to any one of the foregoing, any of the aforementioned RNA molecules, the polynucleotide according to any one of the foregoing, the vaccine according to any one of the foregoing, the vector according to any one of the foregoing, the delivery vehicle according to any one of the foregoing, and/or the pharmaceutical composition according to any one of the foregoing. The kit may be used to provide relevant detection or diagnostic use.

### Method for Treating and/or Preventing Disease and Pharmaceutical Use

The present disclosure also provides a method for treating or preventing a disease, the method comprising administering to a subject in need thereof a therapeutically and/or prophylactically effective amount of any of the aforementioned nucleic acid constructs, any of the aforementioned RNA molecules, any of the aforementioned polynucleotides, any of the aforementioned vectors, any of the aforementioned delivery vehicles, any of the aforementioned pharmaceutical compositions, and/or any of the aforementioned products or kits.

The present disclosure also provides use of any of the aforementioned nucleic acid constructs, any of the aforementioned RNA molecules, any of the aforementioned polynucleotides, any of the aforementioned vectors, any of the aforementioned delivery vehicles, any of the aforementioned pharmaceutical compositions, and/or any of the aforementioned products or kits for preparing a medicament for treating and/or preventing a disease.

In some embodiments, the disease is a disease in which a subject may benefit from the activity of natural hHGF.

In some embodiments, the disease is selected from the group consisting of ischemic diseases, metabolic syndrome, diabetes and complications thereof, restenosis, and nerve injury. In some specific embodiments, the disease is an ischemic disease, e.g., coronary artery disease (CAD) or peripheral artery disease (PAD), e.g., myocardial infarction or lower limb arterial ischemia. In some specific embodiments, the disease is diabetes or a complication thereof, e.g., diabetic peripheral neuropathy. In some specific embodiments, the disease is restenosis, e.g., post-operative restenosis and post-perfusion restenosis. In some specific embodiments, the disease is nerve injury, e.g., a neurodegenerative disease (e.g., amyotrophic lateral sclerosis (ALS), Parkinson's disease, or dementia), traumatic nerve injury, or peripheral neuropathy (e.g., diabetic peripheral neuropathy). In some specific embodiments, the disease is selected from the group consisting of lower limb arterial ischemia, myocardial infarction, and diabetic peripheral neuropathy.

In some embodiments, the disease is selected from the group consisting of peripheral artery disease (PAD), atherosclerosis obliterans (ASO), diabetic artery obliterans (DAO), and thrombosis angiitis obliterans (TAO). In some embodiments, the disease is selected from the group consisting of limb ischemia (e.g., lower limb ischemia or critical limb ischemia (CLI)) and diabetic foot ulcer (DFU).

In some embodiments, provided is a method for promoting growth and/or migration of endothelial cells (e.g., umbilical vein endothelial cells), the method comprising administering to an endothelial cell or a subject in need thereof an effective amount of any of the aforementioned polynucleotides, any of the aforementioned vectors, any of the aforementioned delivery vehicles, any of the aforementioned pharmaceutical compositions, and/or any of the aforementioned products or kits of the present disclosure. Also provided is related pharmaceutical use for preparing a medicament for promoting growth and/or migration of endothelial cells.

In some embodiments, provided is a method for promoting angiogenesis, the method comprising administering to an endothelial cell or a subject in need thereof an effective amount of any of the aforementioned polynucleotides, any of the aforementioned vectors, any of the aforementioned delivery vehicles, any of the aforementioned pharmaceutical compositions, and/or any of the aforementioned products or kits of the present disclosure. In some embodiments, the angiogenesis is microangiogenesis. Also provided is related pharmaceutical use for preparing a medicament for promoting angiogenesis (e.g., microangiogenesis).

### Beneficial Effects

The nucleic acid construct, vector, delivery vehicle, pharmaceutical composition, and product or kit provided by the present disclosure, in which the target gene is HGF, can be advantageously applied to one or more of the following: promoting growth and/or migration of endothelial cells; promoting angiogenesis (e.g., microangiogenesis); treating an ischemic disease, e.g., coronary artery disease (CAD) or peripheral artery disease (PAD), e.g., limb ischemia (e.g., lower limb ischemia or critical limb ischemia); treating metabolic syndrome and diabetes and complications thereof (e.g., diabetic peripheral neuropathy and diabetic foot ulcer); inhibiting restenosis; and promoting repairs to nerve injury (e.g., neurodegenerative diseases, traumatic nerve injury, and peripheral neuropathy).

### BRIEF DESCRIPTION OF THE DRAWINGS

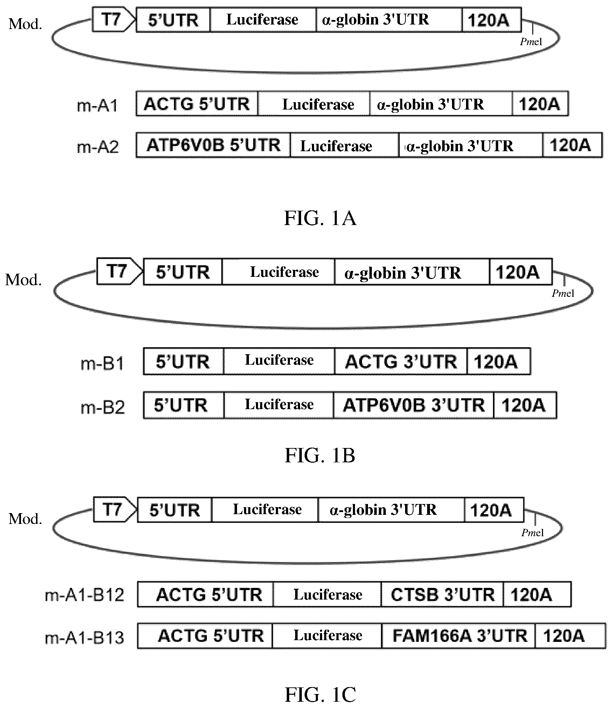

FIGs. 1A to 1C are schematic diagrams of vector construction. FIG. 1A is a schematic diagram of the construction of a 5'UTR element screening vector, with an mRNA sequence containing a 5'UTR element and a 3'UTR element of company Moderna, i.e., 5'UTR-Fluc-α globin 3'UTR-120A (simply referred to as Mod.), as a control, where the 5'-UTR is an artificial nucleic acid sequence, and the 3'UTR is derived from the mRNA of human α globin. In the construction of the 5'UTR screening vector, the 5'UTR region was replaced through a suitable restriction enzyme cutting site. *Pme*I is a linearized restriction enzyme cutting site. FIG. 1B is a schematic diagram of the construction of a 3'UTR element screening vector, with Mod. as a control. In the construction of the 3'UTR screening vector, the 3'UTR region was replaced through a suitable restriction enzyme cutting site. FIG. 1C is a schematic diagram of the construction of a 5'UTR and 3'UTR element combination screening vector, with Mod. as a control. In the construction of the 5'UTR and 3'UTR element combination screening vector, the 5'UTR and 3'UTR regions were replaced through suitable restriction enzyme cutting sites or whole-gene synthesis was performed.

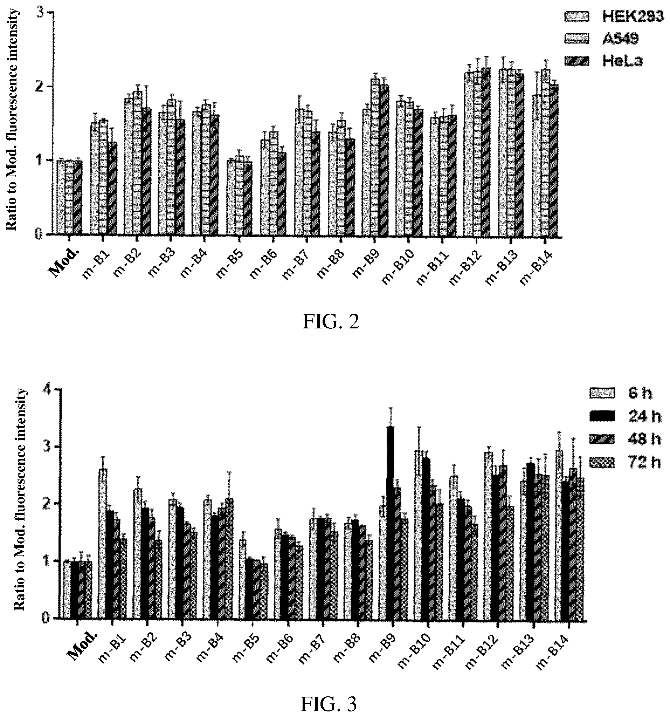

FIG. 2 shows the results of the assessment of the effects of different 3'UTR elements of the present disclosure in different cell lines. mRNAs containing different 3'UTR elements were transfected into HEK293, HeLa, and A549 cells, luciferase expression was measured at 24 h post-transfection, and the influence of the 3'UTR sequences on protein expression level was assessed. Mod. was used as a control, and the expression level of Mod. was set to 1. The results show that in different cell lines, there is a consistency in the influence of the 3'UTR elements on protein expression level.
FIG. 3 shows the influence of different 3'UTR elements of the present disclosure on mRNA expression efficiency. mRNAs containing different 3'UTR elements were transduced into HEK293 cells by lipofection, and luciferase expression levels were measured at 6 h, 24 h, 48 h, and 72 h post-transfection. Mod. was used as a control, and its expression level was set to 1. The results show that the 3'UTR elements numbered B9, B10, B12, B13, and B14 can significantly increase the protein expression level.

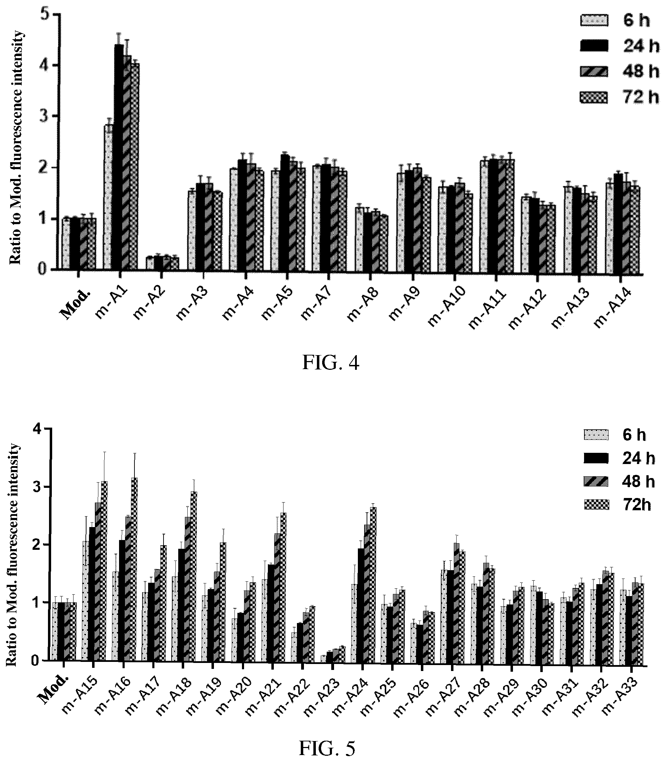

FIG. 4 shows the influence of different 5'UTR elements of the present disclosure on mRNA expression efficiency. mRNAs containing different 5'UTR elements were transduced into HEK293 cells by lipofection, and luciferase expression levels were measured at 6 h, 24 h, 48 h, and 72 h post-transfection. Mod. was used as a control, and its expression level was set to 1. The results show that the 5'UTR elements numbered A1, A3-A7, and A9-A14 can significantly increase the protein expression level.
FIG. 5 shows the influence of different 5'UTR elements of the present disclosure on mRNA expression efficiency. mRNAs containing different 5'UTR elements were transduced into HEK293 cells by lipofection, and luciferase expression levels were measured at 6 h, 24 h, 48 h, and 72 h post-transfection. Mod. was used as a control, and its expression level was set to 1. The results show that the 5'UTR elements numbered A15, A16, A18-A19, A21, A24, A27, A28, and A30-A33 can significantly increase the protein expression level.

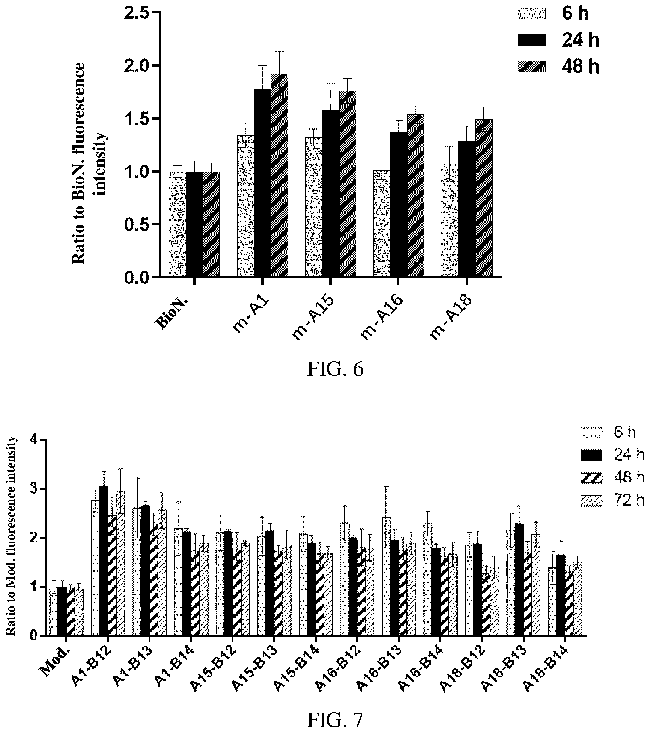

FIG. 6 shows the influence of different 5'UTR elements of the present disclosure on mRNA expression efficiency. mRNAs containing different 5'UTR elements were transduced into HEK293 cells by lipofection, and luciferase expression levels were measured at 6 h, 24 h, and 48 h post-transfection. BioN. was used as a control, and its expression level was set to 1. The results show that the 5'UTR elements numbered A1, A15, A16, and A18 can all significantly increase the protein expression level as compared to the control nucleic acid molecule.
FIG. 7 shows the influence of UTR element combinations of the present disclosure on mRNA expression efficiency. mRNAs containing different 5'UTR and 3'UTR elements were transduced into HEK293 cells by lipofection, and luciferase expression levels were measured 6 h, 24 h, 48 h, and 72 h post-transfection. Mod. was used as a control, and its expression level was set to 1. The results show that combinations of the 5'UTR elements numbered A1, A15, A16, and A18 and the 3'UTR elements numbered B12, B13, and B14 can all significantly increase the protein expression level.

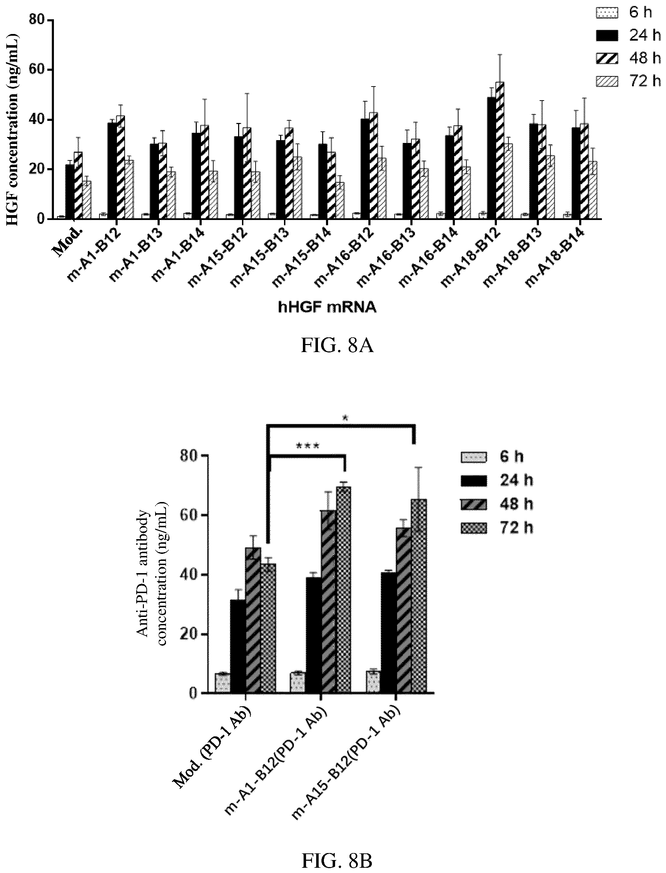

FIGs. 8A to 8B show the influence of the UTR element combinations of the present disclosure on the expression efficiency of different target proteins. FIG. 8A shows the regulation of the hHGF expression level in HEK293 cells by mRNAs containing 5'UTR and 3'UTR elements obtained by screening in the present disclosure. The results show that compared to the control Mod., combinations of the 5'UTR elements numbered A1, A15, A16, and A18 and the 3'UTR elements numbered B12, B13, and B14 can significantly increase the hHGF expression level. FIG. 8B shows the regulation of the anti-PD-1 antibody expression level in HEK293 cells by mRNAs containing 5'UTR and 3'UTR elements obtained by screening in the present disclosure. The results show that compared to the control Mod., the UTR combinations A1-B12 and A15-B12 can significantly increase the anti-PD-1 antibody expression level.

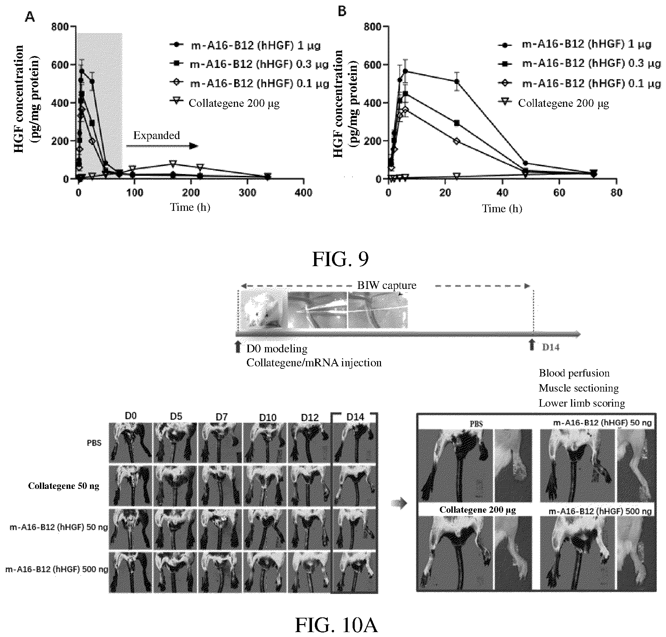

FIG. 9 is a schematic diagram showing experimental results of changes in the hHGF protein expression levels of the m-A16-B12 (hHGF) of the present disclosure and a control plasmid in mouse muscle tissue over time. The results show that both m-A16-B12 (hHGF) injection and Collategene plasmid injection can achieve effective expression of hHGF protein: only 1 h after intramuscular injection of m-A16-B12 (hHGF), hHGF was expressed; at 6 h post-injection, expression peaks of hHGF were achieved in a dose-dependent manner, and a mere low dose of m-A16-B12 (hHGF) can achieve an expression AUC_{inf(hr*pg/mg protein)} comparable to that of Collategene.

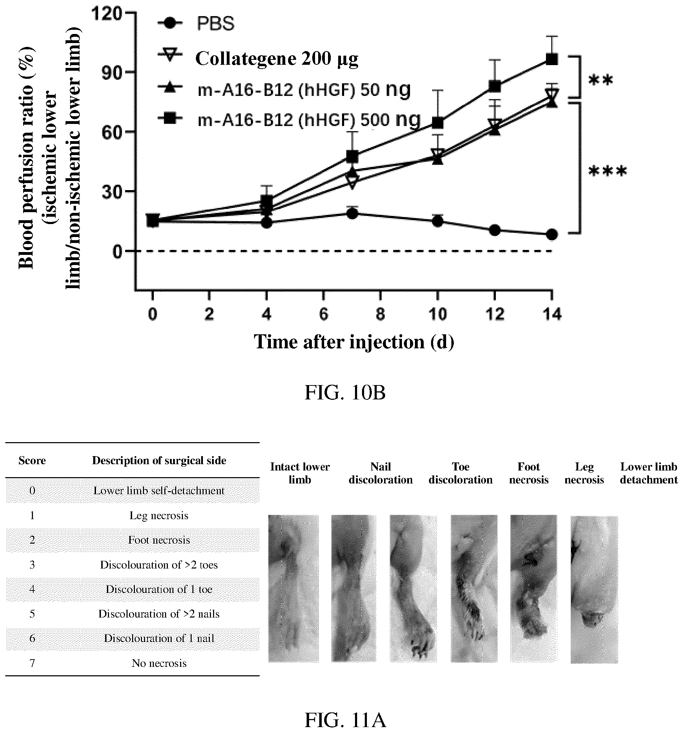

FIGs. 10A to 10B show the therapeutic effects of the m-A16-B12 (hHGF) of the present disclosure and a control plasmid on a lower limb ischemia mouse model. FIG. 10A is a schematic diagram of a study protocol and photographs of experimental results, showing the influence of m-A16-B12 (hHGF) and the Collategene plasmid on blood perfusion in a lower limb ischemia mouse model. FIG. 10B shows statistical results of blood perfusion ratios of m-A16-B12 (hHGF) and the Collategene plasmid in a lower limb ischemia mouse model. The results show that in the case of treatment with 50 ng/animal and 500 ng/animal of m-A16-B12 (hHGF) and 200 ng/animal of the Collategene naked plasmid, the ischemic lower limb blood perfusion ratio of each mouse was significantly improved relative to the control group, and the ischemic lower limb blood perfusion ratio gradually recovered over time; the 50 ng/animal m-A16-B12 (hHGF) group exhibited a blood flow recovery effect similar to that of the 200 ng/animal Collategene naked plasmid group; the 500 ng/animal m-A16-B12 (hHGF) group exhibited a blood flow recovery effect significantly better than that of the 200 ng/animal Collategene naked plasmid group: 500 ng/animal of m-A16-B12 (hHGF) can restore blood perfusion to 90% or higher.

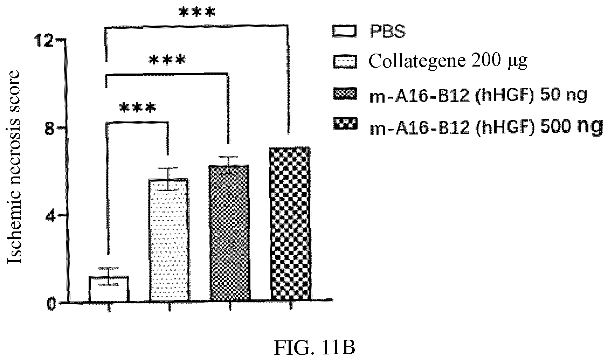

FIGs. 11A to 11B show the therapeutic effects of the m-A16-B12 (hHGF) of the present disclosure and a control plasmid on a lower limb ischemia mouse model. FIG. 11A shows scoring criteria for different degrees of lower limb necrosis in a lower limb ischemia mouse model and representative photographs. FIG. 11B shows statistical results of the influence of m-A16-B12 (hHGF) and the Collategene plasmid on lower limb necrosis in a lower limb ischemia mouse model. The experimental results show that in the m-A16-B12 (hHGF) and Collategene naked plasmid injection treatment groups, the mice can all maintain good lower limb integrity and did not develop necrosis in the lower limbs.

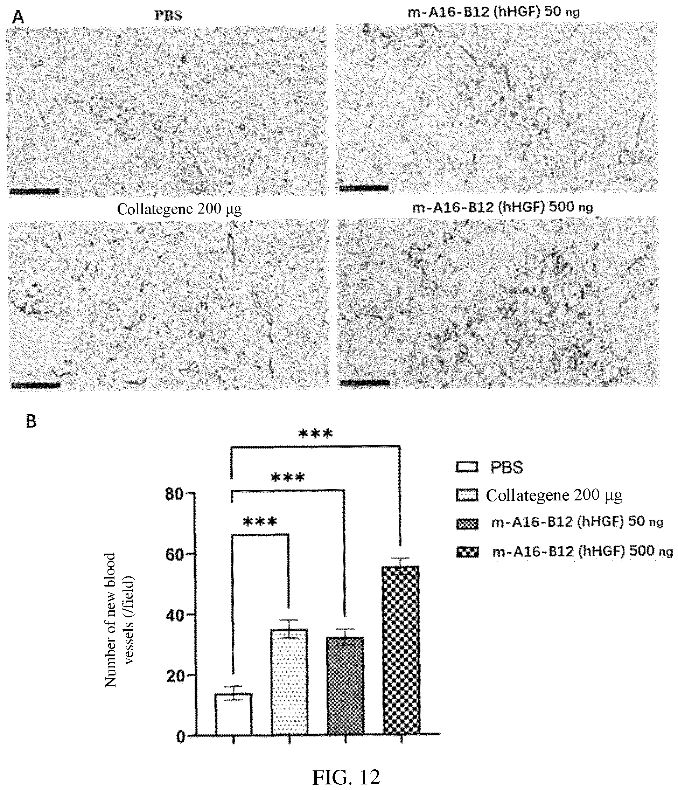

FIG. 12 shows representative photographs and statistical results for the influence of the m-A16-B12 (hHGF) of the present disclosure and a control plasmid on angiogenesis in a lower limb ischemia mouse model. The results show that in the case of treatment with 50 ng/animal and 500 ng/animal of m-A16-B12 (hHGF) and 200 ng/animal of the Collategene naked plasmid, angiogenesis in the muscles of ischemic lower limbs can be significantly promoted.

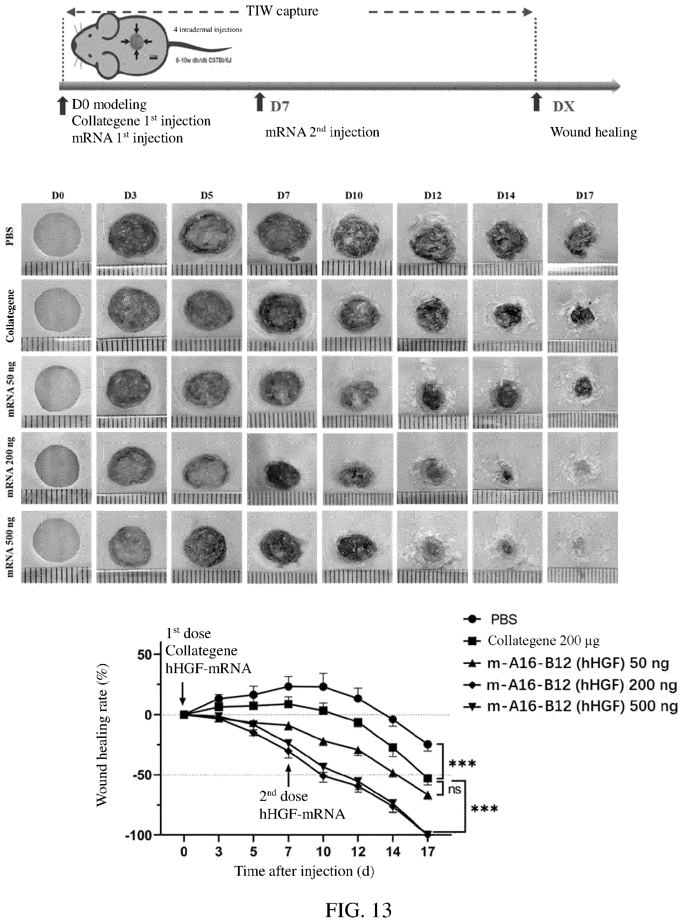

FIG. 13 shows a treatment regimen, representative photographs, and statistical results for the influence of the m-A16-B12 (hHGF) of the present disclosure and a control plasmid on wounds in a Db/Db mouse model of full-thickness skin injury. The results show that under treatment with 50 ng/animal, 200 ng/animal, and 500 ng/animal of m-A16-B12 (hHGF) and 200 µg/animal of the Collategene naked plasmid, the mice's wounds all well healed as compared to the control group, and the wound healing promoting effect of m-A16-B12 (hHGF) was significantly better than that of 200 µg/animal of the Collategene naked plasmid.

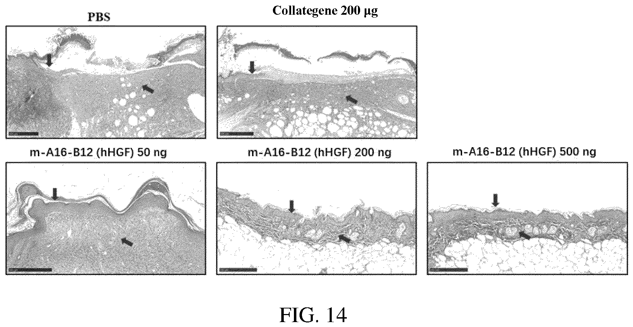

FIG. 14 shows representative photographs of the influence of the m-A16-B12 (hHGF) of the present disclosure and a control plasmid on tissue remodeling in a Db/Db mouse model of full-thickness skin injury. The results show that under treatment with m-A16-B12 (hHGF), full epithelial coverage of the wound epithelium was completed in each group of mice, and tissue remodeling was completed. By contrast, in the control group and the 200 µg/animal Collategene naked plasmid group, epithelial re-coverage was not completed, collagen abnormally proliferated at the wounds, and the structures were disordered.

### DETAILED DESCRIPTION

### Terminology

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. The "HGF" of the present disclosure encompasses natural or wild-type HGF and homologs thereof from different species, including biologically active, naturally occurring human hepatocyte growth factor (hHGF) and variants thereof. The amino acid sequence of natural or wild-type hHGF can be conveniently obtained from various public databases (e.g., the GenBank database). For example, the amino acid sequence of natural hHGF can be found under accession number NP_000592.3 in the GenBank database. As another example, the present disclosure provides an hHGF with an amino acid sequence set forth in SEQ ID NO: 109, a DNA sequence set forth in SEQ ID NO: 110, and a codon-optimized sequence set forth in SEQ ID NOs: 111-113. HGF has a variety of biological activities, including, but not limited to, one or more of the following activities: (1) promoting growth and/or migration of endothelial cells; (2) promoting angiogenesis (e.g., microangiogenesis); and/or (3) promoting repairs to nerve injury (e.g., peripheral neuropathy, e.g., diabetic peripheral neuropathy). Therefore, HGF may have prospects for application to multiple aspects, including, but not limited to, (1) promoting growth and/or migration of endothelial cells; (2) promoting angiogenesis (e.g., microangiogenesis); (3) treating an ischemic disease, e.g., coronary artery disease (CAD) or peripheral artery disease (PAD), e.g., lower limb arterial ischemia; (4) treating metabolic syndrome and diabetes and complications thereof (e.g., diabetic peripheral neuropathy); (5) inhibiting restenosis; and (6) promoting repairs to nerve injury (e.g., neurodegenerative diseases, traumatic nerve injury, and peripheral neuropathy). Therefore, examples of the term "disease that may benefit from the activity of natural hHGF" include, but are not limited to, the diseases described above, for example, ischemic diseases, metabolic syndrome, diabetes and complications thereof, restenosis, and nerve injury.

The "nucleic acid" or "nucleotide" includes RNA, DNA, and cDNA molecules. It will be appreciated that due to the degeneracy of the genetic codes, a large number of nucleotide sequences encoding a given protein may be generated. The term nucleic acid can be used interchangeably with the term "polynucleotide". The "oligonucleotide" refers to a short nucleic acid molecule. The "primer" is an oligonucleotide, whether naturally occurring in a purified restriction enzymatic digest or produced synthetically, that is capable of acting as a start point of synthesis when placed in a condition inducing the synthesis of a primer extension product complementary to a nucleic acid strand (i.e., in the presence of nucleotides and an inducing agent such as DNA polymerase, and at a suitable temperature and pH). To maximize the amplification efficiency, the primer is preferably single-stranded, but may optionally be double-stranded. If double-stranded, the primer is first treated to separate the strands before use in preparing an extension product. Preferably, the primer is a deoxyribonucleotide. The primer must be long enough to initiate the synthesis of the extension product in the presence of an inducer. The exact length of the primer will depend on many factors, including temperature, the source of the primer, and the method used.

The "vector" or "expression vector" is meant a replicon, such as a plasmid, bacmid, phage, virus, virion, or cosmid, to which another DNA segment, i.e., an "insert", may be attached, so as to achieve the replication of the attached segment in a cell. The vector may be a nucleic acid construct designed for delivery to a host cell or transfer between different host cells. As used herein, the vector may be viral or non-viral in its original and/or final form, such as the PUC57 DNA vector used herein. The term "vector" encompasses any genetic element capable of replicating when combined with appropriate control elements and capable of transferring a gene sequence to a cell. In some embodiments, the vector may be an expression vector or a recombinant vector. The "promoter" refers to any nucleic acid sequence that regulates the expression of another nucleic acid sequence by driving the transcription of the nucleic acid sequence, which may be a heterologous target gene encoding a protein or RNA. The promoter may be constitutive, inducible, repressible, or tissue-specific, or any combination thereof. The promoter is a control region of a nucleic acid sequence where the initiation and rate of the transcription of the remainder of the nucleic acid sequence is controlled.

The "gene" refers to a segment of DNA involved in the production of a polypeptide chain, which may or may not include preceding and succeeding coding regions, e.g., 5' untranslated region (5'UTR) or "leader" sequence and 3'UTR or "non-transcribed tail" sequence, as well as intervening sequences (introns) between the coding segments (exons).

The "recombinant" refers to that a polynucleotide is a product of various combinations of cloning, restriction, or ligation procedures, as well as other procedures resulting in constructs different and/or distinct from naturally occurring polynucleotides.

The "introduction", in the context of inserting a nucleic acid sequence into a cell, refers to "transfection", "transformation", or "transduction" and includes reference to the incorporation of a nucleic acid sequence into a eukaryotic or prokaryotic cell, where the nucleic acid sequence may be incorporated into the genome of the cell (e.g., chromosome, plasmid, plastidial, or mitochondrial DNA), transformed to autonomous replication, or transiently expressed (e.g., transfected mRNA).

The "nucleic acid construct" refers to a single-stranded or double-stranded nucleic acid molecule. In some embodiments, the nucleic acid construct is a DNA molecule that is modified or synthesized to comprise a nucleic acid segment in a manner that does not naturally occur; the nucleic acid molecule comprises one or more control sequences or regulatory elements. In some embodiments, the nucleic acid construct is an RNA molecule formed after DNA transcription. In the context of the present disclosure, the nucleic acid construct contains a recombinant nucleotide sequence consisting essentially of optionally one, two, three, or more isolated nucleotide sequences including 5'UTR, open reading frame (ORF), and 3'UTR. In embodiments involving constructs comprising two or more sequences, the sequences are operably linked to each other in the construct.

The "derivative sequence" refers to a nucleotide sequence that is highly homologous (e.g., having at least 80%, 85%, 88%, 90%, 93%, 95%, 96%, 97%, 98%, 99%, or 100% identity) to the UTR sequence of the present disclosure and still retains similar functional activity as the UTR sequence of the present disclosure. In some embodiments, the derivative sequence is a nucleotide sequence having one or more substituted, deleted, or added nucleotides based on a natural UTR sequence. In some embodiments, the derivative sequence is a nucleotide sequence that is truncated on the basis of the natural UTR sequence.

The "operably linked" is defined herein as a structure where a control sequence, i.e., a promoter sequence and/or a 5'UTR sequence, are suitably positioned relative to the coding DNA sequence such that the control sequence directs the transcription of the coding sequence and the translation of the mRNA into a polypeptide sequence encoded by the coding DNA.

The "open reading frame", abbreviated as "ORF", refers to a segment or region of a nucleic acid sequence encoding a polypeptide. The ORF comprises contiguous, non-overlapping, in-frame codons, starting with a start codon and ending with a stop codon in an mRNA sequence, which are translated by the ribosome.

The "endogenous" refers to any substance derived from or produced in an organism, cell, tissue, or system.

The "exogenous" refers to any substance introduced or produced from outside an organism, cell, tissue, or system.

The "sequence identity" refers to sequence identity between genes or proteins at the nucleotide or amino acid level, respectively. The "sequence identity" is a measure of identity between proteins at the amino acid level and between nucleic acids at the nucleotide level. The protein sequence identity can be determined by comparing the amino acid sequences at given positions in the aligned sequences. Similarly, the nucleic acid sequence identity can be determined by comparing the nucleotide sequences at given positions in the aligned sequences. Methods for aligning sequences for comparison are well known in the art, including GAP, BESTFIT, BLAST, FASTA, and TFASTA. In some embodiments, the method for aligning sequences is BLAST. The BLAST algorithm calculates the percent sequence identity and performs a statistical analysis of the similarity between two sequences. Software for performing BLAST analysis is publicly available through the National Center for Biotechnology Information (NCBI) website.

The "homologous" or "homology" is defined as the percentage of nucleotide residues in a target chromosome identical to those in a corresponding sequence after aligning the sequences and introducing gaps, if necessary, to achieve the maximum percentage sequence identity. Alignment to determine percentage nucleotide sequence homology can be accomplished in a variety of ways within the skill in the art, for example, using publicly available computer software such as BLAST, BLAST-2, ALIGN, ClustalW2, or Megalign (DNASTAR). Those skilled in the art can determine suitable parameters for aligning the sequences, including any algorithms necessary to achieve the maximum alignment over the full length of the sequences being compared. In some embodiments, the sequences are considered "homologous" when, for example, a nucleic acid sequence (e.g., a DNA sequence) of a homology arm has at least 70%, at least 75%, at least 80%, at least 85%, at least 90%, at least 91%, at least 92%, at least 93%, at least 94%, at least 95%, at least 96%, at least 97%, at least 98%, at least 99%, or higher identity to a corresponding native or unedited nucleic acid sequence (e.g., a genomic sequence) of a host cell.

The "replacement" is defined as a change in amino acid or nucleotide sequence as follows: one or more amino acids or nucleotides are replaced with a different amino acid or nucleotide, as compared to the amino acid sequence or nucleotide sequence of a reference polypeptide. If the replacement is conservative, the replacement amino acid has similar structural or chemical properties (e.g., charge, polarity, hydrophobicity, etc.) as the replaced amino acid. In some embodiments, a polypeptide variant may have a "non-conservative" change, where the amino acids before and after the replacement differ in structural and/or chemical properties.

The "deletion" is defined as a change in amino acid or nucleotide sequence as follows: one or more amino acid or nucleotide residues are deleted, as compared to the amino acid sequence or nucleotide sequence of a reference polypeptide. In the case of a polypeptide or polynucleotide sequence, considering the length of the polypeptide or polynucleotide sequence being modified, the deletion may involve deletions of 2, 5, 10, up to 20, up to 30, or up to 50 or more amino acid or nucleotide residues.

The "insertion" or "addition" refers to a change in amino acid or nucleotide sequence as follows: one or more amino acids or nucleotides are added, as compared to the amino acid sequence or nucleotide sequence of a reference polypeptide. The "insertion" generally refers to the addition of one or more amino acid residues within the amino acid sequence of a polypeptide (or nucleotide residues within a polynucleotide), while the "addition" may be an insertion or the addition of amino acid residues at the N- or C-terminus of a polypeptide (or the addition of nucleotide residues at the 5' or 3' end of a polynucleotide). In the case of a polypeptide or polynucleotide sequence, the addition or insertion may involve up to 10, up to 20, up to 30, or up to 50 or more amino acids (or nucleotide residues).

The "codon optimization" refers to the replacement of a codon in the target sequence that is rarely seen in highly expressed genes of a given species with a codon commonly seen in highly expressed genes of the species, with the codons before and after the replacement encoding the same amino acid. Various species may exhibit specific usage biases for certain codons for a particular amino acid. The codon usage bias (the difference in codon usage between organisms) is often correlated with the translation efficiency of messenger RNA (mRNA), which in turn is believed to particularly depend on the characteristics of the codons translated and the availability of specific transfer RNA (tRNA) molecules. The predominance of a selected tRNA in a cell is typically a reflection of the codons most frequently used in peptide synthesis. Thus, on the basis of codon optimization, genes can be modified for the optimal gene expression in a given organism. Thus, the option of optimal codons depends on the codon usage bias of the host genome.

The "cell" or "host cell" includes any cell type susceptible to transformation, transfection, transduction, and the like with the nucleic acid construct or vector of the present disclosure. As a non-limiting example, the host cell may be an isolated primary cell, a pluripotent stem cell, a CD34+ cell, an induced pluripotent stem cell, or any one of a number of immortalized cell lines (e.g., a HepG2 cell). Alternatively, the host cell may be an *in situ* or *in vivo* cell in a tissue, organ, or organism.

The "treatment" refers to administering a therapeutic agent, such as a composition comprising any one of the nucleic acid constructs of the present disclosure, either internally or externally to a patient with one or more disease symptoms on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree. The amount of therapeutic agent effective to alleviate any particular disease symptom (also known as a "therapeutically effective amount") may vary depending on a variety of factors, such as the disease state, age, and weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the disease symptom. The "effective amount" or "pharmaceutically effective amount" comprises an amount sufficient to ameliorate or prevent a symptom or condition of a medical disorder. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of adverse effects. An effective amount may be the maximum dose or administration regimen to avoid significant side effects or toxic effects. In some embodiments, the "effective amount" is an effective amount of RNA that can produce an antigen-specific immune response.

The "pharmaceutically acceptable" refers to those therapeutic agents, materials, compositions, and/or dosage forms that are, within the scope of sound medical judgment, suitable for use in contact with the tissues of patients without excessive toxicity, irritation, allergic reaction, or other problems or complications, and are commensurate with a proper benefit/risk ratio and effective for the intended use.

The "polypeptide" and "protein" have the same meaning and are used interchangeably. The "subject" refers to a mammal, including, but not limited to, humans, rodents (mice, rats, guinea pigs), dogs, horses, cows, cats, pigs, monkeys, and chimpanzees), and the like. Preferably, the subject is a human.

### Examples

The present disclosure is further described with reference to the following examples, but these examples are not intended to limit the scope of the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

### Example 1. Screening for 5'UTR and 3'UTR

To screen for a 3'-untranslated region element (3'UTR element) and a 5'-untranslated region element (5'UTR element) that can improve protein expression efficiency, the UTR sequences in housekeeping gene mRNAs were screened in this example.

First, 301 candidate housekeeping genes, including Abhd16a, were determined through the database (https://esbl.nhlbi.nih.gov/helixweb/Database/NephronRNAseq/Housekeeping_Genes.h tmL). Then, the expression levels of the genes described above were analyzed and put in order using bioinformatics methods, and the transcripts and UTR sequence information of the genes were retrieved. Further, UTR elements were screened based on the expression level and UTR sequence length, and multiple candidate UTR sequences were obtained. From the candidate UTR sequences, 33 5'UTRs numbered A1-A33 and 14 3'UTRs numbered B1-B14 were obtained by screening. The gene sources and sequence numbers are shown in Table 1 and Table 2.

**Table 1. New 3'UTRs obtained by screening**

| 3'UTR No. | Gene source (all are Homo sapiens) | Corresponding DNA sequence No. | Corresponding mRNA sequence No. |
|---|---|---|---|
| B1 | ACTG1 | SEQ ID NO:1 | SEQ ID NO:58 |
| B2 | ATP6V0B | SEQ ID NO:2 | SEQ ID NO:59 |
| B3 | ATP6V0B | SEQ ID NO:3 | SEQ ID NO:60 |
| B4 | ATP6V0E1 | SEQ ID NO:4 | SEQ ID NO:61 |
| B5 | ATP6V0E1 | SEQ ID NO:5 | SEQ ID NO:62 |
| B6 | CFL1 | SEQ ID NO:6 | SEQ ID NO:63 |
| B7 | CFL1 | SEQ ID NO:7 | SEQ ID NO:64 |
| B8 | CFL1 | SEQ ID NO:8 | SEQ ID NO:65 |
| B9 | COX4I1 | SEQ ID NO:9 | SEQ ID NO:66 |
| B10 | COX4I1 | SEQ ID NO:10 | SEQ ID NO:67 |
| B11 | COX4I1 | SEQ ID NO:11 | SEQ ID NO:68 |
| B12 | CTSB | SEQ ID NO:12 | SEQ ID NO:69 |
| B13 | FAM166A | SEQ ID NO:13 | SEQ ID NO:70 |
| B14 | NDUFB9 | SEQ ID NO:14 | SEQ ID NO:71 |

**Table 2. New 5'UTRs obtained by screening**

| 5'UTR No. | Gene source (all are Homo sapiens) | Corresponding DNA sequence No. | Corresponding mRNA sequence No. |
|---|---|---|---|
| A1 | ACTG1 | SEQ ID NO:15 | SEQ ID NO:72 |
| A2 | ATP6V0B | SEQ ID NO:16 | SEQ ID NO:73 |
| A3 | ATP6V0B | SEQ ID NO:17 | SEQ ID NO:74 |
| A4 | ATP6V0E1 | SEQ ID NO:18 | SEQ ID NO:75 |
| A5 | ATP6V0E1 | SEQ ID NO:19 | SEQ ID NO:76 |
| A6 | CFL1 | SEQ ID NO:20 | SEQ ID NO:77 |
| A7 | CFL1 | SEQ ID NO:21 | SEQ ID NO:78 |
| A8 | CFL1 | SEQ ID NO:22 | SEQ ID NO:79 |
| A9 | COX4I1 | SEQ ID NO:23 | SEQ ID NO:80 |
| A10 | COX4I1 | SEQ ID NO:24 | SEQ ID NO:81 |
| A11 | COX4I1 | SEQ ID NO:25 | SEQ ID NO:82 |
| A12 | CTSB | SEQ ID NO:26 | SEQ ID NO:83 |
| A13 | FAM166A | SEQ ID NO:27 | SEQ ID NO:84 |
| A14 | NDUFB9 | SEQ ID NO:28 | SEQ ID NO:85 |
| A15 | CHCHD10 | SEQ ID NO:29 | SEQ ID NO:86 |
| A16 | CHCHD10 | SEQ ID NO:30 | SEQ ID NO:87 |
| A17 | SLC38A2 | SEQ ID NO:31 | SEQ ID NO:88 |
| A18 | NDUFA11 | SEQ ID NO:32 | SEQ ID NO:89 |
| A19 | NDUFV3 | SEQ ID NO:33 | SEQ ID NO:90 |
| A20 | PRDX5 | SEQ ID NO:34 | SEQ ID NO:91 |
| A21 | GUK1 | SEQ ID NO:35 | SEQ ID NO:92 |
| A22 | GUK1 | SEQ ID NO:36 | SEQ ID NO:93 |
| A23 | GUK1 | SEQ ID NO:37 | SEQ ID NO:94 |
| A24 | IAH1 | SEQ ID NO:38 | SEQ ID NO:95 |
| A25 | ABHD16A | SEQ ID NO:39 | SEQ ID NO:96 |
| A26 | SLC25A39 | SEQ ID NO:40 | SEQ ID NO:97 |
| A27 | ATPIF1 | SEQ ID NO:41 | SEQ ID NO:98 |
| A28 | ANAPC 11 | SEQ ID NO:42 | SEQ ID NO:99 |
| A29 | ANAPC 11 | SEQ ID NO:43 | SEQ ID NO:100 |
| A30 | CCDC12 | SEQ ID NO:44 | SEQ ID NO:101 |
| A31 | MRPL14 | SEQ ID NO:45 | SEQ ID NO:102 |
| A32 | APOA1BP | SEQ ID NO:46 | SEQ ID NO:103 |
| A33 | APOA1BP | SEQ ID NO:47 | SEQ ID NO:104 |

### Example 2. mRNA Preparation

The UTRs obtained by screening in Example 1 were constructed into a DNA vector for *in vitro* transcription to obtain mRNAs that stably express 5'UTR and 3'UTR elements. The vector contains a T7 promoter, a sequence encoding Firefly Luciferase (Fluc) as an open reading frame (ORF) (SEQ ID NO: 126), and a polyadenylic acid (polyA) sequence that is followed by a restriction site for vector linearization. The polyA was selected from the group consisting of A120 (i.e., 120 contiguous adenylic acids) and A30L70 (SEQ ID NO: 52). The 5'UTR and 3'UTR elements were constructed to the 5' end and 3' end of the open reading frame (ORF) (Fluc), respectively, through appropriate restriction enzyme cutting sites. Examples of constructed vectors are shown in FIGs. 1A to 1C, and combinations of the 5'UTRs and 3'UTRs are shown in Table 3. The control vectors used in this example include Mod.-120A (SEQ ID NO: 53), in which the polyA is A120, and Mod.-A30L70 (SEQ ID NO: 54) and BioN.-A30L70 (SEQ ID NO: 55), in which the polyA is A30L70. The target genes in the control vectors were all Fluc, and the vectors were constructed by whole-gene synthesis. Different UTR elements were constructed to the vectors described above by enzymatic digestion. The construction vector for vectors V-B1 to V-B14 was Mod.-120A, the restriction enzyme cutting sites used were *Age*I and *SacII,* their polyAs were all A120, and the control used was Mod.-120A (SEQ ID NO: 53). The construction vector for vectors V-A1 to V-A14 was Mod.-120A (SEQ ID NO: 53), the restriction enzyme cutting sites used were *BamHI* and *Nhe*I, their polyAs were all A120, and the control used was also Mod.-120A (SEQ ID NO: 53). The construction vector for V-A15 to V-A33 was Mod.-A30L70 (SEQ ID NO: 54), the restriction enzyme cutting sites used were *Bam*HI and *Nhe*I, their polyAs were all A30L70, and the control used was Mod.-A30L70 (SEQ ID NO: 54). The construction vector for vectors V-A1-B12, V-A1-B13, and V-A1-B14 was Mod.-A30L70 (SEQ ID NO: 54), the restriction enzyme cutting sites used were *Bam*HI and *Sac*II, their polyAs were all A30L70, and the controls used were Mod.-A30L70 (SEQ ID NO: 54) and BioN.-A30L70 (SEQ ID NO: 55). The inserted UTR element gene fragments were all whole-gene-synthesized (Suzhou GENEWIZ Biotechnology Co., Ltd.), and the fragments constructed to corresponding vectors by enzymatic digestion ligation. After vector construction was completed, enzymatic digestion and sequencing verification were performed. Vectors that passed the verification were used in the next step.

**Table 3. Combinations of 5'UTRs and 3'UTRs in vectors**

| Vector No. (corresponding mRNA No.) | 5'UTR | 3'UTR |
|---|---|---|
| Mod. | Mod. 5'UTR (SEQ ID NO:48) | α-globin 3'UTR (SEQ ID NO: 49) |
| V-B1 (m-B1) | | B1 (SEQ ID NO:1) |
| V-B2 (m-B2) | | B2 (SEQ ID NO:2) |
| V-B3 (m-B3) | | B3 (SEQ ID NO:3) |
| V-B4 (m-B4) | | B4 (SEQ ID NO:4) |
| V-B5 (m-B5) | | B5 (SEQ ID NO:5) |
| V-B6 (m-B6) | | B6 (SEQ ID NO:6) |
| V-B7 (m-B7) | | B7 (SEQ ID NO:7) |
| V-B8 (m-B8) | | B8 (SEQ ID NO:8) |
| V-B9 (m-B9) | | B9 (SEQ ID NO:9) |
| V-B10 (m-B10) | | B10 (SEQ ID NO:10) |
| V-B11 (m-B11) | | B11 (SEQ ID NO:11) |
| V-B12 (m-B12) | | B12 (SEQ ID NO:12) |
| V-B13 (m-B13) | | B13 (SEQ ID NO:13) |
| V-B14 (m-B14) | | B14 (SEQ ID NO:14) |
| V-A1 (m-A1) | A1 (SEQ ID NO:15) | α-globin 3'UTR (SEQ ID NO: 49) |
| V-A2 (m-A2) | A2 (SEQ ID NO:16) | |
| V-A3 (m-A3) | A3 (SEQ ID NO:17) | |
| V-A4 (m-A4) | A4 (SEQ ID NO:18) | |
| V-A5 (m-A5) | A5 (SEQ ID NO:19) | |
| V-A6 (m-A6) | A6 (SEQ ID NO:20) | |
| V-A7 (m-A7) | A7 (SEQ ID NO:21) | |
| V-A8 (m-A8) | A8 (SEQ ID NO:22) | |
| V-A9 (m-A9) | A9 (SEQ ID NO:23) | |
| V-A10 (m-A10) | A10 (SEQ ID NO:24) | |
| V-A11 (m-A11) | A11 (SEQ ID NO:25) | |
| V-A12 (m-A12) | A12 (SEQ ID NO:26) | |
| V-A13 (m-A3) | A13 (SEQ ID NO:27) | |
| V-A14 (m-A14) | A14 (SEQ ID NO:28) | |
| V-A15 (m-A15) | A15 (SEQ ID NO:29) | |
| V-A16 (m-A16) | A16 (SEQ ID NO:30) | |
| V-A17 (m-A17) | A17 (SEQ ID NO:31) | |
| V-A18 (m-A18) | A18 (SEQ ID NO:32) | |
| V-A19 (m-A19) | A19 (SEQ ID NO:33) | |
| V-A20 (m-A20) | A20 (SEQ ID NO:34) | |
| V-A21 (m-A21) | A21 (SEQ ID NO:35) | |
| V-A22 (m-A22) | A22 (SEQ ID NO:36) | |
| V-A23 (m-A23) | A23 (SEQ ID NO:37) | |
| V-A24 (m-A24) | A24 (SEQ ID NO:38) | |
| V-A25 (m-A25) | A25 (SEQ ID NO:39) | |
| V-A26 (m-A26) | A26 (SEQ ID NO:40) | |
| V-A27 (m-A27) | A27 (SEQ ID NO:41) | |
| V-A28 (m-A28) | A28 (SEQ ID NO:42) | |
| V-A29 (m-A29) | A29 (SEQ ID NO:43) | |
| V-A30 (m-A30) | A30 (SEQ ID NO:44) | |
| V-A31 (m-A31) | A31 (SEQ ID NO:45) | |
| V-A32 (m-A32) | A32 (SEQ ID NO:46) | |
| V-A33 (m-A33) | A33 (SEQ ID NO:47) | |
| V-A1-B12 (m-A1-B12) | A1 (SEQ ID NO:15) | B12 (SEQ ID NO:12) |
| V-A1-B13 (m-A1-B13) | A1 (SEQ ID NO:15) | B13 (SEQ ID NO:13) |
| V-A1-B14 (m-A1-B14) | A1 (SEQ ID NO:15) | B14 (SEQ ID NO:14) |
| BioN. | α-1-globin 5'UTR (SEQ ID NO: 50) | haplogroup U8b1b1 mitochondrion (SEQ ID NO:51) |

The vectors in Table 3 (i.e., DNA templates) were digested by restriction endonuclease treatment to linearize the DNA templates, and then transcribed *in vitro* using T7-RNA polymerase. For *in vitro* transcription, T7 RNA polymerase (Roche), a corresponding reaction buffer, pyrophosphatase, an RNase inhibitor, and NTP were used. For effective capping of RNA, an excess of the cap analogous compound ARCA (3'-O-Me-m⁷G(5')ppp(5')G, Trilink, N-7003-1) or CleanCap (m7G(5')ppp(5')(2'OMeA)pG, Trilink, N-7113) was added to the reaction. For the RNA production used in FIGs. 2 to 5, ARCA was used for capping, and the RNAs used in FIGs. 6 to 9 were capped with CleanCap. Meanwhile, to reduce mRNA immunogenicity and improve translation efficiency, the mRNAs in the present disclosure were all subjected to nucleic acid modification. The uridine triphosphates (abbreviated as UTP) in the reaction system were all replaced with N1-methyl-pseudouridine triphosphates (abbreviated as 1m-ψUTP, purchased from ThermoFisher). The modification method was based on Patent US 2014/0194494 A1. After the *in vitro* transcription system was incubated at 37 °C for 2.5 h, the RNAs were purified using carboxylated magnetic beads (Invitrogen) and resuspended in ribozyme-free water. The concentration and quality of the RNAs were assessed by spectrophotometry and analysis on a 5200 bioanalyzer (Agilent). Analysis showed that the target mRNA was obtained.

The sequence from the 5'UTR to polyA of Mod. of the present disclosure is set forth in SEQ ID NOs: 53 and 54, and the sequence from the 5'UTR to polyA of BioN. is set forth in SEQ ID NO: 55. The 5'UTR and 3'UTR sequences in Mod. agree with the sequences in the patents and publications of company Moderna (US10849920B2, WO2013151667A1, US10730924B2, and DOI: 10.1016/j.cell.2017.02.017), and the 5'UTR and 3'UTR sequences of BioN. are derived from patent WO 2018/160540. Illustratively, the linearized sequence of V-B1 is given, with the sequence from the 5'UTR to polyA set forth in SEQ ID NO: 56; the linearized sequence of V-A1 is given, with the sequence from the 5'UTR to polyA set forth in SEQ ID NO: 57.

### Example 3. Functional Validation of 5'UTRs, 3'UTRs, and Combinations Thereof

In this example, an mRNA lipofection luciferase expression system was used for functional validation of UTRs.

Experimental method: Human embryonic kidney cells (HEK293, purchased from ATCC) were seeded in a 96-well plate at a density of 4 × 10⁴ cells/well. The following day, transfection was performed using a Lipofectamine^{™} MessengerMAX^{™} mRNA transfection reagent. The cells in each well were transfected with 100 ng of the aforementioned capped mRNA prepared in Example 2. At 6 h post-transfection, the culture medium was replaced with a fresh one. 100 µL of culture medium was pipetted off, and 50 µL of luciferase substrate was added. The luminescence intensity was measured using a PerkinElmer multi-mode microplate reader. The experimental method for human cervical cancer cells (HeLa, purchased from ATCC) and human lung cancer cells (A549, purchased from ATCC) was the same as that for HEK293.

Assessment method: In Tables 4 to 7 and Tables 9 to 11, the expression level of luciferase for the reference positive control Mod. in different cell lines was set to 1, and the expression level ratios of different mRNAs to the positive control Mod. were calculated. In Table 8, the expression level of luciferase for the reference positive control BioN. in different cell lines was set to 1, and the expression level ratios of different mRNAs to the positive control BioN. were calculated. The values in the tables are all ratios of the means of measurements.

### 1) Testing of 3'UTRs' ability to regulate target gene expression in different cell lines

To validate the ability of different 3'UTRs to regulate target gene expression in different cell lines, luciferase (Fluc)-expressing mRNAs were produced using the method of Example 2, and the produced RNAs were ARCA-capped, with the polyA tail structure being A120. The RNAs were transfected into human HeLa, HEK293, and A549 cells, and luciferase levels were measured at 24 h post-transfection. The results are shown in Table 4 and FIG. 2.

**Table 4. Expression levels of luciferase (Fluc) expressed by mRNAs carrying different 3'UTRs**

| (ARCA-capped; the target genes were all Fluc, and the polyAs were all A120) | | | | | |
|---|---|---|---|---|---|
| mRNA No. | 5'UTR's mRNA sequence No. | 3'UTR's mRNA sequence No. | Cell line | | |
| | | | HeLa | HEK293 | A549 |
| Mod. | SEQ ID NO: 105 | SEQ ID NO: 106 | 1 | 1 | 1 |
| m-B1 | | SEQ ID NO: 58 | 1.25 | 1.52 | 1.55 |
| m-B2 | | SEQ ID NO: 59 | 1.72 | 1.85 | 1.94 |
| m-B3 | | SEQ ID NO: 60 | 1.56 | 1.66 | 1.83 |
| m-B4 | | SEQ ID NO: 61 | 1.63 | 1.68 | 1.77 |
| m-B5 | | SEQ ID NO: 62 | 0.99 | 1.01 | 1.08 |
| m-B6 | | SEQ ID NO: 63 | 1.13 | 1.30 | 1.40 |
| m-B7 | | SEQ ID NO: 64 | 1.41 | 1.72 | 1.69 |
| m-B8 | | SEQ ID NO: 65 | 1.31 | 1.40 | 1.57 |
| m-B9 | | SEQ ID NO: 66 | 2.04 | 1.71 | 2.13 |
| m-B10 | | SEQ ID NO: 67 | 1.72 | 1.83 | 1.82 |
| m-B11 | | SEQ ID NO: 68 | 1.65 | 1.61 | 1.63 |
| m-B12 | | SEQ ID NO: 69 | 2.29 | 2.22 | 2.24 |
| m-B13 | | SEQ ID NO: 70 | 2.21 | 2.27 | 2.28 |
| m-B14 | | SEQ ID NO: 71 | 2.07 | 1.92 | 2.27 |

The results show that in different cell lines, compared to the positive control Mod., there was a consistency in the influence of 3'UTRs of B1-B14 on protein expression level, and there was no significant difference (p > 0.05) between different cell lines. This indicates that the 3'UTR elements obtained by screening in the present disclosure have a universal influence on protein expression level and can be used to improve the protein expression level of a target gene.

### 2) Testing of 3'UTRs' regulating target gene expression time in cells

To test the ability of different 3'UTRs to regulate target gene expression time in cells, the mRNAs in Table 4 were transfected into HEK293 cells, and luciferase expression levels were measured at 6 h, 24 h, 48 h, and 72 h post-transfection. The results are shown in Table 5 and FIG. 3.

**Table 5. Expression levels of luciferase (Fluc) expressed by mRNAs carrying different 3'UTRs**

| mRNA No. | HEK293 | | | |
|---|---|---|---|---|
| | 6h | 24h | 48h | 72h |
| Mod. | 1 | 1 | 1 | 1 |
| m-B1 | 2.61 | 1.88 | 1.73 | 1.40 |
| m-B2 | 2.26 | 1.94 | 1.78 | 1.38 |
| m-B3 | 2.09 | 1.95 | 1.67 | 1.51 |
| m-B4 | 2.08 | 1.80 | 1.93 | 2.10 |
| m-B5 | 1.41 | 1.05 | 1.04 | 0.98 |
| m-B6 | 1.59 | 1.47 | 1.44 | 1.29 |
| m-B7 | 1.77 | 1.76 | 1.78 | 1.53 |
| m-B8 | 1.69 | 1.75 | 1.64 | 1.41 |
| m-B9 | 1.99 | 3.39 | 2.32 | 1.78 |
| m-B10 | 2.96 | 2.83 | 2.36 | 2.05 |
| m-B11 | 2.52 | 2.13 | 1.99 | 1.68 |
| m-B12 | 2.95 | 2.54 | 2.71 | 2.01 |
| m-B13 | 2.44 | 2.77 | 2.56 | 2.54 |
| m-B14 | 2.99 | 2.44 | 2.68 | 2.51 |

The results show that in HEK293 cells, a change of the 3'UTR element can affect the expression level of the target protein; the 3'UTR elements, other than the B5-3'UTR element, can all increase the protein expression level 1.2-fold or more, and there were significant differences (p < 0.05) at different time points of testing; the 3'UTR elements of B12, B13, and B14 performed best: they can increase the protein expression level 2-fold or more and have relatively good time continuity.

### 3) Testing of 5'UTRs' regulating target gene expression in cells

To test the ability of the different 5'UTRs to regulate target gene expression time in cells, luciferase (Fluc)-expressing mRNAs were prepared using the aforementioned method. The RNAs used in Table 6 and FIG. 4 were ARCA-capped, with the polyA tail structure being A120. The RNAs used in Table 7 and FIG. 5 were CleanCap-capped, with the polyA tail structure being A30L70. The produced RNAs described above were transfected into HEK293 cells, and luciferase expression levels were measured at 6 h, 24 h, 48 h, and 72 h post-transfection. The results are shown in Table 6, Table 7, FIG. 4, and FIG. 5.

**Table 6. Expression levels of luciferase (Fluc) expressed by mRNAs carrying different 5'UTRs**

| (ARCA-capped; the target genes were all Fluc, and the polyAs were all A120) | | | | | | |
|---|---|---|---|---|---|---|
| mRNA No. | 5'UTR's mRNA sequence No. | 3'UTR's mRNA sequence No. | HEK293 | | | |
| | | | 6h | 24h | 48h | 72h |
| Mod. | SEQ ID NO:105 | α-globin (SEQ ID NO: 106) | 1 | 1 | 1 | 1 |
| m-A1 | SEQ ID NO:72 | | 2.83 | 4.42 | 4.22 | 4.05 |
| m-A2 | SEQ ID NO:73 | | 0.25 | 0.27 | 0.26 | 0.25 |
| m-A3 | SEQ ID NO:74 | | 1.56 | 1.72 | 1.72 | 1.55 |
| m-A4 | SEQ ID NO:75 | | 2.02 | 2.18 | 2.12 | 1.97 |
| m-A5 | SEQ ID NO:76 | | 1.97 | 2.28 | 2.17 | 2.04 |
| m-A7 | SEQ ID NO:78 | | 2.08 | 2.10 | 2.06 | 1.97 |
| m-A8 | SEQ ID NO:79 | | 1.26 | 1.17 | 1.18 | 1.11 |
| m-A9 | SEQ ID NO:80 | | 1.95 | 1.99 | 2.05 | 1.87 |
| m-A10 | SEQ ID NO:81 | | 1.69 | 1.70 | 1.78 | 1.55 |
| m-A11 | SEQ ID NO:82 | | 2.20 | 2.23 | 2.24 | 2.23 |
| m-A12 | SEQ ID NO:83 | | 1.51 | 1.48 | 1.36 | 1.34 |
| m-A13 | SEQ ID NO:84 | | 1.72 | 1.70 | 1.59 | 1.52 |
| m-A14 | SEQ ID NO:85 | | 1.80 | 1.97 | 1.81 | 1.73 |

**Table 7. Expression levels of luciferase (Fluc) expressed by mRNAs carrying different 5'UTRs (CleanCap-capped; the target genes were all Fluc, and the polyAs were all A30L70)**

| mRNA No. | 5'UTR's mRNA sequence No. | 3'UTR's mRNA sequence No. | HEK293 | | | |
|---|---|---|---|---|---|---|
| | | | 6h | 24h | 48h | 72h |
| Mod. | SEQ ID NO:105 | α-globin (SEQ ID NO: 106) | 1 | 1 | 1 | 1 |
| m-A15 | SEQ ID NO:86 | | 2.07 | 2.31 | 2.73 | 3.11 |
| m-A16 | SEQ ID NO:87 | | 1.54 | 2.09 | 2.50 | 3.17 |
| m-A17 | SEQ ID NO:88 | | 1.19 | 1.36 | 1.59 | 2.01 |
| m-A18 | SEQ ID NO:89 | | 1.47 | 1.95 | 2.50 | 2.94 |
| m-A19 | SEQ ID NO:90 | | 1.14 | 1.25 | 1.56 | 2.06 |
| m-A20 | SEQ ID NO:91 | | 0.76 | 0.86 | 1.24 | 1.38 |
| m-A21 | SEQ ID NO:92 | | 1.43 | 1.69 | 2.23 | 2.59 |
| m-A22 | SEQ ID NO:93 | | 0.53 | 0.69 | 0.88 | 0.97 |
| m-A23 | SEQ ID NO:94 | | 0.13 | 0.20 | 0.24 | 0.29 |
| m-A24 | SEQ ID NO:95 | | 1.37 | 1.98 | 2.40 | 2.70 |
| m-A25 | SEQ ID NO:96 | | 1.03 | 0.98 | 1.20 | 1.27 |
| m-A26 | SEQ ID NO:97 | | 0.70 | 0.69 | 0.93 | 0.89 |
| m-A27 | SEQ ID NO:98 | | 1.62 | 1.62 | 2.09 | 1.94 |
| m-A28 | SEQ ID NO:99 | | 1.39 | 1.34 | 1.76 | 1.65 |
| m-A29 | SEQ ID NO:100 | | 1.01 | 1.04 | 1.28 | 1.34 |
| m-A30 | SEQ ID NO:101 | | 1.37 | 1.27 | 1.13 | 1.06 |
| m-A31 | SEQ ID NO:102 | | 1.17 | 1.09 | 1.34 | 1.43 |
| m-A32 | SEQ ID NO:103 | | 1.31 | 1.41 | 1.64 | 1.60 |
| m-A33 | SEQ ID NO:104 | | 1.31 | 1.20 | 1.44 | 1.42 |

FIGs. 4 and 5 show screening through different capping methods and polyA tail structures. The results show that in different screening vectors, a change of the 5'UTR element can affect the expression level of the target protein, and the 5'UTR elements' improvements to the expression level of the target protein have universality. The 5'UTR elements numbered A1, A3 to A7, A9 to A16, A18, A21, A24, A27, A28, A32, and A33 can all increase the protein expression level 1.2-fold or more, and there were significant differences (p < 0.05) at the time points of testing of 24 h, 48 h, and 72 h; the 5'UTR elements of A1, A15, A16, and A18 performed best: they can increase the protein expression level 2-fold or more and have relatively good time continuity.

### 4) Testing of candidate 5'UTRs' regulating target gene expression intensity compared to positive control BioN. vector

To test the ability of different candidate 5'UTRs to regulate target gene expression intensity compared to the positive control BioN. vector, the aforementioned relatively good 5'UTR elements of A1, A15, A16, and A18 obtained by screening were constructed into vectors containing an A30L70 polyA tail element. Luciferase (Fluc)-expressing mRNAs were produced using the aforementioned method and transfected into HEK293 cells, and luciferase expression levels were measured at 6 h, 24 h, 48 h, and 72 h post-transfection. The results are shown in Table 8 and FIG. 6.

**Table 8. Expression levels of luciferase (Fluc) expressed by mRNAs carrying different 5'UTRs**

| (Cleancap-capped; the target genes were all Fluc, and the polyAs were all A30L70) | | | | | |
|---|---|---|---|---|---|
| mRNA No. | 5'UTR's mRNA sequence No. | 3'UTR's mRNA sequence No. | HEK293 | | |
| | | | 6h | 24h | 48h |
| BioN. | SEQ ID NO:107 | SEQ ID NO:108 | 1 | 1 | 1 |
| m-A1 | SEQ ID NO:72 | α-globin (SEQ ID NO: 106) | 1.34 | 1.78 | 1.92 |
| m-A15 | SEQ ID NO:86 | | 1.32 | 1.58 | 1.76 |
| m-A16 | SEQ ID NO:87 | | 1.01 | 1.37 | 1.54 |
| m-A18 | SEQ ID NO:89 | | 1.07 | 1.29 | 1.49 |

The results show that the candidate 5'UTR elements can all increase the protein expression level at least more than 1.2-fold at 24 h and 48 h as compared to the BioN. vector, and all have significant differences (p < 0.05).

### 5) Testing of different 5'UTR and 3'UTR combinations' regulating target gene expression in cells

To study the influence of different 5'-UTR and 3'-UTR combinations on target protein expression, mRNAs containing different 5'-UTR and 3'-UTR element combinations were compared with the 5'UTR and 3'UTR used by company Moderna. The results are shown in Table 9 and FIG. 7.

**Table 9. Expression levels for mRNAs carrying different 5'UTR and 3'UTR combinations**

| (Cleancap-capped; the target genes were all Fluc, and the polyAs were all A30L70) | | | | | | |
|---|---|---|---|---|---|---|
| mRNA No. | 5'UTR's mRNA sequence No. | 3'UTR's mRNA sequence No. | HEK293 | | | |
| | | | 6h | 24h | 48h | 72h |
| Mod. | SEQ ID NO:105 | SEQ ID NO:106 | 1 | 1 | 1 | 1 |
| m-A1-B12 | SEQ ID NO:72 | SEQ ID NO:69 | 2.78 | 3.06 | 2.46 | 2.96 |
| m-A1-B13 | | SEQ ID NO:70 | 2.62 | 2.67 | 2.30 | 2.57 |
| m-A1-B14 | | SEQ ID NO:71 | 2.20 | 2.14 | 1.74 | 1.90 |
| m-A15-B12 | SEQ ID NO:86 | SEQ ID NO:69 | 2.11 | 2.14 | 1.78 | 1.90 |
| m-A15-B13 | | SEQ ID NO:70 | 2.04 | 2.15 | 1.74 | 1.87 |
| m-A15-B14 | | SEQ ID NO:71 | 2.09 | 1.90 | 1.68 | 1.68 |
| m-A16-B12 | SEQ ID NO:87 | SEQ ID NO:69 | 2.32 | 2.02 | 1.82 | 1.81 |
| m-A16-B13 | | SEQ ID NO:70 | 2.43 | 1.96 | 1.78 | 1.89 |
| m-A16-B14 | | SEQ ID NO:71 | 2.30 | 1.79 | 1.63 | 1.68 |
| m-A18-B12 | SEQ ID NO:89 | SEQ ID NO:69 | 1.87 | 1.90 | 1.27 | 1.41 |
| m-A18-B13 | | SEQ ID NO:70 | 2.17 | 2.31 | 1.71 | 2.08 |
| m-A18-B14 | | SEQ ID NO:71 | 1.40 | 1.66 | 1.31 | 1.51 |

The results show that combinations of the 5'UTR and 3'-UTR elements obtained by screening in the present disclosure can all increase the protein expression level 1.3-fold or more at the time points of testing of 24 h, 48 h, and 72 h as compared to the 5'UTR and 3'UTR elements used by company Moderna, and all have significant differences (*p* < 0.05). The target proteins in 1)-5) above were all luciferase (Fluc), and the target protein in 6) below was a secreted protein (e.g., hHGF or anti-PD-1 antibody).

### 6) Testing of different 5'UTR and 3'UTR combinations' regulating secreted target protein expression

To study the influence of different 5'-UTR and 3'-UTR combinations on mRNA expression of a secreted protein, mRNAs were constructed in such a way that the target protein expressed by the ORF was human hepatocyte growth factor (hHGF) or an anti-PD-1 antibody.

The amino acid sequence of hHGF is set forth in SEQ ID NO: 109, and its DNA sequence is set forth in SEQ ID NO: 110. After codon optimization, mRNAs OS1, OS2, and OS3 were obtained, and their optimized codon sequences are set forth in SEQ ID NOs: 129-131.

The amino acid sequences of the heavy and light chains of the PD-1 antibody are set forth in SEQ ID NOs: 117 and 118, respectively. The DNA sequences for the heavy and light chains are set forth in SEQ ID NOs: 119 and 120, respectively. The mRNA sequences for the heavy and light chains are set forth in SEQ ID NOs: 121 and 122, respectively.

First, to improve the translation efficiency of hHGF protein, the nucleotide sequence of wild-type hHGF (SEQ ID NO: 110) was codon-optimized in the present disclosure. The optimized sequences include hHGF-OS1 (SEQ ID NO: 111), hHGF-OS2 (SEQ ID NO: 112), and hHGF-OS3 (SEQ ID NO: 113), and the corresponding mRNA sequences are set forth in SEQ ID NOs: 129-131, respectively. The natural hHGF and the hHGF sequence with an optimized codon sequence described above were constructed into vectors to produce mRNAs. The expression level of hHGF protein was measured by ELISA, and the hHGF codon-optimized sequence was shown to be capable of improving the expression level of hHGF protein.

hHGF-OS2-expressing mRNAs containing different 5'UTR and 3'-UTR element combinations (the corresponding DNA sequence is SEQ ID NO: 112) were compared with an hHGF-OS2-expressing mRNA containing a combination of the 5'UTR and 3'UTR elements of company Moderna, Mod. (hHGF) (the corresponding DNA sequence is SEQ ID NO: 114). HEK293 cells were transfected with the hHGF-encoding mRNAs. Supernatants were collected at 6 h, 24 h, 48 h, and 72 h post-transfection, and the expression level of hHGF protein was measured by ELISA to assess the increase/prolongation of HGF expression by the candidate vectors. The results are shown in Table 10 and FIG. 8A.

**Table 10. Target protein expression levels for mRNAs carrying different 5'UTR and 3'UTR combinations**

| (Cleancap-capped; the target genes were all hHGF, and the polyAs were all A30L70) | | | | | | |
|---|---|---|---|---|---|---|
| mRNA No. (sequence No.) | 5'UTR's mRNA sequence No. | 3'UTR's mRNA sequence No. | hHGF protein level (ng/mL) | | | |
| | | | 6h | 24h | 48h | 72h |
| Mod.(hHGF) (SEQ ID NO:114) | SEQ ID NO:105 | SEQ ID NO:106 | 1.16 | 21.83 | 26.89 | 15.37 |
| m-A1-B12(hHGF) (SEQ ID NO:115) | SEQ ID NO:72 | SEQ ID NO:69 | 2.06 | 38.65 | 41.49 | 23.76 |
| m-A1-B13(hHGF) | | SEQ ID NO:70 | 1.98 | 30.19 | 30.57 | 19.00 |
| m-A1-B14(hHGF) | | SEQ ID NO:71 | 2.27 | 34.61 | 37.75 | 19.33 |
| m-A15-B12(hHGF) (SEQ ID NO:116) | SEQ ID NO:86 | SEQ ID NO:69 | 1.84 | 33.15 | 36.79 | 19.06 |
| m-A15-B13(hHGF) | | SEQ ID NO:70 | 2.14 | 31.68 | 36.60 | 25.04 |
| m-A15-B14(hHGF) | | SEQ ID NO:71 | 1.74 | 30.08 | 26.85 | 14.81 |
| m-A16-B12(hHGF) (SEQ ID NO:127) | SEQ ID NO:87 | SEQ ID NO:69 | 2.34 | 40.39 | 42.82 | 24.50 |
| m-A16-B13(hHGF) | | SEQ ID NO:70 | 1.95 | 30.53 | 32.31 | 20.36 |
| m-A16-B14(hHGF) | | SEQ ID NO:71 | 2.22 | 33.56 | 37.61 | 21.10 |
| m-A18-B12(hHGF) | SEQ ID NO:89 | SEQ ID NO:69 | 2.45 | 49.06 | 55.14 | 30.24 |
| m-A18-B13(hHGF) | | SEQ ID NO:70 | 1.94 | 38.38 | 38.05 | 25.52 |
| m-A18-B14(hHGF) | | SEQ ID NO:71 | 1.93 | 36.75 | 38.37 | 23.25 |

mRNAs containing different 5'UTR and 3'UTR element combinations and expressing the full length of an anti-PD-1 antibody (the corresponding DNA sequences are SEQ ID NOs: 124-125) were compared with an anti-PD-1 antibody-expressing mRNA containing a combination of the 5'UTR and 3'UTR elements of company Moderna (the corresponding DNA sequence is SEQ ID NO: 123). HEK293 cells were transfected with the mRNAs encoding the full length of the anti-PD-1 antibody. Supernatants were collected at 6 h, 24 h, 48 h, and 72 h post-transfection, and the expression level of the anti-PD-1 antibody was measured by ELISA to assess the increase/prolongation of anti-PD-1 antibody expression by the candidate vectors. The results are shown in Table 11 and FIG. 8B.

**Table 11. Target protein expression levels for mRNAs carrying different 5'UTR and 3'UTR combinations**

| (Cleancap-capped; the target genes were all the heavy and light chains of the anti-PD-1 antibody, and the polyAs were all A30L70) | | | | |
|---|---|---|---|---|
| mRNA No. (corresponding DNA sequence No.) | Anti-PD-1 antibody (ng/mL) | | | |
| | 6h | 24h | 48h | 72h |
| Mod.(PD-1 Ab) (SEQ ID NO:123) | 2.79 | 23.11 | 30.04 | 28.23 |
| m-A1-B12(PD-1 Ab) (SEQ ID NO:124) | 3.22 | 33.47 | 52.50 | 43.19 |
| m-A15-B12(PD-1 Ab) (SEQ ID NO:125) | 2.77 | 34.39 | 45.72 | 49.90 |

The above results show that after 72 h of continuous expression of the secreted proteins, hHGF and the anti-PD-1 antibody, combinations of the 5'UTRs and 3'UTRs obtained by screening in the present disclosure can significantly increase the expression level of the secreted proteins (p < 0.05) as compared to the 5'UTR and 3'UTR elements used by company Moderna.

### Example 4. Validation of hHGF Protein Expression by hHGF-Expressing mRNAs in Mice

To determine the ability of the mRNA molecule m-A16-B12 (hHGF) (the corresponding DNA sequence is SEQ ID NO: 127) to express hHGF in animals, the mRNA was delivered using lipid nanoparticles (LNPs) (containing 50 mol% of ionizable lipid (SM-102), 10 mol% of DSPC, 38.5 mol% of cholesterol, and 1.5 mol% of PEG-DMG). The same lipid nanoparticles (LNPs) were used in Examples 5 and 6 below. The positive control was the Collategene plasmid (company AnGes), which was administered as a naked plasmid. Balb/c mice (6-8 w, male) were randomly divided into 4 groups of 33 and received intramuscular injection of different doses of m-A16-B12 (hHGF) and the Collategene plasmid into the gastrocnemius muscle. Subsequently, the following experimental groups were organized according to the regimens in Table 12: 1) injecting 1.0 µg/animal of m-A16-B12 (hHGF); 2) injecting 0.3 µg/animal of m-A16-B12 (hHGF); 3) injecting 0.1 µg/animal of m-A16-B12 (hHGF); and 4) injecting 200 µg/animal of the Collategene naked plasmid. Gastrocnemius muscle samples were collected from the mice at 1 h, 2 h, 4 h, 6 h, 24 h, 48 h, 72 h, 96 h, 168 h, 216 h, and 336 h. The muscle tissues were homogenized and lysed using RIPA lysis buffer containing a protease inhibitor. The lysates were centrifuged to obtain the supernatant, and the hHGF protein concentration was measured using a BCA protein concentration assay kit. The expression levels of hHGF were assessed using an ELISA method. Data from each group were expressed as mean ± standard deviation (Mean ± SD), and plotting and statistical analysis were performed using Graphpad Prism 9.0 software.

**Table 12. The groups for the experiment of m-A16-B12 (hHGF) and Collategene expressing hHGF in vivo**

| Group | G1 | G2 | G3 | G4 |
|---|---|---|---|---|
| Number of animals | 33 | 33 | 33 | 33 |
| Test drug | Plasmid (Collategene) | m-A16-B12 (hHGF) | m-A16-B12 (hHGF) | m-A16-B12 (hHGF) |
| Dose | 200 µg | 0.1 µg | 0.3 µg | 1.0 µg |
| Route of administration | i.m. | | | |
| Frequency of | Single-dose | | | |
| administration | | | | |
| Detection indexes | Taking blood plasma and muscle samples, and measuring hHGF protein content | | | |
| Sample collection | Taking samples at 1 h/2 h/4 h/6 h/24 h/48 h/72 h/96 h/D7/D10/D14 post-administration | | | |

The experimental results are shown in FIG. 9A: after a mere 1 h following intramuscular injection, m-A16-B12 (hHGF) can express hHGF protein; at 6 h post-injection, expression peaks were achieved in a dose-dependent manner. As shown in FIG. 9B, Collategene achieved an expression peak at 7 days post-injection; m-A16-B12 (hHGF) exhibited a reduced protein expression level at 48 h post-delivery; at 72 h, the 1 µg/animal dose of m-A16-B12 (hHGF) achieved a protein expression level comparable to that of Collategene. Additionally, statistical analysis of the PK data from each group reveals that even at the lowest dose of 0.1 µg, m-A16-B12 (hHGF) achieved a Cₘₐₓ that is 5 times that of Collategene, and the AUC_{inf(hr*pg/mg protein)} of 0.1 µg of m-A16-B12 (hHGF) is also comparable to that of Collategene (Table 13). Therefore, m-A16-B12 (hHGF) can achieve efficient expression of hHGF in animals, and no hHGF expression was detected in the plasma at each time point.

**Table 13. Results for m-A16-B12 (hHGF) and Collategene expressing hHGF in vivo**

| Route | Test drug | Dose (µg) | Cₘₐₓ (pg/mg protein) | AUC_{0-inf} (hr*pg/mg protein) | MRT_{0-inf} (h) |
|---|---|---|---|---|---|
| I.M. | m-A16-B12 (hHGF) | 0.1 µg | 369.1±24.8 | 16158.3±978.7 | 201.5±73.8 |
| | | 0.3 µg | 463.1±16.5 | 20133.6±770.2 | 125.9±26.4 |
| | | 1 µg | 570.6±42.5 | 29652.8±2555.9 | 142.8±71.9 |
| | Collategene | 200 µg | 78.0±5.7 | 15300.4±491.2 | 183.5±3.0 |

### Example 5. Validation of Therapeutic Function of hHGF-Expressing mRNA in Lower Limb Ischemia Mouse Model

The lower limb ischemia mouse model is a classic mouse model for simulating human critical limb ischemia. The creation of this model involves use of a 6-8-week-old male Balb/c mouse. The method is as follows: 1) anesthetizing the animal and placing it in the supine position on the surgical table, completely shaving the hindlimbs and securing them, and disinfecting the surgical skin area; 2) making a skin incision about 1 centimeter long from the knee towards the inner thigh, and sequentially cutting and dissecting the subcutaneous fat tissue to fully expose the femoral artery; 3) using curved microforceps to gently pierce the membranous femoral sheath to expose the neurovascular bundle, isolating the femoral artery from the femoral vein and a proximal position near the groin at the nerve, and once the femoral artery is cleanly isolated, ligating the proximal end of the femoral artery below the proximal end of the femoral artery using 6-0 sutures; 4) separating the femoral artery from the femoral vein at the distal end near the knee, and ligating the end of the femoral artery below the distal end at the proximal end of the popliteal artery using 6-0 sutures; and 5) suturing the incision.

The LNP from Example 4 was used as a carrier to deliver m-A16-B12 (hHGF), and Collategene was administered in the form of a naked plasmid. The mice of the lower limb ischemia model were randomly divided into 4 groups of 5 and received intramuscular injection of m-A16-B12 (hHGF) and the Collategene naked plasmid into the gastrocnemius muscle. The experimental groups are as follows: 1) injecting 500 ng/animal of m-A16-B12 (hHGF); 2) injecting 50 ng/animal of m-A16-B12 (hHGF); 3) injecting 200 µg/animal of the Collategene naked plasmid; and 4) injecting an equal volume of PBS as a control group. The day of modeling was counted as day 0. The legs were observed on day 0, day 4, day 7, day 10, day 12, and day 14 following the treatment in each experimental group. Blood perfusion in the lower limbs of the mice was measured using a blood flowmeter, and photographs were taken.

The blood perfusion ratio was calculated for each group using the following formula: blood perfusion ratio = lower limb blood perfusion level of the mouse on that day/lower limb blood perfusion level of the mouse on day 0 × 100%; data from each group were expressed as mean ± standard deviation (Mean ± SD), and plotting and statistical analysis were performed using Graphpad Prism 9.0 software.

The results are shown in FIGs. 10A and 10B: in the case of injection treatment with 50 ng/animal and 500 ng/animal of m-A16-B12 (hHGF) and 200 ng/animal of the Collategene naked plasmid, the ischemic lower limb blood perfusion ratio of each mouse was significantly improved relative to the control group, and the ischemic lower limb blood perfusion ratio gradually recovered over time. The 50 ng/animal m-A16-B12 (hHGF) injection group exhibited a blood flow recovery effect similar to that of the 200 ng/animal Collategene naked plasmid injection group; particularly, the 500 ng/animal m-A16-B12 (hHGF) injection group exhibited a blood flow recovery effect significantly better than that of the 200 ng/animal Collategene naked plasmid group: 500 ng/animal of m-A16-B12 (hHGF) can restore blood perfusion to 90% or higher.

To further determine the therapeutic effect of m-A16-B12 (hHGF) on the degree of lower limb necrosis in the lower limb ischemia mouse model, on day 14 post-administration, the mice's legs were observed, and photographs were taken. Moreover, the degree of lower limb necrosis in the mice was scored using the criteria in FIG. 11A. At that time, the criteria used were as follows: 0 = lower limb self-detachment; 1 = leg necrosis; 2 = foot necrosis; 3 = discoloration of >2 toes; 4 = discoloration of 1 toe; 5 = discoloration of >2 nails; 6 = discoloration of 1 nail; and 7 = no necrosis. Data from each group were expressed as mean ± standard deviation (Mean ± SD), and plotting and statistical analysis were performed using Graphpad Prism 9.0 software.

The results are shown in FIG. 11B: after the induction of ischemia in the lower limbs of the mice, the mice in the control group developed significant ischemic necrosis in the lower limbs within 2 weeks of undergoing surgery, and the lower limbs of some of the mice completely fell off by day 14; by contrast, in the m-A16-B12 (hHGF) and Collategene naked plasmid injection treatment groups, the mice can all maintain good lower limb integrity and did not develop necrosis in the lower limbs, indicating that m-A16-B12 (hHGF) has an ability to ameliorate lower limb ischemia comparable to that of the control, the Collategene naked plasmid.

CD31 is an important marker of angiogenesis. To determine the role of m-A16-B12 (hHGF) in promoting angiogenesis in the lower limb ischemia mouse model, gastrocnemius muscle samples near the ischemic tissue in the mice were taken on day 14 post-administration, and paraffin sections were prepared. The CD31 in the section samples was immunohistochemically stained with a CD31 antibody, and photographs were taken. Analysis was performed using ImageJ(NIH) software, and the CD31 stained area was calculated. Data from each group were expressed as mean ± standard deviation (Mean ± SD), and plotting and statistical analysis were performed using Graphpad Prism 9.0 software.

The results are shown in FIG. 12: in the case of treatment with 50 ng/animal and 500 ng/animal of m-A16-B12 (hHGF) and 200 ng/animal of the Collategene naked plasmid, angiogenesis in the muscles of ischemic lower limbs can be significantly promoted, and the number of new blood vessels is statistically significantly different (*p* < 0.05) from that of the PBS group; the 50 ng/animal m-A16-B12 (hHGF) group exhibited an angiogenesis-promoting effect comparable to that of 200 ng/animal of the Collategene naked plasmid, and the m-A16-B12 (hHGF) administration group exhibited dose-dependent promotion of angiogenesis.

### Example 6. Validation of Therapeutic Function of hHGF-Expressing mRNA in Full-Thickness Skin Injury Db/Db Mouse Model

The Db/Db mouse model is a classic diabetes mouse model. Mice of this model have a defect in the leptin receptor gene, leading them to develop characteristics similar to those of diabetes patients as their week age increases, such as hyperglycemia, hyperlipidemia, and insulin resistance. In this experiment, a full-thickness skin injury model was used to simulate hard-to-heal skin wounds in diabetic foot ulcer patients. A method of creating the Db/Db mouse model described above is as follows: anesthetizing the Db/Db mouse, then removing its hair using a hair removal cream, disinfecting with 75% alcohol, and creating a full-thickness skin wound on the lower back using a skin sampler, biopunch, with a diameter of 8 mm.

To determine the healing effect of m-A16-B12 (hHGF) on the wound in the full-thickness skin injury mouse model, the LNP in Example 4 was used to deliver m-A16-B12 (hHGF), and the control Collategene was administered in the form of a naked plasmid by 4-point subcutaneous injection. The Db/Db mice of the full-thickness skin injury model were divided into 5 groups of 7 according to their body weight and blood glucose levels. The experimental groups are as follows: 1) injecting 500 ng/animal of m-A16-B12 (hHGF); 2) injecting 200 ng/animal of m-A16-B12 (hHGF); 3) injecting 50 ng/animal of m-A16-B12 (hHGF); 4) injecting 200 µg/animal of the Collategene naked plasmid; and 5) injecting an equal volume of PBS as a control group. The day of modeling was counted as day 0. Wound healing was observed on day 0, day 3, day 5, day 7, day 10, day 12, and day 14 following the treatment in each experimental group, and the wounds were photographed. The images were analyzed using ImageJ(NIH) software, and the wound area was calculated.

The percent wound healing was calculated using the following formula: P = (AD - A0)/A0 × 100% (P: percent wound healing; AD: wound area on the day of photographing; A0: wound area on the day of surgery).

The percent wound healing for each group was expressed as mean ± standard deviation (Mean ± SD), and plotting and statistical analysis were performed using Graphpad Prism 9.0 software.

The results are shown in FIG. 13: under injection treatment with 50 ng/animal, 200 ng/animal, and 500 ng/animal of m-A16-B12 (hHGF) and 200 µg/animal of the Collategene naked plasmid, the mice's wounds all well healed as compared to the control group. Moreover, the promoting effect of m-A16-B12 (hHGF) on the healing of the mice's wounds was dose-dependent: on day 14, the wound healing rate for the 50 ng/animal dose of m-A16-B12 (hHGF) was up to 66%, and the 200 ng/animal and 500 ng/animal doses of m-A16-B12 (hHGF) can achieve 100% wound healing; additionally, the wound healing promoting effect of m-A16-B12 (hHGF) was significantly better than 200 µg/animal of the Collategene naked plasmid (*p* < 0.05), and even the relatively low dose (50 ng/animal) of m-A16-B12 (hHGF) also exhibited a percent wound healing significantly different from that of 200 µg/animal of the Collategene naked plasmid (*p* < 0.05).

To determine the therapeutic effect of m-A16-B12 (hHGF) on tissue remodeling in the full-thickness skin injury mouse model, Masson staining was used to assess epithelial regeneration and tissue remodeling. On day 17 post-administration, full-thickness skin samples were collected from the mice, and paraffin sections were prepared. Masson staining was used to stain the mouse skin tissues.

The results are shown in FIG. 14: under treatment with m-A16-B12 (hHGF), full epithelial coverage of the wound epithelium was completed in each group of mice, and in the low-dose group, the epithelium at the wound abnormally thickened, and collagen proliferated; in the medium-dose and high-dose groups, the epithelium recovered to normal thickness, collagen was orderly arranged, and tissue remodeling was completed. By contrast, in the control group and the 200 µg/animal Collategene naked plasmid group, epithelial re-coverage was not completed, collagen abnormally proliferated at the wounds, and the structures were disordered.

### SEQUENCE LISTING

> B1 (ACTG1 3'UTR) 
CCGACTACCTCATGAAGATCCTCACTGAGCGAGGCTACAGCTTCACCACCAC
GGCCGAGCGGGAAATCGTGCGCGACATCAAGGAGAAGCTGTGCTACGTCGC
CCTGGACTTCGAGCAGGAGATGGCCACCGCCGCATCCTCCTCTTCTCTGGAG
AAGAGCTACGAGCTGCCCGATGGCCAGGTCATCACCATTGGCAATGAGCGGT
TCCGGTGTCCGGAGGCGCTGTTCCAGCCTTCCTTCCTGGGTATGGAATCTTGC
GGCATCCACGAGACCACCTTCAACTCCATCATGAAGTGTGACGTGGACATCC
GCAAAGACCTGTACGCCAACACGGTGCTGTCGGGCGGCACCACCATGTACC
CGGGCATTGCCGACAGGATGCAGAAGGAGATCACCGCCCTGGCGCCCAGCA
CCATGAAGATCAAGATCATCGCACCCCCAGAGCGCAAGTACTCGGTGTGGAT
CGGTGGCTCCATCCTGGCCTCACTGTCCACCTTCCAGCAGATGTGGATTAG
SEQ ID NO: 1
> B2 (ATP6V0B 3'UTR) 
ATGATATGTGTGGGTGGGGCCGTGCCTCACTTTTATTTATTGCTGGTTTTCCTG
GGACAGCTGGAGCTGTGTCCCTTAGCCTTTCAGAGGCTTGGTGTTCAGGGCC
CTCCCTGCACTCCCCTCTTGCTGCGTGTTGATTTGGAGGCACTGCAGTCCAG
GCCGAGTCCTCAGTGCGGGGAGCAGGCTGCTGCTGCTGACTCTGTGCAGCT
GCGCACCTGTGTCCCCCACCTCCACCCTCAACCCATCTTCCTAGTGTTTGTGA
AATAAACTTGGTATTTGTCT
SEQ ID NO: 2
> B3 (ATP6V0B 3'UTR) 
GTTGAAGAACTTCTCTCTACCGAGCAGCCCGTAACCCCCTTTTTCTCCCTCAC
TGCTGCAGGAGTCTGCTACACCATTTTTGATTTGGGCTTCCGCTTTGATGTGG
CATGGTTCCTGACGGAGACTTCGCCCTTCATGTGGTCCAACCTGGGCATTGG
CCTAGCTATCTCCCTGTCTGTGGTTGGGGCAGCCTGGGGCATCTATATTACCG
GCTCCTCCATCATTGGTGGAGGAGTGAAGGCCCCCAGGATCAAGACCAAGA
ACCTGGTCAGCATCATCTTCTGTGAGGCTGTGGCCATCTACGGCAT
SEQ ID NO: 3
> B4 (ATP6V0E1 3'UTR) 
GGAAGAAGACATGCTCTACAGTGCTCAGTCTTTGAGGTGACTATGCTTGTGA
CCTTTCTTATCAGTATGGTCAGGCAGTCAGAGTTTGCCTTGACTTTTTCTCAC
ATTTTATGGCCATTTTAACACGTGGCAAGGGCAAGCTGCTGAATGCAAACAC
GATCTGAAGAGCAGAAATAAAATTGGAACTTAAGCCTTAGCTTACCTAAATCT
CATTAAGATTCTTTTT
 SEQ ID NO: 4
> B5 (ATP6V0E1 3'UTR) 
ATGCAAAATCACCTCCAAACCAGACCACTTTTCTTGACTTGCCTGTTTTGGCC
ATTAGCTGCCTTAAACGTTAACAGCACATTTGAATGCCTTATTCTACAATGCA
GCGTGTTTTCCTTTGCCTTTTTTGCACTTTGGTGAATTACGTGCCTCCATAACC
TGAACTGTGCCGACTCCACAAAACGATTATGTACTCTTCTGAGATAGAAGATG
CTGTTCTTCTGAGAGATACGTTACTCTCTCCTTGGAATCTGTGGATTTGAAGA
TGGCTCCTGCCTTCTCACGTGGGAATCAGTGAAGTGTTTAGAAACTGCTGCA
AGACAAACAAGACTCCAGTGGGGTGGTCAGTAGGAGAGCACGTTCAGAGG
GAAGAGCCATCTCAACAGAATCGCACCAAACTATACTTTCAGGATGAATTTCT
TCTTTCTGCCATCTTTTGGAATAAATATTTTCCTCCTTTC
SEQ ID NO: 5
> B6 (CFL1 3'UTR) 
GCCCCTTCTGGCCCCCTGCCTGGAGCATCTGGCAGCCCCACACCTGCCCTTG
GGGGTTGCAGGCTGCCCCCTTCCTGCCAGACCGGAGGGGCTGGGGGGATCC
CAGCAGGGGGAGGGCAATCCCTTCACCCCAGTTGCCAAACAGACCCCCCAC
CCCCTGGATTTTCCTTCTCCCTCCATCCCTTGACGGTTCTGGCCTTCCCAAAC
TGCTTTTGATCTTTTGATTCCTCTTGGGCTGAAGCAGACCAAGTTCCCCCCAG
GCACCCCAGTTGTGGGGGAGCCTGTATTTTTTTTAACAACATCCCCATTCCCC
ACCTGGTCCTCCCCCTTCCCATGCTGCCAACTTCTAACCGCAATAGTGACTCT
GTGCTTGTCTGTTTAGTTCTGTGTATAAATGGAATGTTGTGGAGATGACCCCT
CCCTGTGCCGGCTGGTTCCTCTCCCTTTTCCCCTGGTCACGGCTACTCATGGA
AGCAGGACCAGTAAGGGACCTTCGATT
SEQ ID NO: 6
> B7 (CFL1 3'UTR) 
GCCCCTTCTGGCCCCCTGCCTGGAGCATCTGGCAGCCCCACACCTGCCCTTG
GGGGTTGCAGGCTGCCCCCTTCCTGCCAGACCGGAGGGGCTGGGGGGATCC
CAGCAGGGGGAGGGCAATCCCTTCACCCCAGTTGCCAAACAGACCCCCCAC
CCCCTGGATTTTCCTTCTCCCTCCATCCCTTGACGGTTCTGGCCTTCCCAAAC
TGCTTTTGATCTTTTGATTCCTCTTGGGCTGAAGCAGACCAAGTTCCCCCCAG
GCACCCCAGTTGTGGGGGAGCC
SEQ ID NO: 7
> B8 (CFL1 3'UTR) 
GCCCCTTCTGGCCCCCTGCCTGGAGCATCTGGCAGCCCCACACCTGCCCTTG
GGGGTTGCAGGCTGCCCCCTTCCTGCCAGACCGGAGGGGCTGGGGGGATCC
CAGCAGGGGGAGGGCAATCCCTTCACCCCAGTTGCCAAACAGACCCCCCAC
CCCCTGGATTTTCCTTCTCCCTCCATCCCTTGACGGTTCTGGCCTTCCCAAAC
TGCTTTTGATCTTTTGATTCCTCTTGGGCTGAAGCAGACCAAGTTCCCCCCAG
GCACCCCAGTTGTGGGGGAGCCTGTATTTTTTTTAACAACATCCCCATTCCCC
ACCTGGTCCTCCCCCTTCCCATGCTGCCAACTTCTAACCGCAATAGTG
 SEQ ID NO: 8
> B9 (COX4I1 3'UTR) 
GAGATGCTGGCCTGCGCCTGCACCTGCGCCTGGCTCTGTCACCGCCATGCAA
CTCCATGCCTATTTACTGGAAACCTGTTATGCCAAACAGTTGTACCACTGCTA
ATAAATGACCAGTTTACCTGAAA
SEQ ID NO: 9
> B10 (COX4I1 3'UTR) 
AGGGACCCACAGGCGCGCCCAGCAGCTCTCGGAAGCGTTGTATCGCATTAAG
TTCAAGGAGAGCTTTGCTGAGATGAACAGGGGCTCGAACGAGTGGAAGACG
GTTGTGGGCGGTGCCATGTTCTTCATCGGTTTCACCGCGCTCGTTATCATGTG
GCAGAAGCACTATGGGCTTAGCCTCCAAGTGGGACTACGAAAAGAACGAGT
GGAAGAAGTGAGAGATGCTGGCCTGCGCCTGCACCTGCGCCTGGCTCTGTC
ACCGCCATGCAACTCCATGCCTATTTACTGGAAACCTGTTATGCCAAACAGTT
GTACCACT
SEQ ID NO: 10
> B11 (COX4I1 3'UTR) 
AGAGGACCTTCTGCTTTTAGCTTATTTTGTTGCTAACTTTTTACAAACAAAGG
GCTAATTTTAAAATGTCAGTGTTGTCAGTGGTCAGAAACCGTGTGTTTGAGC
GGGTGTTGAGTGGCAGGTGGCTCTGCTGACCTGGTGGCTGGTGTGTCGGGA
GGATTTAACCTGTGTGAGGGATTGGCCTAGAAACAACCTGTTGAGATAGTCT
TGCCCCATAACCTGTCTCACACCGTAGTGTACGGCCCCCTCCCGCAAAGCTTT
GACAAAGAGTGGGTGGCCAAGCAGACCAAGAGGATGCTGGAC
SEQ ID NO: 11
> B12 (CTSB 3'UTR) 
TCTGCCGTGGGCCTGTCGTGCCAGTCCTGGGGGCGAGATCGGGAGAATCAC
GTCCTGGAACTGCATGTTCTTGCGACTCTTGGGACTTCATCTTAACTTCTCGC
TGCCCCAGCCATGTTTTCAACCATGGCATCCCTCCCCCAATTAGTTCCCTGTC
ATCCTCGTCAACCTTCTCTGTAAGTGCCTGGTAAGCTTGCCCTTGCTTAAGAA
CTCAAAACATAGCTGTGCTCTA
SEQ ID NO: 12
> B13 (FAM166A 3'UTR) 
AAATGCTTTAATGGTGGTGTCTGTACAGCATGGATGTGGACAGGGAAAACAG
CAAGTGCACACAGTGGGACAGGCCACAGGACAGGCTGGGAGTGAATAAAG
AGTTCACACTGCT
SEQ ID NO: 13
> B14 (NDUFB9 3'UTR) 
TATCTAAGTGTATCAATTTAACATTTATAATCACCTTACAAGTAAGTTAAAGTG
CATTCCCTGGTCGTTTTGGCTTTGTCTTTGATTTTGCTTCAGATCTCCTTCTCT
TTAGTCCTTTGTCTGACTTTTTTGCCTTCTTGCTATACTTTCCCTCAACCTGCC
TCTCCACTATTTTCTTAGGCATTTTTCAGTCATG
 SEQ ID NO: 14
> A1 (ACTG1 5'UTR) 
CTCTCGCACTCTGTTCTTCCGCCGCTCCGCCGTCGCGTTTCTCTGCCGGTCGC
A
SEQ ID NO: 15
> A2 (ATP6V0B 5'UTR) 
GGGCGGGCGGACAGACTGCGGGACGGACGGTGGACGCTGGGACGCGTTTGT
AGCTCCGGCCCCGCCGTTCCGACCCCCGCCGCCGTCGCCGCCATGACGGGGC
TAGCACTGCTCTACTCCGGGGTCTTCGTGGCCTTCTGGGCCTGCGCGCTGGC
CGTGGGTTCCTGACGGAGACTTCGCCCTTC
SEQ ID NO: 16
> A3 (ATP6V0B 5'UTR) 
AGACTGCGGGACGGACGGTGGACGCTGGGACGCGTTTGTAGCTCCGGCCCC
GCCGTTCCGACCCCCGCCGCCGTCGCCGCC
SEQ ID NO: 17
> A4 (ATP6V0E1 5'UTR)
   GGGGTAGGGGTTGGCGGCTCAGGCGGCGACC SEQ ID NO: 18
A% (ATP6V0E1 5'UTR) 
GTCACGCGGTCAGCTATTGACACTTCCTGGTGGGATCCGAGTGAGGCGACGG
GGTAGGGGTTGGCGCTCAGGCGGCGACC
SEQ ID NO: 19
> A6 (CFL1 5'UTR) 
GGCCGGCGGGAAGACTCCGTTACCCAGCGAGCGAGGCGGCGCAGGGC
CAGCGGACTCCATTTCCCGTCGGCTCGCGGTGGGAGCGCCGGAAGCCCGCC
CCACCCCTCATTGTGCGGCTCCTACTAAACGGAAGGGGCCGGGAGAGGCCG
CGTTCAGTCGGGTCCCGGCAGCGGCTGCAGCGCTCTCGTCTTCTGCGGCTCT
CGGTGCCCTCTCCTTTTCGTTTCCGGAAAC
SEQ ID NO: 20
> A7 (CFL1 5'UTR) 
CTCCTGGCTCGGGCACCCCGAAGCACGACGCAGAGTAGGAAGAGTTTAAAG
ACCTCGAGGCCTCTGGGCACTTGAGTTTGGCCTCCGGTGTGGCTGTCTCTGA
TGGTGTCATCAAGGTGTTCAACGAC
SEQ ID NO: 21
> A8 (CFL1 5'UTR) 
ACGTGGCCGGCTTCTTCTGCAGTTCCGGGGAGTTTGGGGGACCAGATTTACC
TTGGATTGCCCCTCCCTCTCCTGGCTCGGGCACCCCGAAGCACGACGCAGAG
CCTCCGGTGTGGCTGTCTCTGATGGTGTCATCAAGGTGTTCAACGAC
SEQ ID NO: 22
> A9 (COX4I1 5'UTR) 
GCGGCCTTGCTCTCTTCCGGTCGCGGGACACCGGGTGTAGAGGGCGGTCGC
GGCGGGCAGTGGCGGCAGA
SEQ ID NO: 23
> A10 (COX4I1 5'UTR) 
GGGACACCGGGTGTAGAGGGCGGTCGCGGCGGGCAGTGGCGGCAGA
SEQ ID NO: 24
> A11 (COX4I1 5'UTR) 
CGCGGCCTTGCTCTCTTCCGGTCGCGGGACACCGGGTGTAGAGGGCGGTCGC
GGCGGGCAGTGGCGGCAGA
SEQ ID NO: 25
> A12 (CTSB 5'UTR) 
GGCCGGGGCTGGCCCAGGCTACGGCGGCTGCAGGGCTCCGGCAACCGCTCC
GGCAACGCCAACCGCTCCGCTGCGCGCAGGCTGGGCTGCAGGCTCTCGGCT
GCAGCGCTGGGTGGATCTAGGATCCGGCTTCCAAC
SEQ ID NO: 26
> A13 (FAM166A 5'UTR) 
AAGGGAGCTGGATGCCGGGAGGGACTGGAGCCAGCAAGGCCAGAGTGAAA
GCAAA
SEQ ID NO: 27
> A14 (NDUFB9 5'UTR) 
GCAGCAGGCGTGCAGTTTCCCGGCTCTCCGCGGCCGGGGAAGGTCAGCG
CCGTA
SEQ ID NO: 28
> A15 (CHCHD10 5'UTR) 
GGCATTTGTCCCCGCGACAGCACCGCTGCCGCCGTCTCTAAGGTCGCCCGGG
TCCCACCGCCGCCACC
SEQ ID NO: 29
> A16 (CHCHD10 5'UTR) 
CCGCTGCCGCCGTCTCTAAGGTCGCCCGGGTCCCACCGCCGCCACC
SEQ ID NO: 30
> A17 (SLC38A2 5'UTR) 
ATGTCTTTTTTGTGTGTTTGTTTTCATGGTATTCCTATGAA
SEQ ID NO: 31
> A18 (NDUFA11 5'UTR) 
GCTTCCCGAGCTGGCGGGGTCCGTGGTGCGGGATCGAGATTGCGGGCT
SEQ ID NO: 32
> A19 (NDUFV3 5'UTR) 
GGCGGCTGTTCAGGCGCGGGTGCGCGCGCAGCTGCTGTGGCCCTGCTTGGT
GCGCCCGCTGTCACCGCC
SEQ ID NO: 33
> A20 (PRDX5 5'UTR) 
CGCGCCTGCGCAGTGGAGGCGGCCCAGGCCCGCCTTCCGCAGGGTGTCGCC
GCTGTGCCGCTAGCGGTGCCCCGCCTGCTGCGGTGGCACCAGCCAGGAGGC
GGAGTGGAAGTGGCCGTGGGGCGGGT
SEQ ID NO: 34
> A21 (GUK1 5'UTR) 
CAGCAGATGGGGACTAGAGGCCGCACTGCTATCCACAGCCTCTCTTCTCACC
CCCAGGC
SEQ ID NO: 35
> A22 (GUK1 5'UTR) 
GCTGGCCGGGCTGGCTGCGGCCGCCCTGGGCCGGGCCCCACCGGACGCCTC
TCTTCTCACCCCCAGGC
SEQ ID NO: 36
> A23 (GUK1 5'UTR) 
AGAGGTGGCCCCGGATGCTGCGGCGCCCGCTGGCCGGGCTGGCTGCGGCCG
CCCTGGGCCGGGCCCCACCGGACGCCTCTCTTCTCACCCCCAGGC
SEQ ID NO: 37
> A24 (IAH1 5'UTR) 
TGGCTGGCGGCCCCGCCCCGCCCCGCCCGGCTGCTCC
SEQ ID NO: 38
> A25 (ABHD16A 5'UTR) 
GGAGGGCGGGGCCGGCAGGGGGACCTGCTGCTGGAAGAGCAGCGGCCCGA
GCCGGGGCC
SEQ ID NO: 39
> A26 (SLC25A39 5'UTR) 
TTTCGGCGTCGGCCTAGGTGCGCTGCGAGCGCGCGCGGACCGCGCACAGGC
GGCGGAGCCGGTATGGGCCCGCCTGACCCTGGGCGCCGCGCCGCACGAGCA
CCAGCCTAGAGCCAGGACTGAAGCTTCAAG
SEQ ID NO: 40
> A27 (ATPIF1 5'UTR) 
CGAGAGACTGCTTGCTGCGGCAGAGACGCCAGAGGTGCAGCTCCAGCAGCA
SEQ ID NO: 41
> A28 (ANAPC11 5'UTR) 
CGGAGTTTCGTCATGTTGGCCAGGCCCATTTGAGATCTTTGAAGATATCCTCA
ACGTGAGGCTCTGCTGCC
SEQ ID NO: 42
> A29 (ANAPC11 5'UTR) 
GGGCGCGGCTTCGGCGGGCGGCAGCCGCTGCAGACGAGCTGCGGGCTCTGC
TGCC
SEQ ID NO: 43
 
> A30 (CCDC12 5'UTR)
GCCTGCGCGATGCAAGACGGAGAAAGGAGGGCGTACGCGGCAAG
SEQ ID NO: 44
> A31 (MRPL14 5'UTR) 
ATCGCGTCCGCCGGGCTGGGCCTGGCGCGCAGGCGCTAGGAAGAGGCCGCG
TGGGGCGAAGGCGGCGCTTGGCTGGTGGGGCCCGCGGCGGGATTTTCCCGG
GCGGCGAGAGCGGATCTATCTTGGGATCCC
SEQ ID NO: 45
> A32 (APOA1BP 5'UTR) 
GCCGGGGGCGCGCTCTGCGAGCTGG
SEQ ID NO: 46
> A33 (APOA1BP 5'UTR) 
GCCGGGGCCGGGCCGGGCCGGGGGCGCGCTCTGCGAGCTGG
SEQ ID NO: 47
> Mod. 5'UTR 
GGGAAATAAGAGAGAAAAGAAGAGTAAGAAGAAATATAAGAGCCACC
SEQ ID NO: 48
> α-globin 3'UTR 
TGATAATAGGCTGGAGCCTCGGTGGCCATGCTTCTTGCCCCTTGGGCC
TCCCCCCAGCCCCTCCTCCCCTTCCTGCACCCGTACCCCCGTGGTCTT
TGAATAAAGTCTGAGTGGGCGGC
SEQ ID NO: 49
> α-1 globin 5'UTR 
AGACGAACUAGUAUUCUUCUGGUCCCCACAGACUCAGAGAGAACCCGCCA
CC
SEQ ID NO: 50
> haplogroup U8blb1 mitochondrion 3'UTR 
CTCGAGCTGGTACTGCATGCACGCAATGCTAGCTGCCCCTTTCCCGTCCTGGG
TACCCCGAGTCTCCCCCGACCTCGGGTCCCAGGTATGCTCCCACCTCCACCT
GCCCCACTCACCACCTCTGCTAGTTCCAGACACCTGCTATACTAATCGCTAGC
CGCGTCGCTCCCCAGGGTTGGTCAATTTCGTGCCAGCCACACCGAGACCTGG
TCCAGAGCCCAAGCACGCCACACCCCCACGGGAAACAGCAGTGATTAACCT
TTAGCAATAAACGAAAGTTTAACTAACAGCAATGCA
GCTCAAAACGCTTAGCCTAG
SEQ ID NO: 51
> A30L70 PolyA 
AAAAAAAAAAAAAAAAAAAAAAAAAAAAAAGCATATGACTAAAAAAAAAA
AAAAAAAAAAAAAAAAAAAAAAAAAAAAAAAAAAAAAAAAAAAAAAAAAA
AAAAAAAAAAA
SEQ ID NO: 52
> 5'UTR-Fluc-α globin 3'UTR-120A (Mod.-120A) 
GGGAAATAAGAGAGAAAAGAAGAGTAAGAAGAAATATAAGAGCCACCATGG
AAGACGCCAAAAACATAAAGAAAGGCCCGGCGCCATTCTATCCGCTGGAAG
ATGGAACCGCTGGAGAGCAACTGCATAAGGCTATGAAGAGATACGCCCTGGT
TCCTGGAACAATTGCTTTTACAGATGCACATATCGAGGTGGACATCACTTACG
CTGAGTACTTCGAAATGTCCGTTCGGTTGGCAGAAGCTATGAAACGATATGG
GCTGAATACAAATCACAGAATCGTCGTATGCAGTGAAAACTCTCTTCAATTCT
TTATGCCGGTGTTGGGCGCGTTATTTATCGGAGTTGCAGTTGCGCCCGCGAAC
GACATTTATAATGAACGTGAATTGCTCAACAGTATGGGCATTTCGCAGCCTAC
CGTGGTGTTCGTTTCCAAAAAGGGGTTGCAAAAAATTTTGAACGTGCAAAA
AAAGCTCCCAATCATCCAAAAAATTATTATCATGGATTCTAAAACGGATTACC
AGGGATTTCAGTCGATGTACACGTTCGTCACATCTCATCTACCTCCCGGTTTT
AATGAATACGATTTTGTGCCAGAGTCCTTCGATAGGGACAAGACAATTGCAC
TGATCATGAACTCCTCTGGATCTACTGGTCTGCCTAAAGGTGTCGCTCTGCCT
CATAGAACTGCCTGCGTGAGATTCTCGCATGCCAGAGATCCTATTTTTGGCAA
TCAAATCATTCCGGATACTGCGATTTTAAGTGTTGTTCCATTCCATCACGGTTT
TGGAATGTTTACTACACTCGGATATTTGATATGTGGATTTCGAGTCGTCTTAAT
GTATAGATTTGAAGAAGAGCTGTTTCTGAGGAGCCTTCAGGATTACAAGATTC
AAAGTGCGCTGCTGGTGCCAACCCTATTCTCCTTCTTCGCCAAAAGCACTCT
GATTGACAAATACGATTTATCTAATTTACACGAAATTGCTTCTGGTGGCGCTCC
CCTCTCTAAGGAAGTCGGGGAAGCGGTTGCCAAGAGGTTCCATCTGCCAGGT
ATCAGGCAAGGATATGGGCTCACTGAGACTACATCAGCTATTCTGATTACACC
CGAGGGGGATGATAAACCGGGCGCGGTCGGTAAAGTTGTTCCATTTTTTGAA
GCGAAGGTTGTGGATCTGGATACCGGGAAAACGCTGGGCGTTAATCAAAGA
GGCGAACTGTGTGTGAGAGGTCCTATGATTATGTCCGGTTATGTAAACAATCC
GGAAGCGACCAACGCCTTGATTGACAAGGATGGATGGCTACATTCTGGAGAC
ATAGCTTACTGGGACGAAGACGAACACTTCTTCATCGTTGACCGCCTGAAGT
CTCTGATTAAGTACAAAGGCTATCAGGTGGCTCCCGCTGAATTGGAATCCATC
TTGCTCCAACACCCCAACATCTTCGACGCAGGTGTCGCAGGTCTTCCCGACG
ATGACGCCGGTGAACTTCCCGCCGCCGTTGTTGTTTTGGAGCACGGAAAGAC
GATGACGGAAAAAGAGATCGTGGATTACGTCGCCAGTCAAGTAACAACCGC
GAAAAAGTTGCGCGGAGGAGTTGTGTTTGTGGACGAAGTACCGAAAGGTCT
TACCGGAAAACTCGACGCAAGAAAAATCAGAGAGATCCTCATAAAGGCCAA
GAAGGGCGGAAAGATCGCCGTGGATTACAAGGATGACGACGATAAGTAAGG
GCCCACCGGTTGATAATAGGCTGGAGCCTCGGTGGCCATGCTTCTTGCCCCTT
GGGCCTCCCCCCAGCCCCTCCTCCCCTTCCTGCACCCGTACCCCCGTGGTCTT
TGAATAAAGTCTGAGTGGGCGGCCTCGAGCCGCGGAAAAAAAAAAAAAAA
AAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAA
AAAAAAATTATTGTTTAAACATTTAAATTCTAGA
 SEQ ID NO: 53
> 5'UTR-Fluc-α globin 3'UTR-A30L70 (Mod.-A30L70) 
GGGAAATAAGAGAGAAAAGAAGAGTAAGAAGAAATATAAGAGCCACCATGG
AAGACGCCAAAAACATAAAGAAAGGCCCGGCGCCATTCTATCCGCTGGAAG
ATGGAACCGCTGGAGAGCAACTGCATAAGGCTATGAAGAGATACGCCCTGGT
TCCTGGAACAATTGCTTTTACAGATGCACATATCGAGGTGGACATCACTTACG
CTGAGTACTTCGAAATGTCCGTTCGGTTGGCAGAAGCTATGAAACGATATGG
GCTGAATACAAATCACAGAATCGTCGTATGCAGTGAAAACTCTCTTCAATTCT
TTATGCCGGTGTTGGGCGCGTTATTTATCGGAGTTGCAGTTGCGCCCGCGAAC
GACATTTATAATGAACGTGAATTGCTCAACAGTATGGGCATTTCGCAGCCTAC
CGTGGTGTTCGTTTCCAAAAAGGGGTTGCAAAAAATTTTGAACGTGCAAAA
AAAGCTCCCAATCATCCAAAAAATTATTATCATGGATTCTAAAACGGATTACC
AGGGATTTCAGTCGATGTACACGTTCGTCACATCTCATCTACCTCCCGGTTTT
AATGAATACGATTTTGTGCCAGAGTCCTTCGATAGGGACAAGACAATTGCAC
TGATCATGAACTCCTCTGGATCTACTGGTCTGCCTAAAGGTGTCGCTCTGCCT
CATAGAACTGCCTGCGTGAGATTCTCGCATGCCAGAGATCCTATTTTTGGCAA
TCAAATCATTCCGGATACTGCGATTTTAAGTGTTGTTCCATTCCATCACGGTTT
TGGAATGTTTACTACACTCGGATATTTGATATGTGGATTTCGAGTCGTCTTAAT
GTATAGATTTGAAGAAGAGCTGTTTCTGAGGAGCCTTCAGGATTACAAGATTC
AAAGTGCGCTGCTGGTGCCAACCCTATTCTCCTTCTTCGCCAAAAGCACTCT
GATTGACAAATACGATTTATCTAATTTACACGAAATTGCTTCTGGTGGCGCTCC
CCTCTCTAAGGAAGTCGGGGAAGCGGTTGCCAAGAGGTTCCATCTGCCAGGT
ATCAGGCAAGGATATGGGCTCACTGAGACTACATCAGCTATTCTGATTACACC
CGAGGGGGATGATAAACCGGGCGCGGTCGGTAAAGTTGTTCCATTTTTTGAA
GCGAAGGTTGTGGATCTGGATACCGGGAAAACGCTGGGCGTTAATCAAAGA
GGCGAACTGTGTGTGAGAGGTCCTATGATTATGTCCGGTTATGTAAACAATCC
GGAAGCGACCAACGCCTTGATTGACAAGGATGGATGGCTACATTCTGGAGAC
ATAGCTTACTGGGACGAAGACGAACACTTCTTCATCGTTGACCGCCTGAAGT
CTCTGATTAAGTACAAAGGCTATCAGGTGGCTCCCGCTGAATTGGAATCCATC
TTGCTCCAACACCCCAACATCTTCGACGCAGGTGTCGCAGGTCTTCCCGACG
ATGACGCCGGTGAACTTCCCGCCGCCGTTGTTGTTTTGGAGCACGGAAAGAC
GATGACGGAAAAAGAGATCGTGGATTACGTCGCCAGTCAAGTAACAACCGC
GAAAAAGTTGCGCGGAGGAGTTGTGTTTGTGGACGAAGTACCGAAAGGTCT
TACCGGAAAACTCGACGCAAGAAAAATCAGAGAGATCCTCATAAAGGCCAA
GAAGGGCGGAAAGATCGCCGTGGATTACAAGGATGACGACGATAAGTAAGG
GCCCACCGGTTGATAATAGGCTGGAGCCTCGGTGGCCATGCTTCTTGCCCCTT
GGGCCTCCCCCCAGCCCCTCCTCCCCTTCCTGCACCCGTACCCCCGTGGTCTT
TGAATAAAGTCTGAGTGGGCGGCCTCGAGCCGCGGAAAAAAAAAAAAAA
AAAAAAAAAAAAAAAAGCATATGACTAAAAAAAAAAAAAAAAAAAAAAAAA
AAAAAAAAAAAAAAAAAAAAAAAAAAAAAAAAAAAAAAAAAAAAATTATT
 
GTTTAAACATTTAAATTCTAGA
SEQ ID NO: 54
> α-1-globin 5'UTR-Fluc-haplogroup U8blb1 mitochondrion 3'UTR-A30L70 (BioN.) 
AGACGAACUAGUAUUCUUCUGGUCCCCACAGACUCAGAGAGAACCCGCCA
CCATGGAAGACGCCAAAAACATAAAGAAAGGCCCGGCGCCATTCTATCCGCT
GGAAGATGGAACCGCTGGAGAGCAACTGCATAAGGCTATGAAGAGATACGC
CCTGGTTCCTGGAACAATTGCTTTTACAGATGCACATATCGAGGTGGACATCA
CTTACGCTGAGTACTTCGAAATGTCCGTTCGGTTGGCAGAAGCTATGAAACG
ATATGGGCTGAATACAAATCACAGAATCGTCGTATGCAGTGAAAACTCTCTTC
AATTCTTTATGCCGGTGTTGGGCGCGTTATTTATCGGAGTTGCAGTTGCGCCC
GCGAACGACATTTATAATGAACGTGAATTGCTCAACAGTATGGGCATTTCGCA
GCCTACCGTGGTGTTCGTTTCCAAAAAGGGGTTGCAAAAAATTTTGAACGTG
CAAAAAAAGCTCCCAATCATCCAAAAAATTATTATCATGGATTCTAAAACGGA
TTACCAGGGATTTCAGTCGATGTACACGTTCGTCACATCTCATCTACCTCCCG
GTTTTAATGAATACGATTTTGTGCCAGAGTCCTTCGATAGGGACAAGACAATT
GCACTGATCATGAACTCCTCTGGATCTACTGGTCTGCCTAAAGGTGTCGCTCT
GCCTCATAGAACTGCCTGCGTGAGATTCTCGCATGCCAGAGATCCTATTTTTG
GCAATCAAATCATTCCGGATACTGCGATTTTAAGTGTTGTTCCATTCCATCACG
GTTTTGGAATGTTTACTACACTCGGATATTTGATATGTGGATTTCGAGTCGTCT
TAATGTATAGATTTGAAGAAGAGCTGTTTCTGAGGAGCCTTCAGGATTACAAG
ATTCAAAGTGCGCTGCTGGTGCCAACCCTATTCTCCTTCTTCGCCAAAAGCAC
TCTGATTGACAAATACGATTTATCTAATTTACACGAAATTGCTTCTGGTGGCGC
TCCCCTCTCTAAGGAAGTCGGGGAAGCGGTTGCCAAGAGGTTCCATCTGCCA
GGTATCAGGCAAGGATATGGGCTCACTGAGACTACATCAGCTATTCTGATTAC
ACCCGAGGGGGATGATAAACCGGGCGCGGTCGGTAAAGTTGTTCCATTTTTT
GAAGCGAAGGTTGTGGATCTGGATACCGGGAAAACGCTGGGCGTTAATCAA
AGAGGCGAACTGTGTGTGAGAGGTCCTATGATTATGTCCGGTTATGTAAACAA
TCCGGAAGCGACCAACGCCTTGATTGACAAGGATGGATGGCTACATTCTGGA
GACATAGCTTACTGGGACGAAGACGAACACTTCTTCATCGTTGACCGCCTGA
AGTCTCTGATTAAGTACAAAGGCTATCAGGTGGCTCCCGCTGAATTGGAATCC
ATCTTGCTCCAACACCCCAACATCTTCGACGCAGGTGTCGCAGGTCTTCCCG
ACGATGACGCCGGTGAACTTCCCGCCGCCGTTGTTGTTTTGGAGCACGGAAA
GACGATGACGGAAAAAGAGATCGTGGATTACGTCGCCAGTCAAGTAACAAC
CGCGAAAAAGTTGCGCGGAGGAGTTGTGTTTGTGGACGAAGTACCGAAAGG
TCTTACCGGAAAACTCGACGCAAGAAAAATCAGAGAGATCCTCATAAAGGC
CAAGAAGGGCGGAAAGATCGCCGTGGATTACAAGGATGACGACGATAAGTA
AGGGCCCACCGGCTCGAGCTGGTACTGCATGCACGCAATGCTAGCTGCCCCT
TTCCCGTCCTGGGTACCCCGAGTCTCCCCCGACCTCGGGTCCCAGGTATGCTC
CCACCTCCACCTGCCCCACTCACCACCTCTGCTAGTTCCAGACACCTGCTATA
CTAATCGCTAGCCGCGTCGCTCCCCAGGGTTGGTCAATTTCGTGCCAGCCAC
 
ACCGAGACCTGGTCCAGAGCCCAAGCACGCCACACCCCCACGGGAAACAGC
AGTGATTAACCTTTAGCAATAAACGAAAGTTTAACTAACAGCAATGCAGCTC
AAAACGCTTAGCCTAGCTCGAGCCGCGGAAAAAAAAAAAAAAAAAAAAA
AAAAAAAAAGCATATGACTAAAAAAAAAAAAAAAAAAAAAAAAAAAAAA
AAAAAAAAAAAAAAAAAAAAAAAAAAAAAAAAAAAAAAAATTATTGTTTAAA
CATTTAAATTCTAGA
SEQ ID NO: 55
> 5'UTR-Fluc-ACTG1 3'UTR-120A (B1) 
GGGAAATAAGAGAGAAAAGAAGAGTAAGAAGAAATATAAGAGCCACCATGG
AAGACGCCAAAAACATAAAGAAAGGCCCGGCGCCATTCTATCCGCTGGAAG
ATGGAACCGCTGGAGAGCAACTGCATAAGGCTATGAAGAGATACGCCCTGGT
TCCTGGAACAATTGCTTTTACAGATGCACATATCGAGGTGGACATCACTTACG
CTGAGTACTTCGAAATGTCCGTTCGGTTGGCAGAAGCTATGAAACGATATGG
GCTGAATACAAATCACAGAATCGTCGTATGCAGTGAAAACTCTCTTCAATTCT
TTATGCCGGTGTTGGGCGCGTTATTTATCGGAGTTGCAGTTGCGCCCGCGAAC
GACATTTATAATGAACGTGAATTGCTCAACAGTATGGGCATTTCGCAGCCTAC
CGTGGTGTTCGTTTCCAAAAAGGGGTTGCAAAAAATTTTGAACGTGCAAAA
AAAGCTCCCAATCATCCAAAAAATTATTATCATGGATTCTAAAACGGATTACC
AGGGATTTCAGTCGATGTACACGTTCGTCACATCTCATCTACCTCCCGGTTTT
AATGAATACGATTTTGTGCCAGAGTCCTTCGATAGGGACAAGACAATTGCAC
TGATCATGAACTCCTCTGGATCTACTGGTCTGCCTAAAGGTGTCGCTCTGCCT
CATAGAACTGCCTGCGTGAGATTCTCGCATGCCAGAGATCCTATTTTTGGCAA
TCAAATCATTCCGGATACTGCGATTTTAAGTGTTGTTCCATTCCATCACGGTTT
TGGAATGTTTACTACACTCGGATATTTGATATGTGGATTTCGAGTCGTCTTAAT
GTATAGATTTGAAGAAGAGCTGTTTCTGAGGAGCCTTCAGGATTACAAGATTC
AAAGTGCGCTGCTGGTGCCAACCCTATTCTCCTTCTTCGCCAAAAGCACTCT
GATTGACAAATACGATTTATCTAATTTACACGAAATTGCTTCTGGTGGCGCTCC
CCTCTCTAAGGAAGTCGGGGAAGCGGTTGCCAAGAGGTTCCATCTGCCAGGT
ATCAGGCAAGGATATGGGCTCACTGAGACTACATCAGCTATTCTGATTACACC
CGAGGGGGATGATAAACCGGGCGCGGTCGGTAAAGTTGTTCCATTTTTTGAA
GCGAAGGTTGTGGATCTGGATACCGGGAAAACGCTGGGCGTTAATCAAAGA
GGCGAACTGTGTGTGAGAGGTCCTATGATTATGTCCGGTTATGTAAACAATCC
GGAAGCGACCAACGCCTTGATTGACAAGGATGGATGGCTACATTCTGGAGAC
ATAGCTTACTGGGACGAAGACGAACACTTCTTCATCGTTGACCGCCTGAAGT
CTCTGATTAAGTACAAAGGCTATCAGGTGGCTCCCGCTGAATTGGAATCCATC
TTGCTCCAACACCCCAACATCTTCGACGCAGGTGTCGCAGGTCTTCCCGACG
ATGACGCCGGTGAACTTCCCGCCGCCGTTGTTGTTTTGGAGCACGGAAAGAC
GATGACGGAAAAAGAGATCGTGGATTACGTCGCCAGTCAAGTAACAACCGC
GAAAAAGTTGCGCGGAGGAGTTGTGTTTGTGGACGAAGTACCGAAAGGTCT
TACCGGAAAACTCGACGCAAGAAAAATCAGAGAGATCCTCATAAAGGCCAA
 
GAAGGGCGGAAAGATCGCCGTGGATTACAAGGATGACGACGATAAGTAAGG
GCCCACCGGCCGACTACCTCATGAAGATCCTCACTGAGCGAGGCTACAGCTT
CACCACCACGGCCGAGCGGGAAATCGTGCGCGACATCAAGGAGAAGCTGTG
CTACGTCGCCCTGGACTTCGAGCAGGAGATGGCCACCGCCGCATCCTCCTCT
TCTCTGGAGAAGAGCTACGAGCTGCCCGATGGCCAGGTCATCACCATTGGCA
ATGAGCGGTTCCGGTGTCCGGAGGCGCTGTTCCAGCCTTCCTTCCTGGGTAT
GGAATCTTGCGGCATCCACGAGACCACCTTCAACTCCATCATGAAGTGTGAC
GTGGACATCCGCAAAGACCTGTACGCCAACACGGTGCTGTCGGGCGGCACC
ACCATGTACCCGGGCATTGCCGACAGGATGCAGAAGGAGATCACCGCCCTGG
CGCCCAGCACCATGAAGATCAAGATCATCGCACCCCCAGAGCGCAAGTACTC
GGTGTGGATCGGTGGCTCCATCCTGGCCTCACTGTCCACCTTCCAGCAGATG
TGGATTAGCTCGAGCCGCGGAAAAAAAAAAAAAAAAAAAAAAAAAAAAAAA
AAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAATTATTGTTT
AAACATTTAAATTCTAGA
SEQ ID NO: 56
> ACTG1 5'UTR-Fluc-α globin 3'UTR-120A (A1) 
CTCTCGCACTCTGTTCTTCCGCCGCTCCGCCGTCGCGTTTCTCTGCCGGTCGC
AGCCACCATGGAAGACGCCAAAAACATAAAGAAAGGCCCGGCGCCATTCTA
TCCGCTGGAAGATGGAACCGCTGGAGAGCAACTGCATAAGGCTATGAAGAG
ATACGCCCTGGTTCCTGGAACAATTGCTTTTACAGATGCACATATCGAGGTGG
ACATCACTTACGCTGAGTACTTCGAAATGTCCGTTCGGTTGGCAGAAGCTATG
AAACGATATGGGCTGAATACAAATCACAGAATCGTCGTATGCAGTGAAAACT
CTCTTCAATTCTTTATGCCGGTGTTGGGCGCGTTATTTATCGGAGTTGCAGTTG
CGCCCGCGAACGACATTTATAATGAACGTGAATTGCTCAACAGTATGGGCATT
TCGCAGCCTACCGTGGTGTTCGTTTCCAAAAAGGGGTTGCAAAAAATTTTGA
ACGTGCAAAAAAAGCTCCCAATCATCCAAAAAATTATTATCATGGATTCTAAA
ACGGATTACCAGGGATTTCAGTCGATGTACACGTTCGTCACATCTCATCTACC
TCCCGGTTTTAATGAATACGATTTTGTGCCAGAGTCCTTCGATAGGGACAAGA
CAATTGCACTGATCATGAACTCCTCTGGATCTACTGGTCTGCCTAAAGGTGTC
GCTCTGCCTCATAGAACTGCCTGCGTGAGATTCTCGCATGCCAGAGATCCTAT
TTTTGGCAATCAAATCATTCCGGATACTGCGATTTTAAGTGTTGTTCCATTCCA
TCACGGTTTTGGAATGTTTACTACACTCGGATATTTGATATGTGGATTTCGAGT
CGTCTTAATGTATAGATTTGAAGAAGAGCTGTTTCTGAGGAGCCTTCAGGATT
ACAAGATTCAAAGTGCGCTGCTGGTGCCAACCCTATTCTCCTTCTTCGCCAA
AAGCACTCTGATTGACAAATACGATTTATCTAATTTACACGAAATTGCTTCTG
GTGGCGCTCCCCTCTCTAAGGAAGTCGGGGAAGCGGTTGCCAAGAGGTTCC
ATCTGCCAGGTATCAGGCAAGGATATGGGCTCACTGAGACTACATCAGCTATT
CTGATTACACCCGAGGGGGATGATAAACCGGGCGCGGTCGGTAAAGTTGTTC
CATTTTTTGAAGCGAAGGTTGTGGATCTGGATACCGGGAAAACGCTGGGCGT
 
TAATCAAAGAGGCGAACTGTGTGTGAGAGGTCCTATGATTATGTCCGGTTATG
TAAACAATCCGGAAGCGACCAACGCCTTGATTGACAAGGATGGATGGCTACA
TTCTGGAGACATAGCTTACTGGGACGAAGACGAACACTTCTTCATCGTTGAC
CGCCTGAAGTCTCTGATTAAGTACAAAGGCTATCAGGTGGCTCCCGCTGAATT
GGAATCCATCTTGCTCCAACACCCCAACATCTTCGACGCAGGTGTCGCAGGT
CTTCCCGACGATGACGCCGGTGAACTTCCCGCCGCCGTTGTTGTTTTGGAGC
ACGGAAAGACGATGACGGAAAAAGAGATCGTGGATTACGTCGCCAGTCAAG
TAACAACCGCGAAAAAGTTGCGCGGAGGAGTTGTGTTTGTGGACGAAGTAC
CGAAAGGTCTTACCGGAAAACTCGACGCAAGAAAAATCAGAGAGATCCTCA
TAAAGGCCAAGAAGGGCGGAAAGATCGCCGTGGATTACAAGGATGACGACG
ATAAGTAAGGGCCCACCGGTTGATAATAGGCTGGAGCCTCGGTGGCCATGCT
TCTTGCCCCTTGGGCCTCCCCCCAGCCCCTCCTCCCCTTCCTGCACCCGTACC
CCCGTGGTCTTTGAATAAAGTCTGAGTGGGCGGCCTCGAGCCGCGGAAAAA
AAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAA
AAAAAAAAAAAAAAAAAAAATTATTGTTTAAACATTTAAATTCTAGA
SEQ ID NO: 57
> B1 mRNA sequence (ACTG1 3'UTR) 
CCGACUACCUCAUGAAGAUCCUCACUGAGCGAGGCUACAGCUUCACCACC
ACGGCCGAGCGGGAAAUCGUGCGCGACAUCAAGGAGAAGCUGUGCUACG
UCGCCCUGGACUUCGAGCAGGAGAUGGCCACCGCCGCAUCCUCCUCUUCU
CUGGAGAAGAGCUACGAGCUGCCCGAUGGCCAGGUCAUCACCAUUGGCAA
UGAGCGGUUCCGGUGUCCGGAGGCGCUGUUCCAGCCUUCCUUCCUGGGUA
UGGAAUCUUGCGGCAUCCACGAGACCACCUUCAACUCCAUCAUGAAGUGU
GACGUGGACAUCCGCAAAGACCUGUACGCCAACACGGUGCUGUCGGGCGG
CACCACCAUGUACCCGGGCAUUGCCGACAGGAUGCAGAAGGAGAUCACCG
CCCUGGCGCCCAGCACCAUGAAGAUCAAGAUCAUCGCACCCCCAGAGCGC
AAGUACUCGGUGUGGAUCGGUGGCUCCAUCCUGGCCUCACUGUCCACCUU
CCAGCAGAUGUGGAUUAG
SEQ ID NO: 58
> B2 mRNA sequence (ATP6V0B 3'UTR) 
AUGAUAUGUGUGGGUGGGGCCGUGCCUCACUUUUAUUUAUUGCUGGUUU
UCCUGGGACAGCUGGAGCUGUGUCCCUUAGCCUUUCAGAGGCUUGGUGU
UCAGGGCCCUCCCUGCACUCCCCUCUUGCUGCGUGUUGAUUUGGAGGCAC
UGCAGUCCAGGCCGAGUCCUCAGUGCGGGGAGCAGGCUGCUGCUGCUGAC
UCUGUGCAGCUGCGCACCUGUGUCCCCCACCUCCACCCUCAACCCAUCUU
CCUAGUGUUUGUGAAAUAAACUUGGUAUUUGUCU
SEQ ID NO: 59
> B3 mRNA sequence (ATP6V0B 3'UTR) 
GUUGAAGAACUUCUCUCUACCGAGCAGCCCGUAACCCCCUUUUUCUCCCU
CACUGCUGCAGGAGUCUGCUACACCAUUUUUGAUUUGGGCUUCCGCUUUG
AUGUGGCAUGGUUCCUGACGGAGACUUCGCCCUUCAUGUGGUCCAACCUG
GGCAUUGGCCUAGCUAUCUCCCUGUCUGUGGUUGGGGCAGCCUGGGGCAU
CUAUAUUACCGGCUCCUCCAUCAUUGGUGGAGGAGUGAAGGCCCCCAGGA
UCAAGACCAAGAACCUGGUCAGCAUCAUCUUCUGUGAGGCUGUGGCCAUC
UACGGCAU
SEQ ID NO: 60
> B4 mRNA sequence (ATP6V0E1 3'UTR) 
GGAAGAAGACAUGCUCUACAGUGCUCAGUCUUUGAGGUGACUAUGCUUG
UGACCUUUCUUAUCAGUAUGGUCAGGCAGUCAGAGUUUGCCUUGACUUU
UUCUCACAUUUUAUGGCCAUUUUAACACGUGGCAAGGGCAAGCUGCUGA
AUGCAAACACGAUCUGAAGAGCAGAAAUAAAAUUGGAACUUAAGCCUUA
GCUUACCUAAAUCUCAUUAAGAUUCUUUUU
SEQ ID NO: 61
> B5 mRNA sequence (ATP6V0E1 3'UTR) 
AUGCAAAAUCACCUCCAAACCAGACCACUUUUCUUGACUUGCCUGUUUUG
GCCAUUAGCUGCCUUAAACGUUAACAGCACAUUUGAAUGCCUUAUUCUAC
AAUGCAGCGUGUUUUCCUUUGCCUUUUUUGCACUUUGGUGAAUUACGUG
CCUCCAUAACCUGAACUGUGCCGACUCCACAAAACGAUUAUGUACUCUUC
UGAGAUAGAAGAUGCUGUUCUUCUGAGAGAUACGUUACUCUCUCCUUGG
AAUCUGUGGAUUUGAAGAUGGCUCCUGCCUUCUCACGUGGGAAUCAGUG
AAGUGUUUAGAAACUGCUGCAAGACAAACAAGACUCCAGUGGGGUGGUC
AGUAGGAGAGCACGUUCAGAGGGAAGAGCCAUCUCAACAGAAUCGCACC
AAACUAUACUUUCAGGAUGAAUUUCUUCUUUCUGCCAUCUUUUGGAAUA
AAUAUUUUCCUCCUUUC
SEQ ID NO: 62
> B6 mRNA sequence (CFL1 3'UTR) 
GCCCCUUCUGGCCCCCUGCCUGGAGCAUCUGGCAGCCCCACACCUGCCCU
UGGGGGUUGCAGGCUGCCCCCUUCCUGCCAGACCGGAGGGGCUGGGGGGA
UCCCAGCAGGGGGAGGGCAAUCCCUUCACCCCAGUUGCCAAACAGACCCC
CCACCCCCUGGAUUUUCCUUCUCCCUCCAUCCCUUGACGGUUCUGGCCUU
CCCAAACUGCUUUUGAUCUUUUGAUUCCUCUUGGGCUGAAGCAGACCAAG
UUCCCCCCAGGCACCCCAGUUGUGGGGGAGCCUGUAUUUUUUUUAACAAC
AUCCCCAUUCCCCACCUGGUCCUCCCCCUUCCCAUGCUGCCAACUUCUAAC
CGCAAUAGUGACUCUGUGCUUGUCUGUUUAGUUCUGUGUAUAAAUGGAA
UGUUGUGGAGAUGACCCCUCCCUGUGCCGGCUGGUUCCUCUCCCUUUUCC
CCUGGUCACGGCUACUCAUGGAAGCAGGACCAGUAAGGGACCUUCGAUU
SEQ ID NO: 63
> B7 mRNA sequence (CFL1 3'UTR) 
GCCCCUUCUGGCCCCCUGCCUGGAGCAUCUGGCAGCCCCACACCUGCCCU
UGGGGGUUGCAGGCUGCCCCCUUCCUGCCAGACCGGAGGGGCUGGGGGGA
UCCCAGCAGGGGGAGGGCAAUCCCUUCACCCCAGUUGCCAAACAGACCCC
CCACCCCCUGGAUUUUCCUUCUCCCUCCAUCCCUUGACGGUUCUGGCCUU
CCCAAACUGCUUUUGAUCUUUUGAUUCCUCUUGGGCUGAAGCAGACCAAG
UUCCCCCCAGGCACCCCAGUUGUGGGGGAGCC
SEQ ID NO: 64
> B8 mRNA sequence (CFL1 3'UTR) 
GCCCCUUCUGGCCCCCUGCCUGGAGCAUCUGGCAGCCCCACACCUGCCCU
UGGGGGUUGCAGGCUGCCCCCUUCCUGCCAGACCGGAGGGGCUGGGGGGA
UCCCAGCAGGGGGAGGGCAAUCCCUUCACCCCAGUUGCCAAACAGACCCC
CCACCCCCUGGAUUUUCCUUCUCCCUCCAUCCCUUGACGGUUCUGGCCUU
CCCAAACUGCUUUUGAUCUUUUGAUUCCUCUUGGGCUGAAGCAGACCAAG
UUCCCCCCAGGCACCCCAGUUGUGGGGGAGCCUGUAUUUUUUUUAACAAC
AUCCCCAUUCCCCACCUGGUCCUCCCCCUUCCCAUGCUGCCAACUUCUAAC
CGCAAUAGUG
SEQ ID NO: 65
> B9 mRNA sequence (COX4I1 3'UTR) 
GAGAUGCUGGCCUGCGCCUGCACCUGCGCCUGGCUCUGUCACCGCCAUGC
AACUCCAUGCCUAUUUACUGGAAACCUGUUAUGCCAAACAGUUGUACCAC
UGCUAAUAAAUGACCAGUUUACCUGAAA
SEQ ID NO: 66
> B10 mRNA sequence (COX4I1 3'UTR) 
AGGGACCCACAGGCGCGCCCAGCAGCUCUCGGAAGCGUUGUAUCGCAUUA
AGUUCAAGGAGAGCUUUGCUGAGAUGAACAGGGGCUCGAACGAGUGGAA
GACGGUUGUGGGCGGUGCCAUGUUCUUCAUCGGUUUCACCGCGCUCGUUA
UCAUGUGGCAGAAGCACUAUGGGCUUAGCCUCCAAGUGGGACUACGAAA
AGAACGAGUGGAAGAAGUGAGAGAUGCUGGCCUGCGCCUGCACCUGCGCC
UGGCUCUGUCACCGCCAUGCAACUCCAUGCCUAUUUACUGGAAACCUGUU
AUGCCAAACAGUUGUACCACU
SEQ ID NO: 67
> B11 mRNA sequence (COX4I1 3'UTR) 
AGAGGACCUUCUGCUUUUAGCUUAUUUUGUUGCUAACUUUUUACAAACA
AAGGGCUAAUUUAAAUGUCAGUGUUGUCAGUGGUCAGAAACCGUGUG
UUUGAGCGGGUGUUGAGUGGCAGGUGGCUCUGCUGACCUGGUGGCUGGU
GUGUCGGGAGGAUUUAACCUGUGUGAGGGAUUGGCCUAGAAACAACCUG
UUGAGAUAGUCUUGCCCCAUAACCUGUCUCACACCGUAGUGUACGGCCCC
CUCCCGCAAAGCUUUGACAAAGAGUGGGUGGCCAAGCAGACCAAGAGGA
UGCUGGAC
SEQ ID NO: 68
> B12 mRNA sequence (CTSB 3'UTR) 
UCUGCCGUGGGCCUGUCGUGCCAGUCCUGGGGGCGAGAUCGGGAGAAUCA
CGUCCUGGAACUGCAUGUUCUUGCGACUCUUGGGACUUCAUCUUAACUUC
UCGCUGCCCCAGCCAUGUUUUCAACCAUGGCAUCCCUCCCCCAAUUAGUU
CCCUGUCAUCCUCGUCAACCUUCUCUGUAAGUGCCUGGUAAGCUUGCCCU
UGCUUAAGAACUCAAAACAUAGCUGUGCUCUA
SEQ ID NO: 69
> B13 mRNA sequence (FAM166A 3'UTR) 
AAAUGCUUUAAUGGUGGUGUCUGUACAGCAUGGAUGUGGACAGGGAAAA
CAGCAAGUGCACACAGUGGGACAGGCCACAGGACAGGCUGGGAGUGAAU
AAAGAGUUCACACUGCU
SEQ ID NO: 70
> B14 mRNA sequence (NDUFB9 3'UTR) 
UAUCUAAGUGUAUCAAUUUAACAUUUAUAAUCACCUUACAAGUAAGUUA
AAGUGCAUUCCCUGGUCGUUUUGGCUUUGUCUUUGAUUUUGCUUCAGAU
CUCCUUCUCUUUAGUCCUUUGUCUGACUUUUUUGCCUUCUUGCUAUACUU
UCCCUCAACCUGCCUCUCCACUAUUUUCUUAGGCAUUUUUCAGUCAUG
SEQ ID NO: 71
> A1 mRNA sequence (ACTG1 5'UTR) 
CUCUCGCACUCUGUUCUUCCGCCGCUCCGCCGUCGCGUUUCUCUGCCGGU
CGCA
SEQ ID NO: 72
> A2 mRNA sequence (ATP6V0B 5'UTR) 
GGGCGGGCGGACAGACUGCGGGACGGACGGUGGACGCUGGGACGCGUUU
GUAGCUCCGGCCCCGCCGUUCCGACCCCCGCCGCCGUCGCCGCCAUGACGG
GGCUAGCACUGCUCUACUCCGGGGUCUUCGUGGCCUUCUGGGCCUGCGCG
CUGGCCGUGGGUUCCUGACGGAGACUUCGCCCUUC
SEQ ID NO: 73
> A3 mRNA sequence (ATP6V0B 5'UTR) 
AGACUGCGGGACGGACGGUGGACGCUGGGACGCGUUUGUAGCUCCGGCCC
CGCCGUUCCGACCCCCGCCGCCGUCGCCGCC
SEQ ID NO: 74
> A4 mRNA sequence (ATP6V0E1 5'UTR) 
GGGGUAGGGGUUGGCGCUCAGGCGGCGACC
SEQ ID NO: 75
> A5 mRNA sequence (ATP6V0E1 5'UTR) 
GUCACGCGGUCAGCUAUUGACACUUCCUGGUGGGAUCCGAGUGAGGCGAC
GGGGUAGGGGUUGGCGCUCAGGCGGCGACC
SEQ ID NO: 76
> A6 mRNA sequence (CFL1 5'UTR) 
GGCCGGCGGGAAGACUCCGUUACCCAGCGAGCGAGGCGGCGCAGGGC
CAGCGGACUCCAUUUCCCGUCGGCUCGCGGUGGGAGCGCCGGAAGCCCGC
CCCACCCCUCAUUGUGCGGCUCCUACUAAACGGAAGGGGCCGGGAGAGGC
CGCGUUCAGUCGGGUCCCGGCAGCGGCUGCAGCGCUCUCGUCUUCUGCGG
CUCUCGGUGCCCUCUCCUUUUCGUUUCCGGAAAC
SEQ ID NO: 77
> A7 mRNA sequence (CFL1 5'UTR) 
CUCCUGGCUCGGGCACCCCGAAGCACGACGCAGAGUAGGAAGAGUUUAAA
GACCUCGAGGCCUCUGGGCACUUGAGUUUGGCCUCCGGUGUGGCUGUCUC
UGAUGGUGUCAUCAAGGUGUUCAACGAC
SEQ ID NO: 78
> A8 mRNA sequence (CFL1 5'UTR) 
ACGUGGCCGGCUUCUUCUGCAGUUCCGGGGAGUUGGGGACCAGAUUU
ACCUUGGAUUGCCCCUCCCUCUCCUGGCUCGGGCACCCCGAAGCACGACG
CAGAGCCUCCGGUGUGGCUGUCUCUGAUGGUGUCAUCAAGGUGUUCAAC
GAC
SEQ ID NO: 79
> A9 mRNA sequence (COX4I1 5'UTR) 
GCGGCCUUGCUCUCUUCCGGUCGCGGGACACCGGGUGUAGAGGGCGGUCG
CGGCGGGCAGUGGCGGCAGA
SEQ ID NO: 80
> A10 mRNA sequence (COX4I1 5'UTR) 
GGGACACCGGGUGUAGAGGGCGGUCGCGGCGGGCAGUGGCGGCAGA
SEQ ID NO: 81
> A11 mRNA sequence (COX4I1 5'UTR) 
CGCGGCCUUGCUCUCUUCCGGUCGCGGGACACCGGGUGUAGAGGGCGGUC
GCGGCGGGCAGUGGCGGCAGA
SEQ ID NO: 82
> A12 mRNA sequence (CUSB 5'UTR) 
GGCCGGGGCUGGCCCAGGCUACGGCGGCUGCAGGGCUCCGGCAACCGCUC
CGGCAACGCCAACCGCUCCGCUGCGCGCAGGCUGGGCUGCAGGCUCUCGG
CUGCAGCGCUGGGUGGAUCUAGGAUCCGGCUUCCAAC
SEQ ID NO: 83
> A13 mRNA sequence (FAM166A 5'UTR) 
AAGGGAGCUGGAUGCCGGGAGGGACUGGAGCCAGCAAGGCCAGAGUGAA
AGCAAA
SEQ ID NO: 84
> A14 mRNA sequence (NDUFB9 5'UTR) 
GCAGCAGGCGUGCAGUUUCCCGGCUCUCCGCGGCCGGGGAAGGUCAGC
GCCGUA
 SEQ ID NO: 85
> A15 mRNA sequence (CHCHD10 5'UTR) 
GGCAUUUGUCCCCGCGACAGCACCGCUGCCGCCGUCUCUAAGGUCGCCCG
GGUCCCACCGCCGCCACC
SEQ ID NO: 86
> A16 mRNA sequence (CHCHD10 5'UTR) 
CCGCUGCCGCCGUCUCUAAGGUCGCCCGGGUCCCACCGCCGCCACC
SEQ ID NO: 87
> A17 mRNA sequence (SLC38A2 5'UTR) 
AUGUCUUUUUUGUGUGUUUGUUUUCAUGGUAUUCCUAUGAA
SEQ ID NO: 88
> A18 mRNA sequence (NDUFA11 5'UTR) 
GCUUCCCGAGCUGGCGGGUCCGUGGUGCGGAUCGAGAUUGCGGCU
SEQ ID NO: 89
> A19 mRNA sequence (NDUFV3 5'UTR) 
GGCGGCUGUUCAGGCGCGGGUGCGCGCGCAGCUGCUGUGGCCCUGCUUGG
UGCGCCCGCUGUCACCGCC
SEQ ID NO: 90
> A20 mRNA sequence (PRDX5 5'UTR) 
CGCGCCUGCGCAGUGGAGGCGGCCCAGGCCCGCCUUCCGCAGGGUGUCGC
CGCUGUGCCGCUAGCGGUGCCCCGCCUGCUGCGGUGGCACCAGCCAGGAG
GCGGAGUGGAAGUGGCCGUGGGGCGGGU
SEQ ID NO: 91
> A21 mRNA sequence (GUK1 5'UTR) 
CAGCAGAUGGGGACUAGAGGCCGCACUGCUAUCCACAGCCUCUCUUCUCA
CCCCCAGGC
SEQ ID NO: 92
> A22 mRNA sequence (GUK1 5'UTR) 
GCUGGCCGGGCUGGCUGCGGCCGCCCUGGGCCGGGCCCCACCGGACGCCU
CUCUUCUCACCCCCAGGC
SEQ ID NO: 93
> A23 mRNA sequence (GUK1 5'UTR) 
AGAGGUGGCCCCGGAUGCUGCGGCGCCCGCUGGCCGGGCUGGCUGCGGCC
GCCCUGGGCCGGGCCCCACCGGACGCCUCUCUUCUCACCCCCAGGC
SEQ ID NO: 94
> A24 mRNA sequence (IAH1 5'UTR) 
UGGCUGGCGGCCCCGCCCCGCCCCGCCCGGCUGCUCC
SEQ ID NO: 95
> A25 mRNA sequence (ABHD16A 5'UTR) GGAGGGCGGGGCCGGCAGGGGGACCUGCUGCUGGAAGAGCAGCGGCCCGA 
GCCGGGGCC
SEQ ID NO: 96
> A26 mRNA sequence (SLC25A39 5'UTR) 
UUUCGGCGUCGGCCUAGGUGCGCUGCGAGCGCGCGGACCGCACAGG
CGGCGGAGCCGGUAUGGGCCCGCCUGACCCUGGGCGCCGCGCCGCACGAG
CACCAGCCUAGAGCCAGGACUGAAGCUUCAAG
SEQ ID NO: 97
> A27 mRNA sequence (AUPIF1 5'UTR) 
CGAGAGACUGCUUGCUGCGGCAGAGACGCCAGAGGUGCAGCUCCAGCAGC
A
SEQ ID NO: 98
> A28 mRNA sequence (ANAPC11 5'UTR) 
CGGAGUUUCGUCAUGUUGGCCAGGCCCAUUUGAGAUCUUUGAAGAUAUC
CUCAACGUGAGGCUCUGCUGCC
SEQ ID NO: 99
> A29 mRNA sequence (ANAPC11 5'UTR) 
GGGCGCGGCUUCGGCGGGCGGCAGCCGCUGCAGACGAGCUGCGGGCUCUG
CUGCC
SEQ ID NO: 100
> A30 mRNA sequence (CCDC12 5'UTR) 
GCCUGCGCGAUGCAAGACGGAGAAAGGAGGGGCGUACGCGGGCAAG
SEQ ID NO: 101
> A31 mRNA sequence (MRPL14 5'UTR) 
AUCGCGUCCGCCGGGCUGGGCCUGGCGCGCAGGCGCUAGGAAGAGGCCGC
GUGGGGCGAAGGCGGCGCUUGGCUGGUGGGGCCCGCGGCGGGAUUUUCCC
GGGCGGCGAGAGCGGAUCUAUCUUGGGAUCCC
SEQ ID NO: 102
> A32 mRNA sequence (APOA1BP 5'UTR) 
GCCGGGGGCGCGCUCUGCGAGCUGG
SEQ ID NO: 103
> A33 mRNA sequence (APOA1BP 5'UTR) 
GCCGGGGCCGGGCCGGGCCGGGGCGCGCUCUGCGAGCUGG
SEQ ID NO: 104
> Mod. 5'UTR mRNA sequence 
GGGAAAUAAGAGAGAAAAGAAGAGUAAGAAGAAAUAUAAGAGCCACC
SEQ ID NO: 105
> α-globin 3'UTR mRNA sequence 
UGAUAAUAGGCUGGAGCCUCGGUGGCCAUGCUUCUUGCCCCUUGGGCC
UCCCCCCAGCCCCUCCUCCCCUUCCUGCACCCGUACCCCCGUGGUCUU
UGAAUAAAGUCUGAGUGGGCGGC
 SEQ ID NO: 106
> α-1 globin 5'UTR mRNA sequence 
AGACGAACUAGUAUUCUUCUGGUCCCCACAGACUCAGAGAGAACCCGCCA
CC
SEQ ID NO: 107
> haplogroup U8b1b1 mitochondrion 3'UTR mRNA sequence 
CUCGAGCUGGUACUGCAUGCACGCAAUGCUAGCUGCCCCUUUCCCGUCCU
GGGUACCCCGAGUCUCCCCCGACCUCGGGUCCCAGGUAUGCUCCCACCUC
CACCUGCCCCACUCACCACCUCUGCUAGUUCCAGACACCUGCUAUACUAA
UCGCUAGCCGCGUCGCUCCCCAGGGUUGGUCAAUUUCGUGCCAGCCACAC
CGAGACCUGGUCCAGAGCCCAAGCACGCCACACCCCCACGGGAAACAGCA
GUGAUUAACCUUUAGCAAUAAACGAAAGUUUAACUAACAGCAAUGCA
GCUCAAAACGCUUAGCCUAG
SEQ ID NO: 108
> Amino acid sequence of hHGF 
MWVTKLLPALLLQHVLLHLLLLPIAIPYAEGQRKRRNTIHEFKKSAKTTLIKIDPA
LKIKTKKVNTADQCANRCTRNKGLPFTCKAFVFDKARKQCLWFPFNSMSSGVK
KEFGHEFDLYENKDYIRNCIIGKGRSYKGTVSITKSGIKCQPWSSMIPHEHSFLPS
SYRGKDLQENYCRNPRGEEGGPWCFTSNPEVRYEVCDIPQCSEVECMTCNGES
YRGLMDHTESGKICQRWDHQTPHRHKFLPERYPDKGFDDNYCRNPDGQPRPW
CYTLDPHTRWEYCAIKTCADNTMNDTDVPLETTECIQGQGEGYRGTVNTIWNG
IPCQRWDSQYPHEHDMTPENFKCKDLRENYCRNPDGSESPWCFTTDPNIRVGY
CSQIPNCDMSHGQDCYRGNGKNYMGNLSQTRSGLTCSMWDKNMEDLHRHIF
WEPDASKLNENYCRNPDDDAHGPWCYTGNPLIPWDYCPISRCEGDTTPTIVNL
DHPVISCAKTKQLRVVNGIPTRTNIGWMVSLRYRNKHICGGSLIKESWVLTARQ
CFPSRDLKDYEAWLGIHDVHGRGDEKCKQVLNVSQLVYGPEGSDLVLMKLARP
AVLDDFVSTIDLPNYGCTIPEKTSCSVYGWGYTGLINYDGLLRVAHLYIMGNEK
CSQHHRGKVTLNESEICAGAEKIGSGPCEGDYGGPLVCEQHKMRMVLGVIVPG
RGCAIPNRPGIFVRVAYYAKWIHKIILTYKVPQS
SEQ ID NO: 109
> Nucleotide sequence of hHGF 
ATGTGGGTGACCAAACTCCTGCCAGCCCTGCTGCTGCAGCATGTCCTCCTGC
ATCTCCTCCTGCTCCCCATCGCCATCCCCTATGCAGAGGGACAAAGGAAAAG
AAGAAATACAATTCATGAATTCAAAAAATCAGCAAAGACTACCCTAATCAAA
ATAGATCCAGCACTGAAGATAAAAACCAAAAAAGTGAATACTGCAGACCAAT
GTGCTAATAGATGTACTAGGAATAAAGGACTTCCATTCACTTGCAAGGCTTTT
GTTTTTGATAAAGCAAGAAAACAATGCCTCTGGTTCCCCTTCAATAGCATGTC
AAGTGGAGTGAAAAAAGAATTTGGCCATGAATTTGACCTCTATGAAAACAAA
GACTACATTAGAAACTGCATCATTGGTAAAGGACGCAGCTACAAGGGAACAG
TATCTATCACTAAGAGTGGCATCAAATGTCAGCCCTGGAGTTCCATGATACCA
 
CACGAACACAGCTTTTTGCCTTCGAGCTATCGGGGTAAAGACCTACAGGAAA
ACTACTGTCGAAATCCTCGAGGGGAAGAAGGGGGACCCTGGTGTTTCACAA
GCAATCCAGAGGTACGCTACGAAGTCTGTGACATTCCTCAGTGTTCAGAAGT
TGAATGCATGACCTGCAATGGGGAGAGTTATCGAGGTCTCATGGATCATACAG
AATCAGGCAAGATTTGTCAGCGCTGGGATCATCAGACACCACACCGGCACAA
ATTCTTGCCTGAAAGATATCCCGACAAGGGCTTTGATGATAATTATTGCCGCA
ATCCCGATGGCCAGCCGAGGCCATGGTGCTATACTCTTGACCCTCACACCCGC
TGGGAGTACTGTGCAATTAAAACATGCGCTGACAATACTATGAATGACACTGA
TGTTCCTTTGGAAACAACTGAATGCATCCAAGGTCAAGGAGAAGGCTACAG
GGGCACTGTCAATACCATTTGGAATGGAATTCCATGTCAGCGTTGGGATTCTC
AGTATCCTCACGAGCATGACATGACTCCTGAAAATTTCAAGTGCAAGGACCT
ACGAGAAAATTACTGCCGAAATCCAGATGGGTCTGAATCACCCTGGTGTTTT
ACCACTGATCCAAACATCCGAGTTGGCTACTGCTCCCAAATTCCAAACTGTG
ATATGTCACATGGACAAGATTGTTATCGTGGGAATGGCAAAAATTATATGGGC
AACTTATCCCAAACAAGATCTGGACTAACATGTTCAATGTGGGACAAGAACA
TGGAAGACTTACATCGTCATATCTTCTGGGAACCAGATGCAAGTAAGCTGAAT
GAGAATTACTGCCGAAATCCAGATGATGATGCTCATGGACCCTGGTGCTACAC
GGGAAATCCACTCATTCCTTGGGATTATTGCCCTATTTCTCGTTGTGAAGGTG
ATACCACACCTACAATAGTCAATTTAGACCATCCCGTAATATCTTGTGCCAAA
ACGAAACAATTGCGAGTTGTAAATGGGATTCCAACACGAACAAACATAGGAT
GGATGGTTAGTTTGAGATACAGAAATAAACATATCTGCGGAGGATCATTGATA
AAGGAGAGTTGGGTTCTTACTGCACGACAGTGTTTCCCTTCTCGAGACTTGA
AAGATTATGAAGCTTGGCTTGGAATTCATGATGTCCACGGAAGAGGAGATGA
GAAATGCAAACAGGTTCTCAATGTTTCCCAGCTGGTATATGGCCCTGAAGGAT
CAGATCTGGTTTTAATGAAGCTTGCCAGGCCTGCTGTCCTGGATGATTTTGTT
AGTACGATTGATTTACCTAATTATGGATGCACAATTCCTGAAAAGACCAGTTG
CAGTGTTTATGGCTGGGGCTACACTGGATTGATCAACTATGATGGCCTATTAC
GAGTGGCACATCTCTATATAATGGGAAATGAGAAATGCAGCCAGCATCATCGA
GGGAAGGTGACTCTGAATGAGTCTGAAATATGTGCTGGGGCTGAAAAGATTG
GATCAGGACCATGTGAGGGGGATTATGGTGGCCCACTTGTTTGTGAGCAACA
TAAAATGAGAATGGTTCTTGGTGTCATTGTTCCTGGTCGTGGATGTGCCATTC
CAAATCGTCCTGGTATTTTTGTCCGAGTAGCATATTATGCAAAATGGATACACA
AAATTATTTTAACATATAAGGTACCACAGTCATAG
SEQ ID NO: 110
> hHGF codon-optimized sequence 1 (hHGF-OS1) 
ATGTGGGTGACCAAACTGCTCCCCGCTCTGCTGCTGCAGCATGTGCTGCTCC
ACCTGCTGCTGCTGCCAATCGCCATCCCCTACGCTGAAGGACAGCGGAAGCG
CAGAAACACAATTCACGAATTCAAGAAGTCCGCCAAAACAACACTGATCAA
GATCGACCCTGCCCTGAAAATCAAAACCAAGAAGGTGAACACTGCCGATCA
GTGCGCCAACAGATGCACAAGAAACAAAGGCCTGCCCTTCACATGCAAGGC
 
CTTTGTGTTCGACAAGGCCAGAAAGCAGTGCCTGTGGTTCCCTTTCAACAGC
ATGAGCTCTGGCGTGAAGAAAGAGTTCGGCCACGAATTCGACCTGTACGAG
AACAAGGATTACATCAGAAATTGTATCATCGGCAAGGGCAGAAGCTACAAAG
GCACAGTGTCCATCACCAAAAGCGGCATTAAGTGTCAGCCTTGGAGCAGCAT
GATCCCTCACGAGCACAGCTTCCTGCCTAGCTCTTACAGAGGCAAGGACCTG
CAGGAGAATTACTGCCGGAACCCTAGAGGCGAGGAAGGCGGCCCTTGGTGC
TTTACCAGCAACCCTGAGGTGCGGTACGAGGTGTGTGACATCCCCCAGTGCA
GCGAAGTGGAATGCATGACCTGTAATGGCGAGAGCTACAGAGGCCTCATGGA
CCATACAGAGAGCGGAAAGATTTGTCAAAGATGGGATCACCAGACACCTCAC
AGACACAAATTCCTGCCCGAAAGATATCCTGACAAGGGCTTCGACGACAACT
ACTGCAGAAATCCTGATGGCCAACCCAGACCTTGGTGCTACACCCTGGATCC
TCATACCAGATGGGAGTACTGTGCCATCAAGACCTGCGCTGATAATACCATGA
ACGACACCGATGTGCCTCTGGAAACAACCGAGTGCATCCAGGGACAGGGCG
AAGGCTACCGGGGCACCGTGAACACCATCTGGAACGGCATCCCCTGCCAGA
GATGGGACAGCCAGTACCCCCACGAACACGATATGACCCCTGAGAACTTCAA
GTGCAAGGACCTGAGAGAAAATTACTGCAGAAACCCAGACGGAAGCGAGTC
TCCTTGGTGTTTTACCACCGACCCCAACATCCGGGTGGGTTACTGCAGCCAA
ATCCCCAATTGCGACATGAGCCACGGCCAGGACTGCTACCGGGGAAACGGC
AAAAACTACATGGGCAATCTGAGCCAGACCCGGAGCGGTCTGACCTGCTCCA
TGTGGGATAAGAACATGGAAGATCTGCACCGGCACATCTTCTGGGAGCCTGA
CGCTTCTAAGCTGAACGAGAACTACTGTAGGAACCCAGACGACGATGCCCAC
GGTCCCTGGTGCTACACCGGCAACCCGCTGATCCCTTGGGACTATTGTCCTAT
CAGCCGCTGCGAGGGCGATACAACACCTACCATCGTGAACCTGGACCACCCT
GTGATCTCTTGCGCCAAGACCAAGCAGCTGAGAGTGGTGAACGGCATCCCA
ACACGGACCAACATCGGATGGATGGTCTCCCTGAGATACCGGAACAAGCACA
TCTGCGGCGGCTCTCTGATCAAGGAATCTTGGGTCCTGACCGCCAGACAGTG
CTTCCCCAGCCGGGACCTGAAGGACTATGAGGCCTGGCTGGGAATCCACGAC
GTGCACGGCAGAGGCGACGAGAAGTGCAAGCAAGTGCTGAACGTGTCCCA
GCTGGTGTACGGCCCTGAGGGATCTGATCTGGTCCTGATGAAACTGGCCAGA
CCCGCCGTGCTGGACGACTTTGTGTCCACCATCGACCTGCCTAACTACGGCT
GCACCATCCCTGAAAAGACCAGCTGCAGCGTGTACGGCTGGGGCTATACAGG
CCTGATCAACTACGACGGTCTGCTGCGGGTTGCTCACCTGTACATCATGGGC
AACGAGAAGTGCAGCCAGCACCACAGAGGAAAGGTTACACTTAACGAGAG
CGAGATCTGCGCCGGCGCCGAGAAAATCGGCTCCGGCCCTTGTGAAGGCGA
CTACGGAGGCCCTCTGGTGTGCGAACAGCACAAGATGAGAATGGTGCTGGG
CGTGATCGTGCCCGGAAGAGGCTGTGCAATCCCTAATAGGCCCGGCATTTTC
GTGCGGGTGGCCTACTACGCCAAGTGGATCCACAAGATCATCCTGACGTACA
AGGTGCCTCAGAGCTGA
SEQ ID NO: 111
> hHGF codon-optimized sequence 2 (hHGF-OS2) 
ATGTGGGTCACCAAACTGCTGCCCGCCCTGCTGCTGCAGCACGTGCTCCTGC
ACCTGCTGCTGCTGCCTATCGCCATCCCTTACGCCGAAGGCCAGAGAAAGCG
GAGAAATACCATTCACGAATTTAAGAAGAGCGCCAAGACCACACTGATCAAG
ATCGACCCTGCCCTGAAAATCAAGACCAAGAAAGTGAACACAGCCGACCAA
TGCGCTAATAGATGCACAAGAAACAAAGGCCTGCCTTTCACCTGCAAGGCCT
TCGTGTTCGATAAGGCCAGAAAGCAGTGCCTGTGGTTCCCCTTCAACAGCAT
GAGCAGCGGCGTGAAGAAAGAATTCGGTCACGAGTTCGACCTGTACGAGAA
TAAGGACTACATTAGAAACTGCATCATCGGCAAGGGCCGGAGCTACAAGGGC
ACCGTGAGCATCACCAAGTCTGGCATCAAGTGCCAGCCATGGAGTTCCATGA
TCCCTCATGAGCACAGCTTCCTGCCAAGCTCTTACCGGGGAAAGGATCTGCA
GGAGAACTATTGTAGAAATCCCAGAGGCGAGGAAGGAGGACCCTGGTGTTTT
ACCAGCAACCCAGAGGTGCGGTACGAGGTGTGCGACATACCCCAGTGCAGC
GAGGTGGAATGCATGACATGCAACGGCGAGAGCTACCGGGGCCTGATGGAC
CACACAGAGAGCGGCAAAATCTGCCAAAGATGGGATCACCAGACCCCTCAT
AGGCACAAGTTCCTGCCCGAGAGATACCCTGACAAGGGCTTCGACGACAATT
ACTGCCGGAACCCTGATGGACAGCCTAGACCTTGGTGCTACACCCTGGACCC
TCACACCAGATGGGAATACTGCGCCATCAAGACATGTGCCGATAACACCATG
AACGACACCGATGTGCCACTCGAGACAACTGAGTGTATCCAGGGCCAGGGC
GAGGGCTACAGGGGCACCGTGAACACCATCTGGAACGGCATCCCTTGTCAG
CGGTGGGACAGCCAATACCCCCACGAACATGATATGACCCCAGAAAACTTCA
AGTGCAAAGATCTGCGGGAAAACTACTGTAGAAACCCTGACGGCTCCGAGA
GCCCTTGGTGCTTCACCACCGACCCTAACATCAGGGTGGGCTACTGTAGCCA
GATCCCCAACTGCGACATGAGCCACGGTCAGGACTGCTACAGAGGAAACGG
CAAGAATTACATGGGCAACCTGAGCCAGACCAGAAGCGGCCTGACCTGCAG
CATGTGGGACAAAAACATGGAAGATCTGCACCGGCACATCTTTTGGGAGCCT
GACGCCAGCAAGCTGAATGAGAACTACTGTCGGAACCCTGACGACGATGCT
CACGGCCCTTGGTGCTACACAGGCAACCCTCTGATCCCTTGGGACTATTGTCC
TATTAGCAGGTGTGAAGGCGACACGACCCCTACAATCGTGAACCTGGATCAC
CCAGTGATCAGCTGTGCTAAAACAAAGCAACTGAGAGTGGTGAATGGTATCC
CGACCCGGACCAACATCGGATGGATGGTCAGCCTGCGGTACAGAAACAAGC
ACATCTGCGGCGGTAGCCTGATCAAGGAATCCTGGGTGTTGACAGCCAGACA
GTGTTTTCCCAGCAGAGATCTGAAGGACTACGAGGCCTGGCTGGGCATCCAC
GACGTGCACGGCAGAGGCGACGAGAAGTGCAAGCAGGTTCTGAACGTGTCC
CAGCTGGTGTACGGCCCCGAGGGCTCTGATCTGGTTCTGATGAAGCTTGCTA
GACCCGCTGTGCTGGATGACTTCGTGTCCACCATCGACCTGCCAAATTACGG
ATGTACAATTCCTGAAAAGACCTCCTGTTCTGTGTACGGCTGGGGCTACACC
GGACTCATCAACTATGACGGCCTGCTGAGAGTGGCCCACCTGTATATCATGGG
CAACGAGAAGTGCAGCCAGCACCATAGAGGCAAGGTGACCCTGAACGAGTC
TGAAATCTGCGCCGGAGCCGAGAAAATCGGATCTGGCCCCTGCGAAGGAGA
TTACGGCGGCCCTCTGGTCTGCGAGCAGCACAAGATGAGAATGGTGCTGGGC
 
GTGATCGTGCCCGGCAGAGGCTGCGCCATCCCTAACCGGCCTGGAATCTTCG
TGCGCGTGGCCTATTACGCCAAATGGATCCACAAAATCATCCTGACATACAAG
GTCCCACAGTCTTGA
SEQ ID NO: 112
> hHGF codon-optimized sequence 3 (hHGF-OS3) 
ATGTGGGTGACCAAGCTGCTGCCCGCCCTGCTCCTGCAGCACGTGCTGCTGC
ACCTGCTCCTGCTGCCCATCGCCATCCCCTACGCCGAGGGGCAGAGAAAGAG
AAGAAACACCATCCACGAGTTCAAGAAGAGCGCCAAGACCACCCTGATCAA
GATCGACCCCGCCCTGAAGATCAAGACCAAGAAGGTGAACACCGCCGATCA
GTGCGCCAACAGATGCACAAGAAACAAGGGCCTGCCCTTCACCTGCAAGGC
CTTCGTGTTCGACAAGGCTAGAAAGCAGTGCCTGTGGTTCCCCTTCAACAGC
ATGAGCAGCGGCGTGAAGAAGGAGTTCGGCCACGAGTTCGACCTGTACGAG
AACAAGGACTACATCAGAAACTGCATCATCGGCAAGGGCAGAAGCTACAAG
GGCACCGTGAGCATCACCAAGAGCGGCATCAAGTGTCAGCCCTGGAGCAGC
ATGATCCCCCATGAGCACAGCTTCCTGCCTAGCAGCTACAGAGGGAAGGACC
TGCAAGAGAACTACTGCAGAAACCCTAGAGGCGAGGAGGGCGGCCCTTGGT
GTTTCACAAGCAATCCCGAGGTGAGATACGAGGTGTGCGACATCCCTCAGTG
CAGCGAGGTGGAGTGCATGACCTGCAACGGCGAAAGCTATAGAGGCCTGAT
GGACCACACCGAGAGCGGCAAGATTTGTCAGCGGTGGGATCACCAAACCCC
CCACAGACACAAGTTCCTGCCCGAGAGATACCCCGACAAGGGCTTCGACGA
CAATTACTGTAGAAACCCCGACGGGCAGCCTAGACCCTGGTGTTACACCCTG
GACCCCCACACAAGATGGGAGTACTGCGCCATCAAGACCTGCGCCGACAAC
ACCATGAACGACACCGACGTGCCCCTGGAGACCACCGAGTGCATCCAAGGC
CAAGGCGAGGGCTACAGAGGCACCGTGAACACCATCTGGAACGGCATTCCC
TGTCAGAGATGGGACAGCCAATACCCCCATGAACACGATATGACCCCCGAGA
ACTTCAAGTGCAAGGACCTGAGAGAGAACTACTGCCGGAACCCCGATGGCA
GCGAATCCCCTTGGTGCTTCACCACCGACCCCAACATCAGAGTGGGCTACTG
CAGCCAAATCCCCAACTGCGACATGAGCCACGGCCAAGACTGTTACAGAGG
GAACGGCAAGAACTACATGGGCAACCTGAGCCAAACAAGAAGCGGCCTGAC
CTGCAGCATGTGGGACAAGAACATGGAGGACCTGCACAGACACATCTTCTG
GGAGCCCGACGCTAGCAAGCTGAACGAGAACTACTGTCGGAACCCCGACGA
CGATGCCCACGGCCCCTGGTGCTACACCGGCAACCCCCTGATCCCCTGGGAC
TACTGCCCCATCAGCAGATGCGAGGGCGACACCACCCCCACCATCGTGAACC
TGGACCACCCCGTGATCAGCTGTGCCAAGACCAAGCAACTGAGAGTGGTGA
ACGGCATCCCCACAAGAACCAACATTGGCTGGATGGTGAGCCTGAGATACAG
AAACAAGCACATCTGCGGCGGCAGCCTGATCAAGGAGAGCTGGGTGCTGAC
CGCTAGACAGTGCTTCCCTAGCAGAGACCTGAAGGACTACGAGGCCTGGCT
GGGCATCCACGACGTGCACGGCAGAGGCGACGAGAAGTGTAAACAAGTGCT
GAACGTGAGCCAACTGGTGTACGGCCCCGAGGGCAGCGACCTGGTGCTGAT
GAAGCTGGCTAGACCCGCCGTGCTGGACGACTTCGTGAGCACCATCGACCTG
 
CCCAACTACGGCTGCACCATCCCCGAGAAGACAAGCTGCAGCGTGTACGGC
TGGGGCTACACCGGCCTGATCAACTACGACGGCCTGCTGAGAGTGGCCCACC
TGTACATCATGGGCAACGAGAAGTGCAGCCAACACCACCGGGGCAAAGTGA
CCCTGAACGAGAGCGAGATCTGCGCCGGGGCTGAGAAAATCGGCAGCGGCC
CCTGCGAGGGGGACTACGGGGGCCCCCTGGTGTGCGAGCAGCACAAGATGA
GAATGGTGCTGGGCGTGATCGTGCCCGGCAGAGGCTGCGCCATCCCCAACA
GACCCGGCATCTTCGTGAGAGTGGCCTACTACGCCAAGTGGATCCACAAGAT
CATCCTGACCTACAAGGTGCCTCAGAGCTGA
SEQ ID NO: 113
> 5'UTR-hHGF-α globin 3'UTR-A30L70 (Mod.-hHGF-OS2) 
GGGAAATAAGAGAGAAAAGAAGAGTAAGAAGAAATATAAGAGCCACCGCTA
GCCTTAAGGCCACCATGTGGGTCACCAAACTGCTGCCCGCCCTGCTGCTGCA
GCACGTGCTCCTGCACCTGCTGCTGCTGCCTATCGCCATCCCTTACGCCGAAG
GCCAGAGAAAGCGGAGAAATACCATTCACGAATTTAAGAAGAGCGCCAAGA
CCACACTGATCAAGATCGACCCTGCCCTGAAAATCAAGACCAAGAAAGTGA
ACACAGCCGACCAATGCGCTAATAGATGCACAAGAAACAAAGGCCTGCCTTT
CACCTGCAAGGCCTTCGTGTTCGATAAGGCCAGAAAGCAGTGCCTGTGGTTC
CCCTTCAACAGCATGAGCAGCGGCGTGAAGAAAGAATTCGGTCACGAGTTC
GACCTGTACGAGAATAAGGACTACATTAGAAACTGCATCATCGGCAAGGGCC
GGAGCTACAAGGGCACCGTGAGCATCACCAAGTCTGGCATCAAGTGCCAGC
CATGGAGTTCCATGATCCCTCATGAGCACAGCTTCCTGCCAAGCTCTTACCGG
GGAAAGGATCTGCAGGAGAACTATTGTAGAAATCCCAGAGGCGAGGAAGGA
GGACCCTGGTGTTTTACCAGCAACCCAGAGGTGCGGTACGAGGTGTGCGAC
ATACCCCAGTGCAGCGAGGTGGAATGCATGACATGCAACGGCGAGAGCTACC
GGGGCCTGATGGACCACACAGAGAGCGGCAAAATCTGCCAAAGATGGGATC
ACCAGACCCCTCATAGGCACAAGTTCCTGCCCGAGAGATACCCTGACAAGGG
CTTCGACGACAATTACTGCCGGAACCCTGATGGACAGCCTAGACCTTGGTGC
TACACCCTGGACCCTCACACCAGATGGGAATACTGCGCCATCAAGACATGTG
CCGATAACACCATGAACGACACCGATGTGCCACTCGAGACAACTGAGTGTAT
CCAGGGCCAGGGCGAGGGCTACAGGGGCACCGTGAACACCATCTGGAACGG
CATCCCTTGTCAGCGGTGGGACAGCCAATACCCCCACGAACATGATATGACC
CCAGAAAACTTCAAGTGCAAAGATCTGCGGGAAAACTACTGTAGAAACCCT
GACGGCTCCGAGAGCCCTTGGTGCTTCACCACCGACCCTAACATCAGGGTGG
GCTACTGTAGCCAGATCCCCAACTGCGACATGAGCCACGGTCAGGACTGCTA
CAGAGGAAACGGCAAGAATTACATGGGCAACCTGAGCCAGACCAGAAGCGG
CCTGACCTGCAGCATGTGGGACAAAAACATGGAAGATCTGCACCGGCACATC
TTTTGGGAGCCTGACGCCAGCAAGCTGAATGAGAACTACTGTCGGAACCCTG
ACGACGATGCTCACGGCCCTTGGTGCTACACAGGCAACCCTCTGATCCCTTG
GGACTATTGTCCTATTAGCAGGTGTGAAGGCGACACGACCCCTACAATCGTG
AACCTGGATCACCCAGTGATCAGCTGTGCTAAAACAAAGCAACTGAGAGTG
 
GTGAATGGTATCCCGACCCGGACCAACATCGGATGGATGGTCAGCCTGCGGT
ACAGAAACAAGCACATCTGCGGCGGTAGCCTGATCAAGGAATCCTGGGTGTT
GACAGCCAGACAGTGTTTTCCCAGCAGAGATCTGAAGGACTACGAGGCCTG
GCTGGGCATCCACGACGTGCACGGCAGAGGCGACGAGAAGTGCAAGCAGG
TTCTGAACGTGTCCCAGCTGGTGTACGGCCCCGAGGGCTCTGATCTGGTTCT
GATGAAGCTTGCTAGACCCGCTGTGCTGGATGACTTCGTGTCCACCATCGAC
CTGCCAAATTACGGATGTACAATTCCTGAAAAGACCTCCTGTTCTGTGTACGG
CTGGGGCTACACCGGACTCATCAACTATGACGGCCTGCTGAGAGTGGCCCAC
CTGTATATCATGGGCAACGAGAAGTGCAGCCAGCACCATAGAGGCAAGGTGA
CCCTGAACGAGTCTGAAATCTGCGCCGGAGCCGAGAAAATCGGATCTGGCC
CCTGCGAAGGAGATTACGGCGGCCCTCTGGTCTGCGAGCAGCACAAGATGA
GAATGGTGCTGGGCGTGATCGTGCCCGGCAGAGGCTGCGCCATCCCTAACCG
GCCTGGAATCTTCGTGCGCGTGGCCTATTACGCCAAATGGATCCACAAAATCA
TCCTGACATACAAGGTCCCACAGTCTTGATAATAGGCTGGAGCCTCGGTGGC
CATGCTTCTTGCCCCTTGGGCCTCCCCCCAGCCCCTCCTCCCCTTCCTGCACC
CGTACCCCCGTGGTCTTTGAATAAAGTCTGAGTGGGCGGCCTCGAGCCGCGG
AAAAAAAAAAAAAAAAAAAAAAAAAAAAAAGCATATGACTAAAAAAAAAA
AAAAAAAAAAAAAAAAAAAAAAAAAAAAAAAAAAAAAAAAAAAAAAAAAAAA
AAAAAAAAAAAAGTTTAAACATTTAAATTCTAGA
SEQ ID NO: 114
> ACTG1 5'UTR-hHGF-CTSB 3'UTR-A30L70 (A1B12-hHGF-OS2) 
CTCTCGCACTCTGTTCTTCCGCCGCTCCGCCGTCGCGTTTCTCTGCCGGTCGC
AGCTAGCGCCACCATGTGGGTCACCAAACTGCTGCCCGCCCTGCTGCTGCAG
CACGTGCTCCTGCACCTGCTGCTGCTGCCTATCGCCATCCCTTACGCCGAAGG
CCAGAGAAAGCGGAGAAATACCATTCACGAATTTAAGAAGAGCGCCAAGAC
CACACTGATCAAGATCGACCCTGCCCTGAAAATCAAGACCAAGAAAGTGAA
CACAGCCGACCAATGCGCTAATAGATGCACAAGAAACAAAGGCCTGCCTTTC
ACCTGCAAGGCCTTCGTGTTCGATAAGGCCAGAAAGCAGTGCCTGTGGTTCC
CCTTCAACAGCATGAGCAGCGGCGTGAAGAAAGAATTCGGTCACGAGTTCG
ACCTGTACGAGAATAAGGACTACATTAGAAACTGCATCATCGGCAAGGGCCG
GAGCTACAAGGGCACCGTGAGCATCACCAAGTCTGGCATCAAGTGCCAGCC
ATGGAGTTCCATGATCCCTCATGAGCACAGCTTCCTGCCAAGCTCTTACCGGG
GAAAGGATCTGCAGGAGAACTATTGTAGAAATCCCAGAGGCGAGGAAGGAG
GACCCTGGTGTTTTACCAGCAACCCAGAGGTGCGGTACGAGGTGTGCGACAT
ACCCCAGTGCAGCGAGGTGGAATGCATGACATGCAACGGCGAGAGCTACCG
GGGCCTGATGGACCACACAGAGAGCGGCAAAATCTGCCAAAGATGGGATCA
CCAGACCCCTCATAGGCACAAGTTCCTGCCCGAGAGATACCCTGACAAGGGC
TTCGACGACAATTACTGCCGGAACCCTGATGGACAGCCTAGACCTTGGTGCT
ACACCCTGGACCCTCACACCAGATGGGAATACTGCGCCATCAAGACATGTGC
CGATAACACCATGAACGACACCGATGTGCCACTCGAGACAACTGAGTGTATC
 
CAGGGCCAGGGCGAGGGCTACAGGGGCACCGTGAACACCATCTGGAACGGC
ATCCCTTGTCAGCGGTGGGACAGCCAATACCCCCACGAACATGATATGACCC
CAGAAAACTTCAAGTGCAAAGATCTGCGGGAAAACTACTGTAGAAACCCTG
ACGGCTCCGAGAGCCCTTGGTGCTTCACCACCGACCCTAACATCAGGGTGGG
CTACTGTAGCCAGATCCCCAACTGCGACATGAGCCACGGTCAGGACTGCTAC
AGAGGAAACGGCAAGAATTACATGGGCAACCTGAGCCAGACCAGAAGCGGC
CTGACCTGCAGCATGTGGGACAAAAACATGGAAGATCTGCACCGGCACATCT
TTTGGGAGCCTGACGCCAGCAAGCTGAATGAGAACTACTGTCGGAACCCTG
ACGACGATGCTCACGGCCCTTGGTGCTACACAGGCAACCCTCTGATCCCTTG
GGACTATTGTCCTATTAGCAGGTGTGAAGGCGACACGACCCCTACAATCGTG
AACCTGGATCACCCAGTGATCAGCTGTGCTAAAACAAAGCAACTGAGAGTG
GTGAATGGTATCCCGACCCGGACCAACATCGGATGGATGGTCAGCCTGCGGT
ACAGAAACAAGCACATCTGCGGCGGTAGCCTGATCAAGGAATCCTGGGTGTT
GACAGCCAGACAGTGTTTTCCCAGCAGAGATCTGAAGGACTACGAGGCCTG
GCTGGGCATCCACGACGTGCACGGCAGAGGCGACGAGAAGTGCAAGCAGG
TTCTGAACGTGTCCCAGCTGGTGTACGGCCCCGAGGGCTCTGATCTGGTTCT
GATGAAGCTTGCTAGACCCGCTGTGCTGGATGACTTCGTGTCCACCATCGAC
CTGCCAAATTACGGATGTACAATTCCTGAAAAGACCTCCTGTTCTGTGTACGG
CTGGGGCTACACCGGACTCATCAACTATGACGGCCTGCTGAGAGTGGCCCAC
CTGTATATCATGGGCAACGAGAAGTGCAGCCAGCACCATAGAGGCAAGGTGA
CCCTGAACGAGTCTGAAATCTGCGCCGGAGCCGAGAAAATCGGATCTGGCC
CCTGCGAAGGAGATTACGGCGGCCCTCTGGTCTGCGAGCAGCACAAGATGA
GAATGGTGCTGGGCGTGATCGTGCCCGGCAGAGGCTGCGCCATCCCTAACCG
GCCTGGAATCTTCGTGCGCGTGGCCTATTACGCCAAATGGATCCACAAAATCA
TCCTGACATACAAGGTCCCACAGTCTTGATAAGGGCCCACCGGTTCTGCCGT
GGGCCTGTCGTGCCAGTCCTGGGGGCGAGATCGGGAGAATCACGTCCTGGA
ACTGCATGTTCTTGCGACTCTTGGGACTTCATCTTAACTTCTCGCTGCCCCAG
CCATGTTTTCAACCATGGCATCCCTCCCCCAATTAGTTCCCTGTCATCCTCGTC
AACCTTCTCTGTAAGTGCCTGGTAAGCTTGCCCTTGCTTAAGAACTCAAAAC
ATAGCTGTGCTCTACCGCGGAAAAAAAAAAAAAAAAAAAAAAAAAAAAAA
GCATATGACTAAAAAAAAAAAAAAAAAAAAAAAAAAAAAAAAAAAAAAAAA
AAAAAAAAAAAAAAAAAAAAAAAAAAAAAAAAGTTTAAACATTTAAATTCTAG
A
SEQ ID NO: 115
> CHCHD10 5'UTR-hHGF-CTSB 3'UTR-A30L70 (A15B12-hHGF-OS2) 
GGCATTTGTCCCCGCGACAGCACCGCTGCCGCCGTCTCTAAGGTCGCCCGGG
TCCCACCGCCGCCACCGCTAGCGCCACCATGTGGGTCACCAAACTGCTGCCC
GCCCTGCTGCTGCAGCACGTGCTCCTGCACCTGCTGCTGCTGCCTATCGCCAT
CCCTTACGCCGAAGGCCAGAGAAAGCGGAGAAATACCATTCACGAATTTAAG
AAGAGCGCCAAGACCACACTGATCAAGATCGACCCTGCCCTGAAAATCAAG
 
ACCAAGAAAGTGAACACAGCCGACCAATGCGCTAATAGATGCACAAGAAAC
AAAGGCCTGCCTTTCACCTGCAAGGCCTTCGTGTTCGATAAGGCCAGAAAGC
AGTGCCTGTGGTTCCCCTTCAACAGCATGAGCAGCGGCGTGAAGAAAGAAT
TCGGTCACGAGTTCGACCTGTACGAGAATAAGGACTACATTAGAAACTGCAT
CATCGGCAAGGGCCGGAGCTACAAGGGCACCGTGAGCATCACCAAGTCTGG
CATCAAGTGCCAGCCATGGAGTTCCATGATCCCTCATGAGCACAGCTTCCTGC
CAAGCTCTTACCGGGGAAAGGATCTGCAGGAGAACTATTGTAGAAATCCCAG
AGGCGAGGAAGGAGGACCCTGGTGTTTTACCAGCAACCCAGAGGTGCGGTA
CGAGGTGTGCGACATACCCCAGTGCAGCGAGGTGGAATGCATGACATGCAAC
GGCGAGAGCTACCGGGGCCTGATGGACCACACAGAGAGCGGCAAAATCTGC
CAAAGATGGGATCACCAGACCCCTCATAGGCACAAGTTCCTGCCCGAGAGAT
ACCCTGACAAGGGCTTCGACGACAATTACTGCCGGAACCCTGATGGACAGCC
TAGACCTTGGTGCTACACCCTGGACCCTCACACCAGATGGGAATACTGCGCC
ATCAAGACATGTGCCGATAACACCATGAACGACACCGATGTGCCACTCGAGA
CAACTGAGTGTATCCAGGGCCAGGGCGAGGGCTACAGGGGCACCGTGAACA
CCATCTGGAACGGCATCCCTTGTCAGCGGTGGGACAGCCAATACCCCCACGA
ACATGATATGACCCCAGAAAACTTCAAGTGCAAAGATCTGCGGGAAAACTAC
TGTAGAAACCCTGACGGCTCCGAGAGCCCTTGGTGCTTCACCACCGACCCTA
ACATCAGGGTGGGCTACTGTAGCCAGATCCCCAACTGCGACATGAGCCACGG
TCAGGACTGCTACAGAGGAAACGGCAAGAATTACATGGGCAACCTGAGCCA
GACCAGAAGCGGCCTGACCTGCAGCATGTGGGACAAAAACATGGAAGATCT
GCACCGGCACATCTTTTGGGAGCCTGACGCCAGCAAGCTGAATGAGAACTAC
TGTCGGAACCCTGACGACGATGCTCACGGCCCTTGGTGCTACACAGGCAACC
CTCTGATCCCTTGGGACTATTGTCCTATTAGCAGGTGTGAAGGCGACACGACC
CCTACAATCGTGAACCTGGATCACCCAGTGATCAGCTGTGCTAAAACAAAGC
AACTGAGAGTGGTGAATGGTATCCCGACCCGGACCAACATCGGATGGATGGT
CAGCCTGCGGTACAGAAACAAGCACATCTGCGGCGGTAGCCTGATCAAGGA
ATCCTGGGTGTTGACAGCCAGACAGTGTTTTCCCAGCAGAGATCTGAAGGAC
TACGAGGCCTGGCTGGGCATCCACGACGTGCACGGCAGAGGCGACGAGAAG
TGCAAGCAGGTTCTGAACGTGTCCCAGCTGGTGTACGGCCCCGAGGGCTCTG
ATCTGGTTCTGATGAAGCTTGCTAGACCCGCTGTGCTGGATGACTTCGTGTCC
ACCATCGACCTGCCAAATTACGGATGTACAATTCCTGAAAAGACCTCCTGTTC
TGTGTACGGCTGGGGCTACACCGGACTCATCAACTATGACGGCCTGCTGAGA
GTGGCCCACCTGTATATCATGGGCAACGAGAAGTGCAGCCAGCACCATAGAG
GCAAGGTGACCCTGAACGAGTCTGAAATCTGCGCCGGAGCCGAGAAAATCG
GATCTGGCCCCTGCGAAGGAGATTACGGCGGCCCTCTGGTCTGCGAGCAGCA
CAAGATGAGAATGGTGCTGGGCGTGATCGTGCCCGGCAGAGGCTGCGCCATC
CCTAACCGGCCTGGAATCTTCGTGCGCGTGGCCTATTACGCCAAATGGATCCA
CAAAATCATCCTGACATACAAGGTCCCACAGTCTTGATAAGGGCCCACCGGT
TCTGCCGTGGGCCTGTCGTGCCAGTCCTGGGGGCGAGATCGGGAGAATCAC
 
GTCCTGGAACTGCATGTTCTTGCGACTCTTGGGACTTCATCTTAACTTCTCGC
TGCCCCAGCCATGTTTTCAACCATGGCATCCCTCCCCCAATTAGTTCCCTGTC
ATCCTCGTCAACCTTCTCTGTAAGTGCCTGGTAAGCTTGCCCTTGCTTAAGAA
CTCAAAACATAGCTGTGCTCTACCGCGGAAAAAAAAAAAAAAAAAAAA
AAAAAAAGCATATGACTAAAAAAAAAAAAAAAAAAAAAAAAAAAAAA
AAAAAAAAAAAAAAAAAAAAAAAAAAAAAAAAAAAGTTTAAACATTTA
AATTCTAGA
SEQ ID NO: 116
> PD-1 antibody heavy chain amino acid sequence 
MEWSWVFLFFLSVTTGVHSEVQLVESGGGLVQPGGSLRLSCAASGFTFSSYMM
SWVRQAPGKGLEWVATISGGGANTYYPDSVKGRFTISRDNAKNSLYLQMNSLR
AEDTAVYYCARQLYYFDYWGQGTTVTVSSASTKGPSVFPLAPCSRSTSESTAAL
GCLVKDYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDTLMISR
TPEVTCVVVDVSQEDPEVQFNWYVDGVEVHNAKTKPREEQFNSTYRVVSVLT
VLHQDWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSRLTVDKS
RWQEGNVFSCSVMHEALHNHYTQKSLSLSLGK
SEQ ID NO: 117
> PD-1 antibody light chain amino acid sequence 
MSVPTQVLGLLLLWLTDARCDIQMTQSPSSLSASVGDRVTITCLASQTIGTWLT
WYQQKPGKAPKLLIYTATSLADGVPSRFSGSGSGTDFTLTISSLQPEDFATYYCQ
QVYSIPWTFGGGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVT
HQGLSSPVTKSFNRGEC
SEQ ID NO: 118
> PD-1 antibody heavy chain DNA sequence 
ATGGAATGGTCCTGGGTGTTCCTGTTCTTCCTGTCCGTGACCACCGGCGTGC
ACTCCGAAGTGCAGCTGGTGGAATCTGGCGGCGGACTGGTGCAGCCTGGCG
GATCTCTGAGACTGTCTTGTGCCGCCTCCGGCTTCACCTTCTCCAGCTACATG
ATGAGCTGGGTGCGACAGGCCCCTGGCAAGGGACTGGAATGGGTGGCCACA
ATCTCTGGCGGAGGCGCCAACACCTACTACCCCGACTCTGTGAAGGGCCGGT
TCACCATCTCTCGGGACAACGCCAAGAACTCCCTGTACCTGCAGATGAACAG
CCTGCGGGCCGAGGACACCGCCGTGTACTACTGTGCCCGGCAGCTGTACTAC
TTCGACTACTGGGGCCAGGGCACCACCGTGACCGTGTCCTCTGCTTCTACCA
AGGGCCCCTCCGTGTTCCCTCTGGCTCCTTGCTCCAGATCCACCTCCGAGTCC
ACCGCTGCTCTGGGCTGCCTCGTGAAGGACTACTTCCCCGAGCCTGTGACAG
TGTCCTGGAACTCTGGCGCCCTGACCTCTGGCGTGCACACCTTTCCAGCAGT
GCTGCAGTCCTCCGGCCTGTACTCCCTGTCCTCCGTCGTGACAGTGCCCTCC
AGCTCTCTGGGCACCAAGACCTACACCTGTAACGTGGACCACAAGCCCTCCA
 
ACACCAAGGTGGACAAGAGAGTGGAATCTAAGTACGGCCCTCCCTGCCCCC
CTTGTCCTGCCCCTGAATTTCTGGGCGGACCCTCTGTGTTTCTGTTCCCCCCA
AAGCCCAAGGACACCCTGATGATCTCCCGGACCCCCGAAGTGACCTGCGTG
GTGGTGGATGTGTCCCAGGAAGATCCCGAGGTGCAGTTCAATTGGTACGTGG
ACGGCGTGGAAGTGCACAATGCCAAGACCAAGCCTAGAGAGGAACAGTTCA
ACTCCACCTACCGGGTGGTGTCCGTGCTGACCGTGCTGCATCAGGACTGGCT
GAACGGCAAAGAGTACAAGTGCAAGGTGTCCAACAAGGGCCTGCCCAGCTC
CATCGAAAAGACCATCTCCAAGGCCAAGGGCCAGCCCCGGGAACCCCAGGT
GTACACACTGCCTCCAAGCCAGGAAGAGATGACCAAGAACCAGGTGTCCCT
GACCTGCCTCGTGAAAGGCTTCTACCCCTCCGATATCGCCGTGGAATGGGAG
TCCAACGGCCAGCCTGAGAACAACTACAAGACCACCCCCCCTGTGCTGGAC
TCCGACGGCTCCTTCTTTCTGTACTCTCGCCTGACCGTGGACAAGTCCCGGT
GGCAGGAAGGCAACGTGTTCTCCTGCAGCGTGATGCACGAGGCCCTGCACA
ACCACTACACCCAGAAGTCCCTGTCCCTGTCTCTGGGAAAG
SEQ ID NO: 119
> PD-1 antibody light chain DNA sequence 
ATGTCCGTGCCTACCCAGGTGCTGGGACTGCTGCTGCTGTGGCTGACCGACG
CCAGATGCGACATCCAGATGACCCAGTCCCCCTCCAGCCTGTCTGCCTCTGT
GGGCGACAGAGTGACCATCACCTGTCTGGCCTCCCAGACCATCGGCACCTGG
CTGACCTGGTATCAGCAGAAGCCTGGCAAGGCCCCCAAGCTGCTGATCTACA
CCGCCACCAGTCTGGCCGATGGCGTGCCCTCTAGATTCTCCGGCTCTGGCTCT
GGCACCGACTTTACCCTGACCATCAGCTCCCTGCAGCCCGAGGACTTCGCCA
CCTACTACTGCCAGCAGGTGTACTCCATCCCCTGGACCTTTGGCGGAGGCAC
CAAGGTGGAAATCAAGCGGACCGTGGCCGCTCCCTCCGTGTTCATCTTCCCA
CCTTCCGACGAGCAGCTGAAGTCCGGAACCGCCAGTGTCGTGTGCCTGCTG
AACAACTTCTACCCCCGCGAGGCCAAGGTGCAGTGGAAGGTGGACAACGCC
CTGCAGTCCGGCAACTCCCAGGAATCCGTGACCGAGCAGGACTCCAAGGAC
AGCACCTACTCCCTGTCCTCCACCCTGACCCTGTCCAAGGCCGACTACGAGA
AGCACAAGGTGTACGCCTGCGAAGTGACCCACCAGGGCCTGTCTAGCCCCG
TGACCAAGTCTTTCAACCGGGGCGAGTGC
SEQ ID NO: 120
> PD-1 antibody heavy chain mRNA sequence 
AUGGAAUGGUCCUGGGUGUUCCUGUUCUUCCUGUCCGUGACCACCGGCGU
GCACUCCGAAGUGCAGCUGGUGGAAUCUGGCGGCGGACUGGUGCAGCCUG
GCGGAUCUCUGAGACUGUCUUGUGCCGCCUCCGGCUUCACCUUCUCCAGC
UACAUGAUGAGCUGGGUGCGACAGGCCCCUGGCAAGGGACUGGAAUGGG
UGGCCACAAUCUCUGGCGGAGGCGCCAACACCUACUACCCCGACUCUGUG
AAGGGCCGGUUCACCAUCUCUCGGGACAACGCCAAGAACUCCCUGUACCU
GCAGAUGAACAGCCUGCGGGCCGAGGACACCGCCGUGUACUACUGUGCCC
GGCAGCUGUACUACUUCGACUACUGGGGCCAGGGCACCACCGUGACCGUG
 
UCCUCUGCUUCUACCAAGGGCCCCUCCGUGUUCCCUCUGGCUCCUUGCUC
CAGAUCCACCUCCGAGUCCACCGCUGCUCUGGGCUGCCUCGUGAAGGACU
ACUUCCCCGAGCCUGUGACAGUGUCCUGGAACUCUGGCGCCCUGACCUCU
GGCGUGCACACCUUUCCAGCAGUGCUGCAGUCCUCCGGCCUGUACUCCCU
GUCCUCCGUCGUGACAGUGCCCUCCAGCUCUCUGGGCACCAAGACCUACA
CCUGUAACGUGGACCACAAGCCCUCCAACACCAAGGUGGACAAGAGAGUG
GAAUCUAAGUACGGCCCUCCCUGCCCCCCUUGUCCUGCCCCUGAAUUUCU
GGGCGGACCCUCUGUGUUUCUGUUCCCCCCAAAGCCCAAGGACACCCUGA
UGAUCUCCCGGACCCCCGAAGUGACCUGCGUGGUGGUGGAUGUGUCCCAG
GAAGAUCCCGAGGUGCAGUUCAAUUGGUACGUGGACGGCGUGGAAGUGC
ACAAUGCCAAGACCAAGCCUAGAGAGGAACAGUUCAACUCCACCUACCGG
GUGGUGUCCGUGCUGACCGUGCUGCAUCAGGACUGGCUGAACGGCAAAG
AGUACAAGUGCAAGGUGUCCAACAAGGGCCUGCCCAGCUCCAUCGAAAAG
ACCAUCUCCAAGGCCAAGGGCCAGCCCCGGGAACCCCAGGUGUACACACU
GCCUCCAAGCCAGGAAGAGAUGACCAAGAACCAGGUGUCCCUGACCUGCC
UCGUGAAAGGCUUCUACCCCUCCGAUAUCGCCGUGGAAUGGGAGUCCAAC
GGCCAGCCUGAGAACAACUACAAGACCACCCCCCCUGUGCUGGACUCCGA
CGGCUCCUUCUUUCUGUACUCUCGCCUGACCGUGGACAAGUCCCGGUGGC
AGGAAGGCAACGUGUUCUCCUGCAGCGUGAUGCACGAGGCCCUGCACAAC
CACUACACCCAGAAGUCCCUGUCCCUGUCUCUGGGAAAG
SEQ ID NO: 121
> PD-1 antibody light chain mRNA sequence 
AUGUCCGUGCCUACCCAGGUGCUGGGACUGCUGCUGCUGUGGCUGACCGA
CGCCAGAUGCGACAUCCAGAUGACCCAGUCCCCCUCCAGCCUGUCUGCCU
CUGUGGGCGACAGAGUGACCAUCACCUGUCUGGCCUCCCAGACCAUCGGC
ACCUGGCUGACCUGGUAUCAGCAGAAGCCUGGCAAGGCCCCCAAGCUGCU
GAUCUACACCGCCACCAGUCUGGCCGAUGGCGUGCCCUCUAGAUUCUCCG
GCUCUGGCUCUGGCACCGACUUUACCCUGACCAUCAGCUCCCUGCAGCCC
GAGGACUUCGCCACCUACUACUGCCAGCAGGUGUACUCCAUCCCCUGGAC
CUUUGGCGGAGGCACCAAGGUGGAAAUCAAGCGGACCGUGGCCGCUCCCU
CCGUGUUCAUCUUCCCACCUUCCGACGAGCAGCUGAAGUCCGGAACCGCC
AGUGUCGUGUGCCUGCUGAACAACUUCUACCCCCGCGAGGCCAAGGUGCA
GUGGAAGGUGGACAACGCCCUGCAGUCCGGCAACUCCCAGGAAUCCGUGA
CCGAGCAGGACUCCAAGGACAGCACCUACUCCCUGUCCUCCACCCUGACC
CUGUCCAAGGCCGACUACGAGAAGCACAAGGUGUACGCCUGCGAAGUGAC
CCACCAGGGCCUGUCUAGCCCCGUGACCAAGUCUUUCAACCGGGGCGAGU
GC
SEQ ID NO: 122
> 5'UTR-anti PD-1-α globin 3'UTR-A30L70 (Mod.-anti PD-1) GGGAAATAAGAGAGAAAAGAAGAGTAAGAAGAAATATAAGAGCCACCATGG 
AATGGTCCTGGGTGTTCCTGTTCTTCCTGTCCGTGACCACCGGCGTGCACTCC
GAAGTGCAGCTGGTGGAATCTGGCGGCGGACTGGTGCAGCCTGGCGGATCT
CTGAGACTGTCTTGTGCCGCCTCCGGCTTCACCTTCTCCAGCTACATGATGAG
CTGGGTGCGACAGGCCCCTGGCAAGGGACTGGAATGGGTGGCCACAATCTC
TGGCGGAGGCGCCAACACCTACTACCCCGACTCTGTGAAGGGCCGGTTCAC
CATCTCTCGGGACAACGCCAAGAACTCCCTGTACCTGCAGATGAACAGCCTG
CGGGCCGAGGACACCGCCGTGTACTACTGTGCCCGGCAGCTGTACTACTTCG
ACTACTGGGGCCAGGGCACCACCGTGACCGTGTCCTCTGCTTCTACCAAGGG
CCCCTCCGTGTTCCCTCTGGCTCCTTGCTCCAGATCCACCTCCGAGTCCACCG
CTGCTCTGGGCTGCCTCGTGAAGGACTACTTCCCCGAGCCTGTGACAGTGTC
CTGGAACTCTGGCGCCCTGACCTCTGGCGTGCACACCTTTCCAGCAGTGCTG
CAGTCCTCCGGCCTGTACTCCCTGTCCTCCGTCGTGACAGTGCCCTCCAGCTC
TCTGGGCACCAAGACCTACACCTGTAACGTGGACCACAAGCCCTCCAACAC
CAAGGTGGACAAGAGAGTGGAATCTAAGTACGGCCCTCCCTGCCCCCCTTGT
CCTGCCCCTGAATTTCTGGGCGGACCCTCTGTGTTTCTGTTCCCCCCAAAGCC
CAAGGACACCCTGATGATCTCCCGGACCCCCGAAGTGACCTGCGTGGTGGTG
GATGTGTCCCAGGAAGATCCCGAGGTGCAGTTCAATTGGTACGTGGACGGCG
TGGAAGTGCACAATGCCAAGACCAAGCCTAGAGAGGAACAGTTCAACTCCA
CCTACCGGGTGGTGTCCGTGCTGACCGTGCTGCATCAGGACTGGCTGAACGG
CAAAGAGTACAAGTGCAAGGTGTCCAACAAGGGCCTGCCCAGCTCCATCGA
AAAGACCATCTCCAAGGCCAAGGGCCAGCCCCGGGAACCCCAGGTGTACAC
ACTGCCTCCAAGCCAGGAAGAGATGACCAAGAACCAGGTGTCCCTGACCTG
CCTCGTGAAAGGCTTCTACCCCTCCGATATCGCCGTGGAATGGGAGTCCAAC
GGCCAGCCTGAGAACAACTACAAGACCACCCCCCCTGTGCTGGACTCCGAC
GGCTCCTTCTTTCTGTACTCTCGCCTGACCGTGGACAAGTCCCGGTGGCAGG
AAGGCAACGTGTTCTCCTGCAGCGTGATGCACGAGGCCCTGCACAACCACTA
CACCCAGAAGTCCCTGTCCCTGTCTCTGGGAAAGGCCACCAACTTCTCCCTG
CTGAAGCAGGCCGGCGACGTGGAGGAGAACCCCGGCCCCATGTCCGTGCCT
ACCCAGGTGCTGGGACTGCTGCTGCTGTGGCTGACCGACGCCAGATGCGAC
ATCCAGATGACCCAGTCCCCCTCCAGCCTGTCTGCCTCTGTGGGCGACAGAG
TGACCATCACCTGTCTGGCCTCCCAGACCATCGGCACCTGGCTGACCTGGTAT
CAGCAGAAGCCTGGCAAGGCCCCCAAGCTGCTGATCTACACCGCCACCAGT
CTGGCCGATGGCGTGCCCTCTAGATTCTCCGGCTCTGGCTCTGGCACCGACTT
TACCCTGACCATCAGCTCCCTGCAGCCCGAGGACTTCGCCACCTACTACTGC
CAGCAGGTGTACTCCATCCCCTGGACCTTTGGCGGAGGCACCAAGGTGGAAA
TCAAGCGGACCGTGGCCGCTCCCTCCGTGTTCATCTTCCCACCTTCCGACGA
GCAGCTGAAGTCCGGAACCGCCAGTGTCGTGTGCCTGCTGAACAACTTCTAC
CCCCGCGAGGCCAAGGTGCAGTGGAAGGTGGACAACGCCCTGCAGTCCGGC
AACTCCCAGGAATCCGTGACCGAGCAGGACTCCAAGGACAGCACCTACTCC
CTGTCCTCCACCCTGACCCTGTCCAAGGCCGACTACGAGAAGCACAAGGTGT
 
ACGCCTGCGAAGTGACCCACCAGGGCCTGTCTAGCCCCGTGACCAAGTCTTT
CAACCGGGGCGAGTGCTGATAAGGGCCCACCGGTTGATAATAGGCTGGAGCC
TCGGTGGCCATGCTTCTTGCCCCTTGGGCCTCCCCCCAGCCCCTCCTCCCCTT
CCTGCACCCGTACCCCCGTGGTCTTTGAATAAAGTCTGAGTGGGCGGCCTCG
AGCCGCGGAAAAAAAAAAAAAAAAAAAAAAAAAAAAAAGCATATGACTAA
AAAAAAAAAAAAAAAAAAAAAAAAAAAAAAAAAAAAAAAAAAAAAAAAAAAA
AAAAAAAAAAAAAAAAAAAAATTATTGTTTAAACATTTAAATTCTAGA
SEQ ID NO: 123
> ACTG1 5'UTR-anti PD-1-CTSB 3'UTR-A30L70 (A1B12-anti PD-1) 
CTCTCGCACTCTGTTCTTCCGCCGCTCCGCCGTCGCGTTTCTCTGCCGGTCGC
ACCACCATGGAATGGTCCTGGGTGTTCCTGTTCTTCCTGTCCGTGACCACCG
GCGTGCACTCCGAAGTGCAGCTGGTGGAATCTGGCGGCGGACTGGTGCAGC
CTGGCGGATCTCTGAGACTGTCTTGTGCCGCCTCCGGCTTCACCTTCTCCAGC
TACATGATGAGCTGGGTGCGACAGGCCCCTGGCAAGGGACTGGAATGGGTG
GCCACAATCTCTGGCGGAGGCGCCAACACCTACTACCCCGACTCTGTGAAGG
GCCGGTTCACCATCTCTCGGGACAACGCCAAGAACTCCCTGTACCTGCAGAT
GAACAGCCTGCGGGCCGAGGACACCGCCGTGTACTACTGTGCCCGGCAGCT
GTACTACTTCGACTACTGGGGCCAGGGCACCACCGTGACCGTGTCCTCTGCT
TCTACCAAGGGCCCCTCCGTGTTCCCTCTGGCTCCTTGCTCCAGATCCACCTC
CGAGTCCACCGCTGCTCTGGGCTGCCTCGTGAAGGACTACTTCCCCGAGCCT
GTGACAGTGTCCTGGAACTCTGGCGCCCTGACCTCTGGCGTGCACACCTTTC
CAGCAGTGCTGCAGTCCTCCGGCCTGTACTCCCTGTCCTCCGTCGTGACAGT
GCCCTCCAGCTCTCTGGGCACCAAGACCTACACCTGTAACGTGGACCACAAG
CCCTCCAACACCAAGGTGGACAAGAGAGTGGAATCTAAGTACGGCCCTCCC
TGCCCCCCTTGTCCTGCCCCTGAATTTCTGGGCGGACCCTCTGTGTTTCTGTT
CCCCCCAAAGCCCAAGGACACCCTGATGATCTCCCGGACCCCCGAAGTGAC
CTGCGTGGTGGTGGATGTGTCCCAGGAAGATCCCGAGGTGCAGTTCAATTGG
TACGTGGACGGCGTGGAAGTGCACAATGCCAAGACCAAGCCTAGAGAGGAA
CAGTTCAACTCCACCTACCGGGTGGTGTCCGTGCTGACCGTGCTGCATCAGG
ACTGGCTGAACGGCAAAGAGTACAAGTGCAAGGTGTCCAACAAGGGCCTGC
CCAGCTCCATCGAAAAGACCATCTCCAAGGCCAAGGGCCAGCCCCGGGAAC
CCCAGGTGTACACACTGCCTCCAAGCCAGGAAGAGATGACCAAGAACCAGG
TGTCCCTGACCTGCCTCGTGAAAGGCTTCTACCCCTCCGATATCGCCGTGGAA
TGGGAGTCCAACGGCCAGCCTGAGAACAACTACAAGACCACCCCCCCTGTG
CTGGACTCCGACGGCTCCTTCTTTCTGTACTCTCGCCTGACCGTGGACAAGT
CCCGGTGGCAGGAAGGCAACGTGTTCTCCTGCAGCGTGATGCACGAGGCCC
TGCACAACCACTACACCCAGAAGTCCCTGTCCCTGTCTCTGGGAAAGGCCAC
CAACTTCTCCCTGCTGAAGCAGGCCGGCGACGTGGAGGAGAACCCCGGCCC
CATGTCCGTGCCTACCCAGGTGCTGGGACTGCTGCTGCTGTGGCTGACCGAC
GCCAGATGCGACATCCAGATGACCCAGTCCCCCTCCAGCCTGTCTGCCTCTG
 
TGGGCGACAGAGTGACCATCACCTGTCTGGCCTCCCAGACCATCGGCACCTG
GCTGACCTGGTATCAGCAGAAGCCTGGCAAGGCCCCCAAGCTGCTGATCTAC
ACCGCCACCAGTCTGGCCGATGGCGTGCCCTCTAGATTCTCCGGCTCTGGCT
CTGGCACCGACTTTACCCTGACCATCAGCTCCCTGCAGCCCGAGGACTTCGC
CACCTACTACTGCCAGCAGGTGTACTCCATCCCCTGGACCTTTGGCGGAGGC
ACCAAGGTGGAAATCAAGCGGACCGTGGCCGCTCCCTCCGTGTTCATCTTCC
CACCTTCCGACGAGCAGCTGAAGTCCGGAACCGCCAGTGTCGTGTGCCTGC
TGAACAACTTCTACCCCCGCGAGGCCAAGGTGCAGTGGAAGGTGGACAACG
CCCTGCAGTCCGGCAACTCCCAGGAATCCGTGACCGAGCAGGACTCCAAGG
ACAGCACCTACTCCCTGTCCTCCACCCTGACCCTGTCCAAGGCCGACTACGA
GAAGCACAAGGTGTACGCCTGCGAAGTGACCCACCAGGGCCTGTCTAGCCC
CGTGACCAAGTCTTTCAACCGGGGCGAGTGCTGATAAGGGCCCACCGGTCTG
CCGTGGGCCTGTCGTGCCAGTCCTGGGGGCGAGATCGGGAGAATCACGTCCT
GGAACTGCATGTTCTTGCGACTCTTGGGACTTCATCTTAACTTCTCGCTGCCC
CAGCCATGTTTTCAACCATGGCATCCCTCCCCCAATTAGTTCCCTGTCATCCTC
GTCAACCTTCTCTGTAAGTGCCTGGTAAGCTTGCCCTTGCTTAAGAACTCAA
AACATAGCTGTGCTCTACTCGAGCCGCGGAAAAAAAAAAAAAAAAAAAAA
AAAAAAAAAGCATATGACTAAAAAAAAAAAAAAAAAAAAAAAAAAAAAAA
AAAAAAAAAAAAAAAAAAAAAAAAAAAAAAAAAAAAAAATTATTGTTTAAA
CATTTAAATTCTAGA
SEQ ID NO: 124
> CHCHD10 5'UTR-anti PD-1-CTSB 3'UTR-A30L70 (A15B12-anti PD-1) 
GGCATTTGTCCCCGCGACAGCACCGCTGCCGCCGTCTCTAAGGTCGCCCGGG
TCCCACCGCCGCCACCATGGAATGGTCCTGGGTGTTCCTGTTCTTCCTGTCCG
TGACCACCGGCGTGCACTCCGAAGTGCAGCTGGTGGAATCTGGCGGCGGAC
TGGTGCAGCCTGGCGGATCTCTGAGACTGTCTTGTGCCGCCTCCGGCTTCAC
CTTCTCCAGCTACATGATGAGCTGGGTGCGACAGGCCCCTGGCAAGGGACTG
GAATGGGTGGCCACAATCTCTGGCGGAGGCGCCAACACCTACTACCCCGACT
CTGTGAAGGGCCGGTTCACCATCTCTCGGGACAACGCCAAGAACTCCCTGTA
CCTGCAGATGAACAGCCTGCGGGCCGAGGACACCGCCGTGTACTACTGTGCC
CGGCAGCTGTACTACTTCGACTACTGGGGCCAGGGCACCACCGTGACCGTGT
CCTCTGCTTCTACCAAGGGCCCCTCCGTGTTCCCTCTGGCTCCTTGCTCCAGA
TCCACCTCCGAGTCCACCGCTGCTCTGGGCTGCCTCGTGAAGGACTACTTCC
CCGAGCCTGTGACAGTGTCCTGGAACTCTGGCGCCCTGACCTCTGGCGTGCA
CACCTTTCCAGCAGTGCTGCAGTCCTCCGGCCTGTACTCCCTGTCCTCCGTCG
TGACAGTGCCCTCCAGCTCTCTGGGCACCAAGACCTACACCTGTAACGTGGA
CCACAAGCCCTCCAACACCAAGGTGGACAAGAGAGTGGAATCTAAGTACGG
CCCTCCCTGCCCCCCTTGTCCTGCCCCTGAATTTCTGGGCGGACCCTCTGTGT
TTCTGTTCCCCCCAAAGCCCAAGGACACCCTGATGATCTCCCGGACCCCCGA
AGTGACCTGCGTGGTGGTGGATGTGTCCCAGGAAGATCCCGAGGTGCAGTTC
 
AATTGGTACGTGGACGGCGTGGAAGTGCACAATGCCAAGACCAAGCCTAGA
GAGGAACAGTTCAACTCCACCTACCGGGTGGTGTCCGTGCTGACCGTGCTGC
ATCAGGACTGGCTGAACGGCAAAGAGTACAAGTGCAAGGTGTCCAACAAGG
GCCTGCCCAGCTCCATCGAAAAGACCATCTCCAAGGCCAAGGGCCAGCCCC
GGGAACCCCAGGTGTACACACTGCCTCCAAGCCAGGAAGAGATGACCAAGA
ACCAGGTGTCCCTGACCTGCCTCGTGAAAGGCTTCTACCCCTCCGATATCGCC
GTGGAATGGGAGTCCAACGGCCAGCCTGAGAACAACTACAAGACCACCCCC
CCTGTGCTGGACTCCGACGGCTCCTTCTTTCTGTACTCTCGCCTGACCGTGGA
CAAGTCCCGGTGGCAGGAAGGCAACGTGTTCTCCTGCAGCGTGATGCACGA
GGCCCTGCACAACCACTACACCCAGAAGTCCCTGTCCCTGTCTCTGGGAAAG
GCCACCAACTTCTCCCTGCTGAAGCAGGCCGGCGACGTGGAGGAGAACCCC
GGCCCCATGTCCGTGCCTACCCAGGTGCTGGGACTGCTGCTGCTGTGGCTGA
CCGACGCCAGATGCGACATCCAGATGACCCAGTCCCCCTCCAGCCTGTCTGC
CTCTGTGGGCGACAGAGTGACCATCACCTGTCTGGCCTCCCAGACCATCGGC
ACCTGGCTGACCTGGTATCAGCAGAAGCCTGGCAAGGCCCCCAAGCTGCTGA
TCTACACCGCCACCAGTCTGGCCGATGGCGTGCCCTCTAGATTCTCCGGCTCT
GGCTCTGGCACCGACTTTACCCTGACCATCAGCTCCCTGCAGCCCGAGGACT
TCGCCACCTACTACTGCCAGCAGGTGTACTCCATCCCCTGGACCTTTGGCGG
AGGCACCAAGGTGGAAATCAAGCGGACCGTGGCCGCTCCCTCCGTGTTCATC
TTCCCACCTTCCGACGAGCAGCTGAAGTCCGGAACCGCCAGTGTCGTGTGCC
TGCTGAACAACTTCTACCCCCGCGAGGCCAAGGTGCAGTGGAAGGTGGACA
ACGCCCTGCAGTCCGGCAACTCCCAGGAATCCGTGACCGAGCAGGACTCCA
AGGACAGCACCTACTCCCTGTCCTCCACCCTGACCCTGTCCAAGGCCGACTA
CGAGAAGCACAAGGTGTACGCCTGCGAAGTGACCCACCAGGGCCTGTCTAG
CCCCGTGACCAAGTCTTTCAACCGGGGCGAGTGCTGATAAGGGCCCACCGGT
CTGCCGTGGGCCTGTCGTGCCAGTCCTGGGGGCGAGATCGGGAGAATCACG
TCCTGGAACTGCATGTTCTTGCGACTCTTGGGACTTCATCTTAACTTCTCGCT
GCCCCAGCCATGTTTTCAACCATGGCATCCCTCCCCCAATTAGTTCCCTGTCA
TCCTCGTCAACCTTCTCTGTAAGTGCCTGGTAAGCTTGCCCTTGCTTAAGAAC
TCAAAACATAGCTGTGCTCTACTCGAGCCGCGGAAAAAAAAAAAAAAAAAA
AAAAAAAAAAAAGCATATGACTAAAAAAAAAAAAAAAAAAAAAAAAAA
AAAAAAAAAAAAAAAAAAAAAAAAAAAAAAAAAAAAAAAAAAAATTATTGTT
TAAACATTTAAATTCTAGA
SEQ ID NO: 125
> Nucleotide sequence of firefly luciferase (Fluc) 
ATGGAAGACGCCAAAAACATAAAGAAAGGCCCGGCGCCATTCTATCCGCTGG
AAGATGGAACCGCTGGAGAGCAACTGCATAAGGCTATGAAGAGATACGCCCT
GGTTCCTGGAACAATTGCTTTTACAGATGCACATATCGAGGTGGACATCACTT
ACGCTGAGTACTTCGAAATGTCCGTTCGGTTGGCAGAAGCTATGAAACGATA
TGGGCTGAATACAAATCACAGAATCGTCGTATGCAGTGAAAACTCTCTTCAAT
 
TCTTTATGCCGGTGTTGGGCGCGTTATTTATCGGAGTTGCAGTTGCGCCCGCG
AACGACATTTATAATGAACGTGAATTGCTCAACAGTATGGGCATTTCGCAGCC
TACCGTGGTGTTCGTTTCCAAAAAGGGGTTGCAAAAAATTTTGAACGTGCAA
AAAAAGCTCCCAATCATCCAAAAAATTATTATCATGGATTCTAAAACGGATTA
CCAGGGATTTCAGTCGATGTACACGTTCGTCACATCTCATCTACCTCCCGGTT
TTAATGAATACGATTTTGTGCCAGAGTCCTTCGATAGGGACAAGACAATTGCA
CTGATCATGAACTCCTCTGGATCTACTGGTCTGCCTAAAGGTGTCGCTCTGCC
TCATAGAACTGCCTGCGTGAGATTCTCGCATGCCAGAGATCCTATTTTTGGCA
ATCAAATCATTCCGGATACTGCGATTTTAAGTGTTGTTCCATTCCATCACGGTT
TTGGAATGTTTACTACACTCGGATATTTGATATGTGGATTTCGAGTCGTCTTAA
TGTATAGATTTGAAGAAGAGCTGTTTCTGAGGAGCCTTCAGGATTACAAGATT
CAAAGTGCGCTGCTGGTGCCAACCCTATTCTCCTTCTTCGCCAAAAGCACTC
TGATTGACAAATACGATTTATCTAATTTACACGAAATTGCTTCTGGTGGCGCTC
CCCTCTCTAAGGAAGTCGGGGAAGCGGTTGCCAAGAGGTTCCATCTGCCAG
GTATCAGGCAAGGATATGGGCTCACTGAGACTACATCAGCTATTCTGATTACA
CCCGAGGGGGATGATAAACCGGGCGCGGTCGGTAAAGTTGTTCCATTTTTTG
AAGCGAAGGTTGTGGATCTGGATACCGGGAAAACGCTGGGCGTTAATCAAA
GAGGCGAACTGTGTGTGAGAGGTCCTATGATTATGTCCGGTTATGTAAACAAT
CCGGAAGCGACCAACGCCTTGATTGACAAGGATGGATGGCTACATTCTGGAG
ACATAGCTTACTGGGACGAAGACGAACACTTCTTCATCGTTGACCGCCTGAA
GTCTCTGATTAAGTACAAAGGCTATCAGGTGGCTCCCGCTGAATTGGAATCCA
TCTTGCTCCAACACCCCAACATCTTCGACGCAGGTGTCGCAGGTCTTCCCGA
CGATGACGCCGGTGAACTTCCCGCCGCCGTTGTTGTTTTGGAGCACGGAAAG
ACGATGACGGAAAAAGAGATCGTGGATTACGTCGCCAGTCAAGTAACAACC
GCGAAAAAGTTGCGCGGAGGAGTTGTGTTTGTGGACGAAGTACCGAAAGGT
CTTACCGGAAAACTCGACGCAAGAAAAATCAGAGAGATCCTCATAAAGGCC
AAGAAGGGCGGAAAGATCGCCGTGTAA
SEQ ID NO: 126
> HGF-OS2 expression vector CHCHD10 5'UTR-hHGF-CTSB 3'UTR-A30L70 (A16B12-HGF-OS2) 
CCGCTGCCGCCGTCTCTAAGGTCGCCCGGGTCCCACCGCCGCCACCGCTAGC
GCCACCATGTGGGTCACCAAACTGCTGCCCGCCCTGCTGCTGCAGCACGTGC
TCCTGCACCTGCTGCTGCTGCCTATCGCCATCCCTTACGCCGAAGGCCAGAG
AAAGCGGAGAAATACCATTCACGAATTTAAGAAGAGCGCCAAGACCACACT
GATCAAGATCGACCCTGCCCTGAAAATCAAGACCAAGAAAGTGAACACAGC
CGACCAATGCGCTAATAGATGCACAAGAAACAAAGGCCTGCCTTTCACCTGC
AAGGCCTTCGTGTTCGATAAGGCCAGAAAGCAGTGCCTGTGGTTCCCCTTCA
ACAGCATGAGCAGCGGCGTGAAGAAAGAATTCGGTCACGAGTTCGACCTGT
ACGAGAATAAGGACTACATTAGAAACTGCATCATCGGCAAGGGCCGGAGCTA
CAAGGGCACCGTGAGCATCACCAAGTCTGGCATCAAGTGCCAGCCATGGAG
 
TTCCATGATCCCTCATGAGCACAGCTTCCTGCCAAGCTCTTACCGGGGAAAG
GATCTGCAGGAGAACTATTGTAGAAATCCCAGAGGCGAGGAAGGAGGACCC
TGGTGTTTTACCAGCAACCCAGAGGTGCGGTACGAGGTGTGCGACATACCCC
AGTGCAGCGAGGTGGAATGCATGACATGCAACGGCGAGAGCTACCGGGGCC
TGATGGACCACACAGAGAGCGGCAAAATCTGCCAAAGATGGGATCACCAGA
CCCCTCATAGGCACAAGTTCCTGCCCGAGAGATACCCTGACAAGGGCTTCGA
CGACAATTACTGCCGGAACCCTGATGGACAGCCTAGACCTTGGTGCTACACC
CTGGACCCTCACACCAGATGGGAATACTGCGCCATCAAGACATGTGCCGATA
ACACCATGAACGACACCGATGTGCCACTCGAGACAACTGAGTGTATCCAGGG
CCAGGGCGAGGGCTACAGGGGCACCGTGAACACCATCTGGAACGGCATCCC
TTGTCAGCGGTGGGACAGCCAATACCCCCACGAACATGATATGACCCCAGAA
AACTTCAAGTGCAAAGATCTGCGGGAAAACTACTGTAGAAACCCTGACGGC
TCCGAGAGCCCTTGGTGCTTCACCACCGACCCTAACATCAGGGTGGGCTACT
GTAGCCAGATCCCCAACTGCGACATGAGCCACGGTCAGGACTGCTACAGAG
GAAACGGCAAGAATTACATGGGCAACCTGAGCCAGACCAGAAGCGGCCTGA
CCTGCAGCATGTGGGACAAAAACATGGAAGATCTGCACCGGCACATCTTTTG
GGAGCCTGACGCCAGCAAGCTGAATGAGAACTACTGTCGGAACCCTGACGA
CGATGCTCACGGCCCTTGGTGCTACACAGGCAACCCTCTGATCCCTTGGGAC
TATTGTCCTATTAGCAGGTGTGAAGGCGACACGACCCCTACAATCGTGAACCT
GGATCACCCAGTGATCAGCTGTGCTAAAACAAAGCAACTGAGAGTGGTGAA
TGGTATCCCGACCCGGACCAACATCGGATGGATGGTCAGCCTGCGGTACAGA
AACAAGCACATCTGCGGCGGTAGCCTGATCAAGGAATCCTGGGTGTTGACAG
CCAGACAGTGTTTTCCCAGCAGAGATCTGAAGGACTACGAGGCCTGGCTGG
GCATCCACGACGTGCACGGCAGAGGCGACGAGAAGTGCAAGCAGGTTCTGA
ACGTGTCCCAGCTGGTGTACGGCCCCGAGGGCTCTGATCTGGTTCTGATGAA
GCTTGCTAGACCCGCTGTGCTGGATGACTTCGTGTCCACCATCGACCTGCCA
AATTACGGATGTACAATTCCTGAAAAGACCTCCTGTTCTGTGTACGGCTGGGG
CTACACCGGACTCATCAACTATGACGGCCTGCTGAGAGTGGCCCACCTGTATA
TCATGGGCAACGAGAAGTGCAGCCAGCACCATAGAGGCAAGGTGACCCTGA
ACGAGTCTGAAATCTGCGCCGGAGCCGAGAAAATCGGATCTGGCCCCTGCG
AAGGAGATTACGGCGGCCCTCTGGTCTGCGAGCAGCACAAGATGAGAATGG
TGCTGGGCGTGATCGTGCCCGGCAGAGGCTGCGCCATCCCTAACCGGCCTGG
AATCTTCGTGCGCGTGGCCTATTACGCCAAATGGATCCACAAAATCATCCTGA
CATACAAGGTCCCACAGTCTTGATAAGGGCCCACCGGTTCTGCCGTGGGCCT
GTCGTGCCAGTCCTGGGGGCGAGATCGGGAGAATCACGTCCTGGAACTGCAT
GTTCTTGCGACTCTTGGGACTTCATCTTAACTTCTCGCTGCCCCAGCCATGTT
TTCAACCATGGCATCCCTCCCCCAATTAGTTCCCTGTCATCCTCGTCAACCTTC
TCTGTAAGTGCCTGGTAAGCTTGCCCTTGCTTAAGAACTCAAAACATAGCTGT
GCTCTACCGCGGAAAAAAAAAAAAAAAAAAAAAAAAAAAAAAAAAGCATATGA
CTAAAAAAAAAAAAAAAAAAAAAAAAAAAAAAAAAAAAAAAAAAAAAAAAAA
 
AAAAAAAAAAAAAAAAAAAAAAAAAAGTTTAAACATTTAAATTCTAGA

SEQ ID NO: 127
> mRNA sequence of hHGF protein 
AUGUGGGUGACCAAACUCCUGCCAGCCCUGCUGCUGCAGCAUGUCCUCCU
GCAUCUCCUCCUGCUCCCCAUCGCCAUCCCCUAUGCAGAGGGACAAAGGA
AAAGAAGAAAUACAAUUCAUGAAUUCAAAAAAUCAGCAAAGACUACCCU
AAUCAAAAUAGAUCCAGCACUGAAGAUAAAAACCAAAAAAGUGAAUACU
GCAGACCAAUGUGCUAAUAGAUGUACUAGGAAUAAAGGACUUCCAUUCA
CUUGCAAGGCUUUUGUUUUUGAUAAAGCAAGAAAACAAUGCCUCUGGUU
CCCCUUCAAUAGCAUGUCAAGUGGAGUGAAAAAAGAAUUUGGCCAUGAA
UUUGACCUCUAUGAAAACAAAGACUACAUUAGAAACUGCAUCAUUGGUA
AAGGACGCAGCUACAAGGGAACAGUAUCUAUCACUAAGAGUGGCAUCAA
AUGUCAGCCCUGGAGUUCCAUGAUACCACACGAACACAGCUUUUUGCCUU
CGAGCUAUCGGGGUAAAGACCUACAGGAAAACUACUGUCGAAAUCCUCG
AGGGGAAGAAGGGGGACCCUGGUGUUUCACAAGCAAUCCAGAGGUACGC
UACGAAGUCUGUGACAUUCCUCAGUGUUCAGAAGUUGAAUGCAUGACCU
GCAAUGGGGAGAGUUAUCGAGGUCUCAUGGAUCAUACAGAAUCAGGCAA
GAUUUGUCAGCGCUGGGAUCAUCAGACACCACACCGGCACAAAUUCUUGC
CUGAAAGAUAUCCCGACAAGGGCUUUGAUGAUAAUUAUUGCCGCAAUCC
CGAUGGCCAGCCGAGGCCAUGGUGCUAUACUCUUGACCCUCACACCCGCU
GGGAGUACUGUGCAAUUAAAACAUGCGCUGACAAUACUAUGAAUGACAC
UGAUGUUCCUUUGGAAACAACUGAAUGCAUCCAAGGUCAAGGAGAAGGC
UACAGGGGCACUGUCAAUACCAUUUGGAAUGGAAUUCCAUGUCAGCGUU
GGGAUUCUCAGUAUCCUCACGAGCAUGACAUGACUCCUGAAAAUUUCAA
GUGCAAGGACCUACGAGAAAAUUACUGCCGAAAUCCAGAUGGGUCUGAA
UCACCCUGGUGUUUUACCACUGAUCCAAACAUCCGAGUUGGCUACUGCUC
CCAAAUUCCAAACUGUGAUAUGUCACAUGGACAAGAUUGUUAUCGUGGG
AAUGGCAAAAAUUAUAUGGGCAACUUAUCCCAAACAAGAUCUGGACUAA
CAUGUUCAAUGUGGGACAAGAACAUGGAAGACUUACAUCGUCAUAUCUU
CUGGGAACCAGAUGCAAGUAAGCUGAAUGAGAAUUACUGCCGAAAUCCA
GAUGAUGAUGCUCAUGGACCCUGGUGCUACACGGGAAAUCCACUCAUUCC
UUGGGAUUAUUGCCCUAUUUCUCGUUGUGAAGGUGAUACCACACCUACA
AUAGUCAAUUUAGACCAUCCCGUAAUAUCUUGUGCCAAAACGAAACAAU
UGCGAGUUGUAAAUGGGAUUCCAACACGAACAAACAUAGGAUGGAUGGU
UAGUUUGAGAUACAGAAAUAAACAUAUCUGCGGAGGAUCAUUGAUAAAG
GAGAGUUGGGUUCUUACUGCACGACAGUGUUUCCCUUCUCGAGACUUGA
AAGAUUAUGAAGCUUGGCUUGGAAUUCAUGAUGUCCACGGAAGAGGAGA
UGAGAAAUGCAAACAGGUUCUCAAUGUUUCCCAGCUGGUAUAUGGCCCU
GAAGGAUCAGAUCUGGUUUUAAUGAAGCUUGCCAGGCCUGCUGUCCUGG
AUGAUUUUGUUAGUACGAUUGAUUUACCUAAUUAUGGAUGCACAAUUCC
 
UGAAAAGACCAGUUGCAGUGUUUAUGGCUGGGGCUACACUGGAUUGAUC
AACUAUGAUGGCCUAUUACGAGUGGCACAUCUCUAUAUAAUGGGAAAUG
AGAAAUGCAGCCAGCAUCAUCGAGGGAAGGUGACUCUGAAUGAGUCUGA
AAUAUGUGCUGGGGCUGAAAAGAUUGGAUCAGGACCAUGUGAGGGGGAU
UAUGGUGGCCCACUUGUUUGUGAGCAACAUAAAAUGAGAAUGGUUCUUG
GUGUCAUUGUUCCUGGUCGUGGAUGUGCCAUUCCAAAUCGUCCUGGUAU
UUUUGUCCGAGUAGCAUAUUAUGCAAAAUGGAUACACAAAAUUAUUUUA
ACAUAUAAGGUACCACAGUCAUAG
SEQ ID NO: 128
> hHGF codon-optimized mRNA sequence 1 (hHGF-OS1) 
AUGUGGGUGACCAAACUGCUCCCCGCUCUGCUGCUGCAGCAUGUGCUGCU
CCACCUGCUGCUGCUGCCAAUCGCCAUCCCCUACGCUGAAGGACAGCGGA
AGCGCAGAAACACAAUUCACGAAUUCAAGAAGUCCGCCAAAACAACACUG
AUCAAGAUCGACCCUGCCCUGAAAAUCAAAACCAAGAAGGUGAACACUGC
CGAUCAGUGCGCCAACAGAUGCACAAGAAACAAAGGCCUGCCCUUCACAU
GCAAGGCCUUUGUGUUCGACAAGGCCAGAAAGCAGUGCCUGUGGUUCCCU
UUCAACAGCAUGAGCUCUGGCGUGAAGAAAGAGUUCGGCCACGAAUUCG
ACCUGUACGAGAACAAGGAUUACAUCAGAAAUUGUAUCAUCGGCAAGGG
CAGAAGCUACAAAGGCACAGUGUCCAUCACCAAAAGCGGCAUUAAGUGUC
AGCCUUGGAGCAGCAUGAUCCCUCACGAGCACAGCUUCCUGCCUAGCUCU
UACAGAGGCAAGGACCUGCAGGAGAAUUACUGCCGGAACCCUAGAGGCG
AGGAAGGCGGCCCUUGGUGCUUUACCAGCAACCCUGAGGUGCGGUACGAG
GUGUGUGACAUCCCCCAGUGCAGCGAAGUGGAAUGCAUGACCUGUAAUG
GCGAGAGCUACAGAGGCCUCAUGGACCAUACAGAGAGCGGAAAGAUUUG
UCAAAGAUGGGAUCACCAGACACCUCACAGACACAAAUUCCUGCCCGAAA
GAUAUCCUGACAAGGGCUUCGACGACAACUACUGCAGAAAUCCUGAUGGC
CAACCCAGACCUUGGUGCUACACCCUGGAUCCUCAUACCAGAUGGGAGUA
CUGUGCCAUCAAGACCUGCGCUGAUAAUACCAUGAACGACACCGAUGUGC
CUCUGGAAACAACCGAGUGCAUCCAGGGACAGGGCGAAGGCUACCGGGGC
ACCGUGAACACCAUCUGGAACGGCAUCCCCUGCCAGAGAUGGGACAGCCA
GUACCCCCACGAACACGAUAUGACCCCUGAGAACUUCAAGUGCAAGGACC
UGAGAGAAAAUUACUGCAGAAACCCAGACGGAAGCGAGUCUCCUUGGUG
UUUUACCACCGACCCCAACAUCCGGGUGGGUUACUGCAGCCAAAUCCCCA
AUUGCGACAUGAGCCACGGCCAGGACUGCUACCGGGGAAACGGCAAAAAC
UACAUGGGCAAUCUGAGCCAGACCCGGAGCGGUCUGACCUGCUCCAUGUG
GGAUAAGAACAUGGAAGAUCUGCACCGGCACAUCUUCUGGGAGCCUGAC
GCUUCUAAGCUGAACGAGAACUACUGUAGGAACCCAGACGACGAUGCCCA
CGGUCCCUGGUGCUACACCGGCAACCCGCUGAUCCCUUGGGACUAUUGUC
CUAUCAGCCGCUGCGAGGGCGAUACAACACCUACCAUCGUGAACCUGGAC
 
CACCCUGUGAUCUCUUGCGCCAAGACCAAGCAGCUGAGAGUGGUGAACGG
CAUCCCAACACGGACCAACAUCGGAUGGAUGGUCUCCCUGAGAUACCGGA
ACAAGCACAUCUGCGGCGGCUCUCUGAUCAAGGAAUCUUGGGUCCUGACC
GCCAGACAGUGCUUCCCCAGCCGGGACCUGAAGGACUAUGAGGCCUGGCU
GGGAAUCCACGACGUGCACGGCAGAGGCGACGAGAAGUGCAAGCAAGUG
CUGAACGUGUCCCAGCUGGUGUACGGCCCUGAGGGAUCUGAUCUGGUCCU
GAUGAAACUGGCCAGACCCGCCGUGCUGGACGACUUUGUGUCCACCAUCG
ACCUGCCUAACUACGGCUGCACCAUCCCUGAAAAGACCAGCUGCAGCGUG
UACGGCUGGGGCUAUACAGGCCUGAUCAACUACGACGGUCUGCUGCGGGU
UGCUCACCUGUACAUCAUGGGCAACGAGAAGUGCAGCCAGCACCACAGAG
GAAAGGUUACACUUAACGAGAGCGAGAUCUGCGCCGGCGCCGAGAAAAU
CGGCUCCGGCCCUUGUGAAGGCGACUACGGAGGCCCUCUGGUGUGCGAAC
AGCACAAGAUGAGAAUGGUGCUGGGCGUGAUCGUGCCCGGAAGAGGCUG
UGCAAUCCCUAAUAGGCCCGGCAUUUUCGUGCGGGUGGCCUACUACGCCA
AGUGGAUCCACAAGAUCAUCCUGACGUACAAGGUGCCUCAGAGCUGA
SEQ ID NO: 129
> hHGF codon-optimized mRNA sequence 2 (hHGF-OS2) 
AUGUGGGUCACCAAACUGCUGCCCGCCCUGCUGCUGCAGCACGUGCUCCU
GCACCUGCUGCUGCUGCCUAUCGCCAUCCCUUACGCCGAAGGCCAGAGAA
AGCGGAGAAAUACCAUUCACGAAUUUAAGAAGAGCGCCAAGACCACACU
GAUCAAGAUCGACCCUGCCCUGAAAAUCAAGACCAAGAAAGUGAACACAG
CCGACCAAUGCGCUAAUAGAUGCACAAGAAACAAAGGCCUGCCUUUCACC
UGCAAGGCCUUCGUGUUCGAUAAGGCCAGAAAGCAGUGCCUGUGGUUCCC
CUUCAACAGCAUGAGCAGCGGCGUGAAGAAAGAAUUCGGUCACGAGUUC
GACCUGUACGAGAAUAAGGACUACAUUAGAAACUGCAUCAUCGGCAAGG
GCCGGAGCUACAAGGGCACCGUGAGCAUCACCAAGUCUGGCAUCAAGUGC
CAGCCAUGGAGUUCCAUGAUCCCUCAUGAGCACAGCUUCCUGCCAAGCUC
UUACCGGGGAAAGGAUCUGCAGGAGAACUAUUGUAGAAAUCCCAGAGGC
GAGGAAGGAGGACCCUGGUGUUUUACCAGCAACCCAGAGGUGCGGUACG
AGGUGUGCGACAUACCCCAGUGCAGCGAGGUGGAAUGCAUGACAUGCAA
CGGCGAGAGCUACCGGGGCCUGAUGGACCACACAGAGAGCGGCAAAAUCU
GCCAAAGAUGGGAUCACCAGACCCCUCAUAGGCACAAGUUCCUGCCCGAG
AGAUACCCUGACAAGGGCUUCGACGACAAUUACUGCCGGAACCCUGAUGG
ACAGCCUAGACCUUGGUGCUACACCCUGGACCCUCACACCAGAUGGGAAU
ACUGCGCCAUCAAGACAUGUGCCGAUAACACCAUGAACGACACCGAUGUG
CCACUCGAGACAACUGAGUGUAUCCAGGGCCAGGGCGAGGGCUACAGGGG
CACCGUGAACACCAUCUGGAACGGCAUCCCUUGUCAGCGGUGGGACAGCC
AAUACCCCCACGAACAUGAUAUGACCCCAGAAAACUUCAAGUGCAAAGAU
CUGCGGGAAAACUACUGUAGAAACCCUGACGGCUCCGAGAGCCCUUGGUG
CUUCACCACCGACCCUAACAUCAGGGUGGGCUACUGUAGCCAGAUCCCCA
 
ACUGCGACAUGAGCCACGGUCAGGACUGCUACAGAGGAAACGGCAAGAA
UUACAUGGGCAACCUGAGCCAGACCAGAAGCGGCCUGACCUGCAGCAUGU
GGGACAAAAACAUGGAAGAUCUGCACCGGCACAUCUUUUGGGAGCCUGA
CGCCAGCAAGCUGAAUGAGAACUACUGUCGGAACCCUGACGACGAUGCUC
ACGGCCCUUGGUGCUACACAGGCAACCCUCUGAUCCCUUGGGACUAUUGU
CCUAUUAGCAGGUGUGAAGGCGACACGACCCCUACAAUCGUGAACCUGGA
UCACCCAGUGAUCAGCUGUGCUAAAACAAAGCAACUGAGAGUGGUGAAU
GGUAUCCCGACCCGGACCAACAUCGGAUGGAUGGUCAGCCUGCGGUACAG
AAACAAGCACAUCUGCGGCGGUAGCCUGAUCAAGGAAUCCUGGGUGUUG
ACAGCCAGACAGUGUUUUCCCAGCAGAGAUCUGAAGGACUACGAGGCCUG
GCUGGGCAUCCACGACGUGCACGGCAGAGGCGACGAGAAGUGCAAGCAGG
UUCUGAACGUGUCCCAGCUGGUGUACGGCCCCGAGGGCUCUGAUCUGGUU
CUGAUGAAGCUUGCUAGACCCGCUGUGCUGGAUGACUUCGUGUCCACCAU
CGACCUGCCAAAUUACGGAUGUACAAUUCCUGAAAAGACCUCCUGUUCUG
UGUACGGCUGGGGCUACACCGGACUCAUCAACUAUGACGGCCUGCUGAGA
GUGGCCCACCUGUAUAUCAUGGGCAACGAGAAGUGCAGCCAGCACCAUAG
AGGCAAGGUGACCCUGAACGAGUCUGAAAUCUGCGCCGGAGCCGAGAAA
AUCGGAUCUGGCCCCUGCGAAGGAGAUUACGGCGGCCCUCUGGUCUGCGA
GCAGCACAAGAUGAGAAUGGUGCUGGGCGUGAUCGUGCCCGGCAGAGGC
UGCGCCAUCCCUAACCGGCCUGGAAUCUUCGUGCGCGUGGCCUAUUACGC
CAAAUGGAUCCACAAAAUCAUCCUGACAUACAAGGUCCCACAGUCUUGA
SEQ ID NO: 130
> hHGF codon-optimized mRNA sequence 3 (hHGF-OS3)

AUGUGGGUGACCAAGCUGCUGCCCGCCCUGCUCCUGCAGCACGUGCUGCU
GCACCUGCUCCUGCUGCCCAUCGCCAUCCCCUACGCCGAGGGGCAGAGAA
AGAGAAGAAACACCAUCCACGAGUUCAAGAAGAGCGCCAAGACCACCCUG
AUCAAGAUCGACCCCGCCCUGAAGAUCAAGACCAAGAAGGUGAACACCGC
CGAUCAGUGCGCCAACAGAUGCACAAGAAACAAGGGCCUGCCCUUCACCU
GCAAGGCCUUCGUGUUCGACAAGGCUAGAAAGCAGUGCCUGUGGUUCCCC
UUCAACAGCAUGAGCAGCGGCGUGAAGAAGGAGUUCGGCCACGAGUUCG
ACCUGUACGAGAACAAGGACUACAUCAGAAACUGCAUCAUCGGCAAGGGC
AGAAGCUACAAGGGCACCGUGAGCAUCACCAAGAGCGGCAUCAAGUGUCA
GCCCUGGAGCAGCAUGAUCCCCCAUGAGCACAGCUUCCUGCCUAGCAGCU
ACAGAGGGAAGGACCUGCAAGAGAACUACUGCAGAAACCCUAGAGGCGA
GGAGGGCGGCCCUUGGUGUUUCACAAGCAAUCCCGAGGUGAGAUACGAG
GUGUGCGACAUCCCUCAGUGCAGCGAGGUGGAGUGCAUGACCUGCAACGG
CGAAAGCUAUAGAGGCCUGAUGGACCACACCGAGAGCGGCAAGAUUUGU
CAGCGGUGGGAUCACCAAACCCCCCACAGACACAAGUUCCUGCCCGAGAG
AUACCCCGACAAGGGCUUCGACGACAAUUACUGUAGAAACCCCGACGGGC
AGCCUAGACCCUGGUGUUACACCCUGGACCCCCACACAAGAUGGGAGUAC

UGCGCCAUCAAGACCUGCGCCGACAACACCAUGAACGACACCGACGUGCC
CCUGGAGACCACCGAGUGCAUCCAAGGCCAAGGCGAGGGCUACAGAGGCA
CCGUGAACACCAUCUGGAACGGCAUUCCCUGUCAGAGAUGGGACAGCCAA
UACCCCCAUGAACACGAUAUGACCCCCGAGAACUUCAAGUGCAAGGACCU
GAGAGAGAACUACUGCCGGAACCCCGAUGGCAGCGAAUCCCCUUGGUGCU
UCACCACCGACCCCAACAUCAGAGUGGGCUACUGCAGCCAAAUCCCCAAC
UGCGACAUGAGCCACGGCCAAGACUGUUACAGAGGGAACGGCAAGAACU
ACAUGGGCAACCUGAGCCAAACAAGAAGCGGCCUGACCUGCAGCAUGUGG
GACAAGAACAUGGAGGACCUGCACAGACACAUCUUCUGGGAGCCCGACGC
UAGCAAGCUGAACGAGAACUACUGUCGGAACCCCGACGACGAUGCCCACG
GCCCCUGGUGCUACACCGGCAACCCCCUGAUCCCCUGGGACUACUGCCCC
AUCAGCAGAUGCGAGGGCGACACCACCCCCACCAUCGUGAACCUGGACCA
CCCCGUGAUCAGCUGUGCCAAGACCAAGCAACUGAGAGUGGUGAACGGCA
UCCCCACAAGAACCAACAUUGGCUGGAUGGUGAGCCUGAGAUACAGAAAC
AAGCACAUCUGCGGCGGCAGCCUGAUCAAGGAGAGCUGGGUGCUGACCGC
UAGACAGUGCUUCCCUAGCAGAGACCUGAAGGACUACGAGGCCUGGCUGG
GCAUCCACGACGUGCACGGCAGAGGCGACGAGAAGUGUAAACAAGUGCU
GAACGUGAGCCAACUGGUGUACGGCCCCGAGGGCAGCGACCUGGUGCUGA
UGAAGCUGGCUAGACCCGCCGUGCUGGACGACUUCGUGAGCACCAUCGAC
CUGCCCAACUACGGCUGCACCAUCCCCGAGAAGACAAGCUGCAGCGUGUA
CGGCUGGGGCUACACCGGCCUGAUCAACUACGACGGCCUGCUGAGAGUGG
CCCACCUGUACAUCAUGGGCAACGAGAAGUGCAGCCAACACCACCGGGGC
AAAGUGACCCUGAACGAGAGCGAGAUCUGCGCCGGGGCUGAGAAAAUCG
GCAGCGGCCCCUGCGAGGGGGACUACGGGGGCCCCCUGGUGUGCGAGCAG
CACAAGAUGAGAAUGGUGCUGGGCGUGAUCGUGCCCGGCAGAGGCUGCG
CCAUCCCCAACAGACCCGGCAUCUUCGUGAGAGUGGCCUACUACGCCAAG
UGGAUCCACAAGAUCAUCCUGACCUACAAGGUGCCUCAGAGCUGA
SEQ ID NO: 131.

## Claims

1. A nucleic acid construct, comprising:
(a) an open reading frame (ORF), and
(b) an untranslated region element (UTR), the UTR comprising the following sequence:
any one of SEQ ID NOs: 12 and 26 or a sequence having at least 90% identity thereto,
any one of SEQ ID NOs: 1 and 15 or a sequence having at least 90% identity thereto,
any one of SEQ ID NOs: 4-5 and 18-19 or a sequence having at least 90% identity thereto,
any one of SEQ ID NOs: 6-8 and 20-22 or a sequence having at least 90% identity thereto,
any one of SEQ ID NOs: 9-11 and 23-25 or a sequence having at least 90% identity thereto,
any one of SEQ ID NOs: 13 and 27 or a sequence having at least 90% identity thereto,
any one of SEQ ID NOs: 14 and 28 or a sequence having at least 90% identity thereto,
any one of SEQ ID NOs: 29 and 30 or a sequence having at least 90% identity thereto,
any one of SEQ ID NOs: 35-37 or a sequence having at least 90% identity thereto,
any one of SEQ ID NOs: 42 and 43 or a sequence having at least 90% identity thereto,
any one of SEQ ID NOs: 46 and 47 or a sequence having at least 90% identity thereto, or
any one of SEQ ID NOs: 31-34, 38-41, and 44-45 or a sequence having at least 90% identity thereto.

2. The nucleic acid construct according to claim 1, wherein (b) comprises a combination of a 3' untranslated region element (3'UTR) and a 5' untranslated region element (5'UTR) selected from the group consisting of any one of the following:
1) the 3'UTR comprises the sequence set forth in SEQ ID NO: 1 or a sequence having at least 90% identity thereto, and the 5'UTR comprises the sequence set forth in any one of SEQ ID NOs: 15-47 or a sequence having at least 90% identity to any one of SEQ ID NOs: 15-47;
2) the 3'UTR comprises the sequence set forth in SEQ ID NO: 2 or a sequence having at least 90% identity thereto, and the 5'UTR comprises the sequence set forth in any one of SEQ ID NOs: 15-47 or a sequence having at least 90% identity to any one of SEQ ID NOs: 15-47;
3) the 3'UTR comprises the sequence set forth in SEQ ID NO: 3 or a sequence having at least 90% identity thereto, and the 5'UTR comprises the sequence set forth in any one of SEQ ID NOs: 15-47 or a sequence having at least 90% identity to any one of SEQ ID NOs: 15-47;
4) the 3'UTR comprises the sequence set forth in SEQ ID NO: 4 or a sequence having at least 90% identity thereto, and the 5'UTR comprises the sequence set forth in any one of SEQ ID NOs: 15-47 or a sequence having at least 90% identity to any one of SEQ ID NOs: 15-47;
5) the 3'UTR comprises the sequence set forth in SEQ ID NO: 5 or a sequence having at least 90% identity thereto, and the 5'UTR comprises the sequence set forth in any one of SEQ ID NOs: 15-47 or a sequence having at least 90% identity to any one of SEQ ID NOs: 15-47;
6) the 3'UTR comprises the sequence set forth in SEQ ID NO: 6 or a sequence having at least 90% identity thereto, and the 5'UTR comprises the sequence set forth in any one of SEQ ID NOs: 15-47 or a sequence having at least 90% identity to any one of SEQ ID NOs: 15-47;
7) the 3'UTR comprises the sequence set forth in SEQ ID NO: 7 or a sequence having at least 90% identity thereto, and the 5'UTR comprises the sequence set forth in any one of SEQ ID NOs: 15-47 or a sequence having at least 90% identity to any one of SEQ ID NOs: 15-47;
8) the 3'UTR comprises the sequence set forth in SEQ ID NO: 8 or a sequence having at least 90% identity thereto, and the 5'UTR comprises the sequence set forth in any one of SEQ ID NOs: 15-47 or a sequence having at least 90% identity to any one of SEQ ID NOs: 15-47;
9) the 3'UTR comprises the sequence set forth in SEQ ID NO: 9 or a sequence having at least 90% identity thereto, and the 5'UTR comprises the sequence set forth in any one of SEQ ID NOs: 15-47 or a sequence having at least 90% identity to any one of SEQ ID NOs: 15-47;
10) the 3'UTR comprises the sequence set forth in SEQ ID NO: 10 or a sequence having at least 90% identity thereto, and the 5'UTR comprises the sequence set forth in any one of SEQ ID NOs: 15-47 or a sequence having at least 90% identity to any one of SEQ ID NOs: 15-47;
11) the 3'UTR comprises the sequence set forth in SEQ ID NO: 11 or a sequence having at least 90% identity thereto, and the 5'UTR comprises the sequence set forth in any one of SEQ ID NOs: 15-47 or a sequence having at least 90% identity to any one of SEQ ID NOs: 15-47;
12) the 3'UTR comprises the sequence set forth in SEQ ID NO: 12 or a sequence having at least 90% identity thereto, and the 5'UTR comprises the sequence set forth in any one of SEQ ID NOs: 15-47 or a sequence having at least 90% identity to any one of SEQ ID NOs: 15-47;
13) the 3'UTR comprises the sequence set forth in SEQ ID NO: 13 or a sequence having at least 90% identity thereto, and the 5'UTR comprises the sequence set forth in any one of SEQ ID NOs: 15-47 or a sequence having at least 90% identity to any one of SEQ ID NOs: 15-47;
14) the 3'UTR comprises the sequence set forth in SEQ ID NO: 14 or a sequence having at least 90% identity thereto, and the 5'UTR comprises the sequence set forth in any one of SEQ ID NOs: 15-47 or a sequence having at least 90% identity to any one of SEQ ID NOs: 15-47;
15) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 15 or a sequence having at least 90% identity thereto;
16) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 16 or a sequence having at least 90% identity thereto;
17) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 17 or a sequence having at least 90% identity thereto;
18) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 18 or a sequence having at least 90% identity thereto;
19) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 19 or a sequence having at least 90% identity thereto;
20) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 20 or a sequence having at least 90% identity thereto;
21) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 21 or a sequence having at least 90% identity thereto;
22) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 22 or a sequence having at least 90% identity thereto;
23) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 23 or a sequence having at least 90% identity thereto;
24) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 24 or a sequence having at least 90% identity thereto;
25) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 25 or a sequence having at least 90% identity thereto;
26) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 26 or a sequence having at least 90% identity thereto;
27) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 27 or a sequence having at least 90% identity thereto;
28) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 28 or a sequence having at least 90% identity thereto;
29) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 29 or a sequence having at least 90% identity thereto;
30) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 30 or a sequence having at least 90% identity thereto;
31) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 31 or a sequence having at least 90% identity thereto;
32) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 32 or a sequence having at least 90% identity thereto;
33) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 33 or a sequence having at least 90% identity thereto;
34) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 34 or a sequence having at least 90% or 95% identity thereto;
35) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 35 or a sequence having at least 90% identity thereto;
36) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 36 or a sequence having at least 90% identity thereto;
37) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 37 or a sequence having at least 90% identity thereto;
38) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 38 or a sequence having at least 90% identity thereto;
39) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 39 or a sequence having at least 90% identity thereto;
40) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 40 or a sequence having at least 90% identity thereto;
41) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 41 or a sequence having at least 90% identity thereto;
42) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 42 or a sequence having at least 90% identity thereto;
43) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 43 or a sequence having at least 90% identity thereto;
44) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 44 or a sequence having at least 90% identity thereto;
45) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 45 or a sequence having at least 90% identity to any one of SEQ ID NO: 45;
46) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 46 or a sequence having at least 90% to any one of SEQ ID NO: 46; or
47) the 3'UTR comprises the sequence set forth in any one of SEQ ID NOs: 1-14 or a sequence having at least 90% identity to any one of SEQ ID NOs: 1-14, and the 5'UTR comprises the sequence set forth in SEQ ID NO: 47 or a sequence having at least 90% identity to any one of SEQ ID NO: 47.

3. The nucleic acid construct according to claim 1, wherein:
the 3'UTR comprises the sequence set forth in any one of SEQ ID NOs: 12-14 or a sequence having at least 90% identity to any one of SEQ ID NOs: 12-14, and the 5'UTR comprises the sequence set forth in any one of SEQ ID NOs: 15, 29, 30, and 32 or a sequence having at least 90% identity to any one of SEQ ID NOs: 15, 29, 30, and 32; preferably,
the 3'UTR comprises the sequence set forth in SEQ ID NO: 12 or a sequence having at least 90% identity thereto, and the 5'UTR comprises the sequence set forth in any one of SEQ ID NOs: 15, 29, 30, and 32 or a sequence having at least 90% identity thereto;
the 3'UTR comprises the sequence set forth in SEQ ID NO: 13 or a sequence having at least 90% identity thereto, and the 5'UTR comprises the sequence set forth in any one of SEQ ID NOs: 15, 29, 30, and 32 or a sequence having at least 90% identity thereto;
the 3'UTR comprises the sequence set forth in SEQ ID NO: 14 or a sequence having at least 90% identity thereto, and the 5'UTR comprises the sequence set forth in any one of SEQ ID NOs: 15, 29, 30, and 32 or a sequence having at least 90% identity thereto;
the 3'UTR comprises the sequence set forth in any one of SEQ ID NOs: 12-14 or a sequence having at least 90% identity thereto, and the 5'UTR comprises the sequence set forth in SEQ ID NO: 15 or a sequence having at least 90% identity thereto;
the 3'UTR comprises the sequence set forth in any one of SEQ ID NOs: 12-14 or a sequence having at least 90% identity thereto, and the 5'UTR comprises the sequence set forth in SEQ ID NO: 29 or a sequence having at least 90% identity thereto;
the 3'UTR comprises the sequence set forth in any one of SEQ ID NOs: 12-14 or a sequence having at least 90% identity thereto, and the 5'UTR comprises the sequence set forth in SEQ ID NO: 30 or a sequence having at least 90% identity thereto; or
the 3'UTR comprises the sequence set forth in any one of SEQ ID NOs: 12-14 or a sequence having at least 90% identity thereto, and the 5'UTR comprises the sequence set forth in SEQ ID NO: 32 or a sequence having at least 90% identity thereto.

4. The nucleic acid construct according to any one of claims 1-3, further comprising:
(c) a polyadenylic acid (poly-A) tail,
wherein preferably, the poly-A tail is selected from the group consisting of A120, A30L70, HGH polyA, SV40 polyA, BGH polyA, rbGlob polyA, and SV40late polyA; preferably, the poly-A tail is selected from the group consisting of A120 and A30L70, wherein the A120 comprises 120 adenine nucleotides, and the A30L70 comprises the sequence set forth in SEQ ID NO: 52 or a sequence having at least 90% identity thereto.

5. The nucleic acid construct according to any one of claims 1-4, wherein the ORF encodes hepatocyte growth factor (HGF), an antibody or an antigen-binding fragment thereof, preferably human hepatocyte growth factor (hHGF), an anti-PD-1 antibody or an antigen-binding fragment thereof.

6. The nucleic acid construct according to claim 5, wherein the ORF comprises any one selected from the group consisting of the following 1)-2):
1) a polynucleotide sequence encoding the amino acid sequence set forth in SEQ ID NO: 109; and
2) the polynucleotide sequence set forth in any one of SEQ ID NOs: 110-113 or a polynucleotide sequence having at least 90% identity thereto;
or, the ORF comprises any one selected from the group consisting of the following 1)-3):
1) a polynucleotide sequence encoding a heavy chain amino acid sequence set forth in SEQ ID NO: 117, and/or a polynucleotide sequence encoding a light chain amino acid sequence set forth in SEQ ID NO: 118;
2) a polynucleotide sequence encoding a HCDR1, a HCDR2, and a HCDR3 in the heavy chain amino acid sequence set forth in SEQ ID NO: 117, and a polynucleotide sequence encoding a LCDR1, a LCDR2, and a LCDR3 in the light chain amino acid sequence set forth in SEQ ID NO: 118, the CDRs being defined according to the Kabat, IMGT, Chothia, AbM, or Contact numbering scheme, preferably the Kabat numbering scheme; and
3) the polynucleotide sequence set forth in SEQ ID NO: 119 or a polynucleotide sequence having at least 90% identity thereto, and/or the polynucleotide sequence set forth in SEQ ID NO: 120 or a polynucleotide sequence having at least 90% identity thereto.

7. The nucleic acid construct according to any one of claims 5-6, comprising the sequence set forth in any one of SEQ ID NOs: 115, 116, and 127 or a sequence having at least 90% identity thereto, or comprising the sequence set forth in SEQ ID NO: 124 or a sequence having at least 90% identity thereto and/or the sequence set forth in SEQ ID NO: 125 or a sequence having at least 90% identity thereto.

8. An RNA molecule, comprising:
(a) an open reading frame (ORF), and
(b) an untranslated region element (UTR), the UTR comprising the following sequence:
any one of SEQ ID NOs: 69 and 83 or a sequence having at least 90% identity thereto,
any one of SEQ ID NOs: 58 and 72 or a sequence having at least 90% identity thereto,
any one of SEQ ID NOs: 59-60 and 73-74 or a sequence having at least 90% identity thereto,
any one of SEQ ID NOs: 61-62 and 75-76 or a sequence having at least 90% identity thereto,
any one of SEQ ID NOs: 63-65 and 77-79 or a sequence having at least 90% identity thereto,
any one of SEQ ID NOs: 66-68 and 80-82 or a sequence having at least 90% identity thereto,
any one of SEQ ID NOs: 70 and 84 or a sequence having at least 90% identity thereto,
any one of SEQ ID NOs: 71 and 85 or a sequence having at least 90% identity thereto,
any one of SEQ ID NOs: 86 and 87 or a sequence having at least 90% identity thereto,
any one of SEQ ID NOs: 92-94 or a sequence having at least 90% identity thereto,
any one of SEQ ID NOs: 99 and 100 or a sequence having at least 90% identity thereto,
any one of SEQ ID NOs: 103 and 104 or a sequence having at least 90% identity thereto, or
any one of SEQ ID NOs: 88-91, 95-98, and 101-102 or a sequence having at least 90% identity thereto.

9. The RNA molecule according to claim 8, wherein (b) comprises a combination of a 3' untranslated region element (3'UTR) and a 5' untranslated region element (5'UTR) selected from the group consisting of any one of the following:
1) the 3'UTR comprises the sequence set forth in SEQ ID NO: 58 or a sequence having at least 90% identity thereto, and the 5'UTR comprises the sequences set forth in SEQ ID NOs: 72-104 or a sequence having at least 90% identity to any one of SEQ ID NOs: 72-104;
2) the 3'UTR comprises the sequence set forth in SEQ ID NO: 59 or a sequence having at least 90% identity thereto, and the 5'UTR comprises the sequences set forth in SEQ ID NOs: 72-104 or a sequence having at least 90% identity to any one of SEQ ID NOs: 72-104;
3) the 3'UTR comprises the sequence set forth in SEQ ID NO: 60 or a sequence having at least 90% identity thereto, and the 5'UTR comprises the sequences set forth in SEQ ID NOs: 72-104 or a sequence having at least 90% identity to any one of SEQ ID NOs: 72-104;
4) the 3'UTR comprises the sequence set forth in SEQ ID NO: 61 or a sequence having at least 90% identity thereto, and the 5'UTR comprises the sequences set forth in SEQ ID NOs: 72-104 or a sequence having at least 90% identity to any one of SEQ ID NOs: 72-104;
5) the 3'UTR comprises the sequence set forth in SEQ ID NO: 62 or a sequence having at least 90% identity thereto, and the 5'UTR comprises the sequences set forth in SEQ ID NOs: 72-104 or a sequence having at least 90% identity to any one of SEQ ID NOs: 72-104;
6) the 3'UTR comprises the sequence set forth in SEQ ID NO: 63 or a sequence having at least 90% identity thereto, and the 5'UTR comprises the sequences set forth in SEQ ID NOs: 72-104 or a sequence having at least 90% identity to any one of SEQ ID NOs: 72-104;
7) the 3'UTR comprises the sequence set forth in SEQ ID NO: 64 or a sequence having at least 90% identity thereto, and the 5'UTR comprises the sequences set forth in SEQ ID NOs: 72-104 or a sequence having at least 90% identity to any one of SEQ ID NOs: 72-104;
8) the 3'UTR comprises the sequence set forth in SEQ ID NO: 65 or a sequence having at least 90% identity thereto, and the 5'UTR comprises the sequences set forth in SEQ ID NOs: 72-104 or a sequence having at least 90% identity to any one of SEQ ID NOs: 72-104;
9) the 3'UTR comprises the sequence set forth in SEQ ID NO: 66 or a sequence having at least 90% identity thereto, and the 5'UTR comprises the sequences set forth in SEQ ID NOs: 72-104 or a sequence having at least 90% identity to any one of SEQ ID NOs: 72-104;
10) the 3'UTR comprises the sequence set forth in SEQ ID NO: 67 or a sequence having at least 90% identity thereto, and the 5'UTR comprises the sequence set forth in any one of SEQ ID NOs: 72-104 or a sequence having at least 90% identity to any one of SEQ ID NOs: 72-104;
11) the 3'UTR comprises the sequence set forth in SEQ ID NO: 68 or a sequence having at least 90% identity thereto, and the 5'UTR comprises the sequences set forth in SEQ ID NOs: 72-104 or a sequence having at least 90% identity to any one of SEQ ID NOs: 72-104;
12) the 3'UTR comprises the sequence set forth in SEQ ID NO: 69 or a sequence having at least 90% identity thereto, and the 5'UTR comprises the sequence set forth in any one of SEQ ID NOs: 72-104 or a sequence having at least 90% identity to any one of SEQ ID NOs: 72-104;
13) the 3'UTR comprises the sequence set forth in SEQ ID NO: 70 or a sequence having at least 90% identity thereto, and the 5'UTR comprises the sequence set forth in any one of SEQ ID NOs: 72-104 or a sequence having at least 90% identity to any one of SEQ ID NOs: 72-104;
14) the 3'UTR comprises the sequence set forth in SEQ ID NO: 71 or a sequence having at least 90% identity thereto, and the 5'UTR comprises the sequence set forth in any one of SEQ ID NOs: 72-104 or a sequence having at least 90% identity to any one of SEQ ID NOs: 72-104;
15) the 3'UTR comprises the sequence set forth in any one of SEQ ID NOs: 58-71 or a sequence having at least 90% identity thereto, and the 5'UTR comprises the sequence set forth in SEQ ID NO: 72 or a sequence having at least 90% identity thereto;
16) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 73 or a sequence having at least 90% identity thereto;
17) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 74 or a sequence having at least 90% identity thereto;
18) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 75 or a sequence having at least 90% identity thereto;
19) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 76 or a sequence having at least 90% identity thereto;
20) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 77 or a sequence having at least 90% identity thereto;
21) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 78 or a sequence having at least 90% identity thereto;
22) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 79 or a sequence having at least 90% identity thereto;
23) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 80 or a sequence having at least 90% identity thereto;
24) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 81 or a sequence having at least 90% identity thereto;
25) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 82 or a sequence having at least 90% identity thereto;
26) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 83 or a sequence having at least 90% identity thereto;
27) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 84 or a sequence having at least 90% identity thereto;
28) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 85 or a sequence having at least 90% identity thereto;
29) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 86 or a sequence having at least 90% identity thereto;
30) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 87 or a sequence having at least 90% identity thereto;
31) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 88 or a sequence having at least 90% identity thereto;
32) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 89 or a sequence having at least 90% identity thereto;
33) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 90 or a sequence having at least 90% identity thereto;
34) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 91 or a sequence having at least 90% or 95% identity to any one of SEQ ID NO: 91;
35) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 92 or a sequence having at least 90% identity thereto;
36) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 93 or a sequence having at least 90% identity thereto;
37) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 94 or a sequence having at least 90% identity thereto;
38) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 95 or a sequence having at least 90% identity thereto;
39) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 96 or a sequence having at least 90% identity thereto;
40) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 97 or a sequence having at least 90% identity thereto;
41) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 98 or a sequence having at least 90% identity thereto;
42) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 99 or a sequence having at least 90% identity thereto;
43) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 100 or a sequence having at least 90% identity thereto;
44) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 101 or a sequence having at least 90% identity thereto;
45) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 102 or a sequence having at least 90% identity thereto;
46) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 103 or a sequence having at least 90% identity thereto; or
47) the 3'UTR comprises the sequence set forth in any one of SEQ ID NOs: 58-71 or a sequence having at least 90% identity to any one of SEQ ID NOs: 58-71, and the 5'UTR comprises the sequence set forth in SEQ ID NO: 104 or a sequence having at least 90% identity thereto.

10. The RNA molecule according to claim 8, wherein:
the 3'UTR comprises the sequence set forth in any one of SEQ ID NOs: 69-71 or a sequence having at least 90% identity thereto, and the 5'UTR comprises the sequence set forth in any one of SEQ ID NOs: 72, 86, 87, and 89 or a sequence having at least 90% identity to any one of SEQ ID NOs: 72, 86, 87, and 89;
preferably,
the 3'UTR comprises the sequence set forth in SEQ ID NO: 69 or a sequence having at least 90% identity thereto, and the 5'UTR comprises the sequence set forth in any one of SEQ ID NOs: 72, 86, 87, and 89 or a sequence having at least 90% identity thereto;
the 3'UTR comprises the sequence set forth in SEQ ID NO: 70 or a sequence having at least 90% identity thereto, and the 5'UTR comprises the sequence set forth in any one of SEQ ID NOs: 72, 86, 87, and 89 or a sequence having at least 90% identity thereto;
the 3'UTR comprises the sequence set forth in SEQ ID NO: 71 or a sequence having at least 90% identity thereto, and the 5'UTR comprises the sequence set forth in any one of SEQ ID NOs: 72, 86, 87, and 89 or a sequence having at least 90% identity thereto;
the 3'UTR comprises the sequence set forth in any one of SEQ ID NOs: 69-71 or a sequence having at least 90% identity thereto, and the 5'UTR comprises the sequence set forth in SEQ ID NO: 72 or a sequence having at least 90% identity thereto;
the 3'UTR comprises the sequence set forth in any one of SEQ ID NOs: 69-71 or a sequence having at least 90% identity thereto, and the 5'UTR comprises the sequence set forth in SEQ ID NO: 86 or a sequence having at least 90% identity thereto;
the 3'UTR comprises the sequence set forth in any one of SEQ ID NOs: 69-71 or a sequence having at least 90% identity thereto, and the 5'UTR comprises the sequence set forth in SEQ ID NO: 87 or a sequence having at least 90% identity thereto; or
the 3'UTR comprises the sequence set forth in any one of SEQ ID NOs: 69-71 or a sequence having at least 90% identity thereto, and the 5'UTR comprises the sequence set forth in SEQ ID NO: 89 or a sequence having at least 90% identity thereto.

11. The RNA molecule according to any one of claims 8-10, further comprising:
(c) a polyadenylic acid (poly-A) tail,
wherein preferably, the poly-A tail is selected from the group consisting of A120, A30L70, HGH polyA, SV40 polyA, BGH polyA, rbGlob polyA, and SV40late polyA;
preferably, the poly-A tail is selected from the group consisting of A120 and A30L70, wherein the A120 comprises 120 adenine nucleotides, and the A30L70 comprises the sequence set forth in SEQ ID NO: 52 or a sequence having at least 90% or 95% identity thereto.

12. The RNA molecule according to any one of claims 8-11, wherein the ORF encodes hepatocyte growth factor (HGF), an antibody or an antigen-binding fragment thereof, preferably human hepatocyte growth factor (hHGF), an anti-PD-1 antibody or an antigen-binding fragment thereof;
preferably, the ORF comprises any one selected from the group consisting of the following 1)-2):
1) a nucleic acid sequence encoding the amino acid sequence set forth in SEQ ID NO: 109; and
2) an RNA sequence set forth in any one of SEQ ID NOs: 128-131 or an RNA sequence having at least 90%, preferably at least 95%, identity thereto; or,
the ORF comprises any one selected from the group consisting of the following 1)-3):
1) a nucleic acid sequence encoding a heavy chain amino acid sequence set forth in SEQ ID NO: 117, and/or a nucleic acid sequence encoding a light chain amino acid sequence set forth in SEQ ID NO: 118;
2) a nucleic acid sequence encoding a HCDR1, a HCDR2, and a HCDR3 in the heavy chain amino acid sequence set forth in SEQ ID NO: 117, and a nucleic acid sequence encoding a LCDR1, a LCDR2, and a LCDR3 in the light chain amino acid sequence set forth in SEQ ID NO: 118, the CDRs being defined according to the Kabat, IMGT, Chothia, AbM, or Contact numbering scheme, preferably the Kabat numbering scheme; and
3) an RNA sequence set forth in SEQ ID NO: 121 or an RNA sequence having at least 90% identity thereto, and/or an RNA sequence set forth in SEQ ID NO: 122 or an RNA sequence having at least 90% identity thereto.

13. The RNA molecule according to any one of claims 8-12, comprising:
(d) a 5' cap structure (5'Cap),
wherein preferably, the 5'Cap is selected from the group consisting of Cap0, Cap1, Cap2, Cap3, Cap4, ARCA, modified ARCA, inosine, N1-methyl-guanosine, 2'-fluoro-guanosine, 7-deaza-guanosine, 8-oxo-guanosine, 2-amino-guanosine, LNA-guanosine, and 2-azido-guanosine;
more preferably, the 5'Cap is selected from the group consisting of ARCA,
3'-O-Me-m⁷G(5')ppp(5')G, m⁷G(5')ppp(5')(2'OMeA)pU, m⁷Gppp(A2'O-MOE)pG, m⁷G(5')ppp(5')(2'OMeA)pG, m⁷G(5')ppp(5')(2'OMeG)pG, m⁷(3'OMeG)(5')ppp(5')(2'OMeG)pG, and m⁷(3'OMeG)(5')ppp(5')(2'OMeA)pG.

14. The RNA molecule according to any one of claims 8-13, comprising one or more modifications, wherein preferably the modifications include backbone modifications, sugar modifications, base modifications, and/or lipid modifications; more preferably, the base modifications are uracil modifications.

15. The RNA molecule according to any one of claims 8-14, wherein the RNA molecule is used for increasing an expression level of a target protein expressed by the ORF.

16. An isolated polynucleotide, comprising (a) an open reading frame (ORF), the ORF comprising the sequence set forth in any one of SEQ ID NOs: 111-113 and 129-131 or a sequence having at least 90% identity thereto,
wherein preferably, the polynucleotide comprises any one of the following (b)-(d) or any combination thereof:
(b) a 5'UTR and/or a 3'UTR,
(c) a poly-A tail, and
(d) a 5'Cap;
preferably, the poly-A tail is selected from the group consisting of A120, A30L70, HGH polyA, SV40 polyA, BGH polyA, rbGlob polyA, and SV40late polyA; more preferably, the poly-A tail is A120 or A30L70;
preferably, the 5'Cap is selected from the group consisting of ARCA,
3'-O-Me-m⁷G(5')ppp(5')G, m⁷G(5')ppp(5')(2'OMeA)pU, m⁷Gppp(A2'O-MOE)pG, m⁷G(5')ppp(5')(2'OMeA)pG, m⁷G(5')ppp(5')(2'OMeG)pG, m⁷(3'OMeG)(5')ppp(5')(2'OMeG)pG, and m⁷(3'OMeG)(5')ppp(5')(2'OMeA)pG;
preferably, the polynucleotide comprises one or more modifications selected from the group consisting of backbone modifications, sugar modifications, base modifications, and/or lipid modifications, wherein the base modifications are preferably pseudouridine modifications.

17. A nucleic acid construct, comprising:
(a) an open reading frame (ORF), and
(b) an untranslated region element (UTR) derived from a UTR of CTSB, FAM166A, NDUFB9, ACTG1, CHCHD10, NDUFA11, ATP6V0B, ATP6V0E1, CFL1, COX4I1, CTSB, SLC38A2, NDUFV3, PRDX5, GUK1, IAH1, ABHD16A, SLC25A39, ATPIF1, ANAPC11, CCDC12, MRPL14, or APOA1BP, the UTR being a 3' untranslated region element (3'UTR) or a 5' untranslated region element (5'UTR),
wherein preferably, the UTR comprises the sequence set forth in any one of SEQ ID NOs: 1-47 or a sequence having at least 90% identity thereto.

18. An RNA molecule, comprising:
(a) an open reading frame (ORF), and
(b) an untranslated region element (UTR) derived from a UTR of CTSB, FAM166A, NDUFB9, ACTG1, CHCHD10, NDUFA11, ATP6V0B, ATP6V0E1, CFL1, COX4I1, CTSB, SLC38A2, NDUFV3, PRDX5, GUK1, IAH1, ABHD16A, SLC25A39, ATPIF1, ANAPC11, CCDC12, MRPL14, or APOA1BP, the UTR being a 3' untranslated region element (3'UTR) or a 5' untranslated region element (5'UTR),
wherein preferably, the UTR comprises the sequence set forth in any one of SEQ ID NOs: 58-104 or a sequence having at least 90% identity thereto.

19. A vector, comprising the nucleic acid construct according to any one of claims 1-7 and 17, the RNA molecule according to any one of claims 8-15 and 18, or the polynucleotide according to claim 16.

20. A host cell, comprising the vector according to claim 19.

21. A preparation method for the nucleic acid construct according to any one of claims 1-7 and 17, comprising culturing the host cell according to claim 20 and collecting a produced nucleic acid construct from the culture.

22. A preparation method for the RNA molecule according to any one of claims 8-15 and 18, comprising reverse-transcribing the nucleic acid construct according to any one of claims 1-7 and 17 or the vector according to claim 19 to obtain the RNA molecule, wherein preferably, the method further comprises adding a 5'Cap to the 5' end of the RNA molecule.

23. A delivery vehicle, comprising the nucleic acid construct according to any one of claims 1-7 and 17, the RNA molecule according to any one of claims 8-15 and 18, the polynucleotide according to claim 16, or the vector according to claim 19, wherein the delivery vehicle is preferably a cationic lipid delivery particle or a lipid nanoparticle.

24. A pharmaceutical composition, comprising a pharmaceutically acceptable carrier, diluent, or excipient, and any one selected from the group consisting of the following or any combination thereof:
the nucleic acid construct according to any one of claims 1-7 and 17, the RNA molecule according to any one of claims 8-15 and 18, the polynucleotide according to claim 16, the vector according to claim 19, and the delivery vehicle according to claim 23.

25. A product or kit, comprising any one selected from the group consisting of the following or any combination thereof:
the nucleic acid construct according to any one of claims 1-7 and 17, the RNA molecule according to any one of claims 8-15 and 18, the polynucleotide according to claim 19, the delivery vehicle according to claim 23, and the pharmaceutical composition according to claim 24.

26. Use of the nucleic acid construct according to any one of claims 1-7 and 17 or the RNA molecule according to any one of claims 8-15 and 18 for increasing an expression level of a protein expressed by the ORF, or in the preparation of a product for increasing the expression level of the protein expressed by the ORF.

27. A method for treating and/or preventing a disease, comprising administering to a subject in need thereof a therapeutically effective amount of the nucleic acid construct according to any one of claims 1-7 and 17, the RNA molecule according to any one of claims 8-15 and 18, the polynucleotide according to claim 16, the delivery vehicle according to claim 23, and/or the pharmaceutical composition according to claim 24, the disease being selected from the group consisting of ischemic diseases, metabolic syndrome, diabetes and complications thereof, restenosis, and nerve injury,
wherein preferably, the ischemic diseases are selected from the group consisting of coronary artery disease (CAD), peripheral artery disease (PAD), myocardial infarction, limb ischemia, thrombosis angiitis obliterans (TAO), and diabetic artery obliterans (DAO); more preferably, the limb ischemia is lower limb ischemia; most preferably, the limb ischemia is critical limb ischemia (CLI);
preferably, the diabetes and complications thereof are selected from the group consisting of diabetic peripheral neuropathy, diabetic foot ulcer (DFU), and diabetic artery obliterans (DAO);
preferably, the restenosis is selected from the group consisting of post-operative restenosis and post-perfusion restenosis;
preferably, the nerve injury is selected from the group consisting of neurodegenerative diseases, traumatic nerve injury, and peripheral neuropathy; more preferably, the neurodegenerative diseases are selected from the group consisting of amyotrophic lateral sclerosis (ALS), Parkinson's disease, and dementia, and the peripheral neuropathy is diabetic peripheral neuropathy.

28. A method for promoting growth and/or migration of endothelial cells, comprising administering to a subject in need thereof an effective amount of the nucleic acid construct according to any one of claims 1-7 and 17, the RNA molecule according to any one of claims 8-15 and 18, the polynucleotide according to claim 16, the delivery vehicle according to claim 23, and/or the pharmaceutical composition according to claim 24.

29. A method for promoting angiogenesis, comprising administering to a subject in need thereof an effective amount of the nucleic acid construct according to any one of claims 1-7 and 17, the RNA molecule according to any one of claims 8-15 and 18, the polynucleotide according to claim 16, the delivery vehicle according to claim 23, and/or the pharmaceutical composition according to claim 24.
